# EUROPEAN PATENT APPLICATION

(11) **EP 4 483 945 A1**
(43) Date of publication of application: **01.01.2025**
(21) Application number: 23759978.2
(22) Date of filing: 21.02.2023
(51) Int. Cl.: A61N 5/06, A45D 26/00

(54) **PHOTOCOSMETIC DEVICE**

(30) Priority: 25.02.2022 JP 2022028614; 25.02.2022 JP 2022028616; 25.02.2022 JP 2022028618; 25.02.2022 JP 2022028617
(71) Applicant: Panasonic Intellectual Property Management Co., Ltd., Kadoma-shi, Osaka 571-0057 (JP)
(72) Inventor: SAKAMOTO, Ryohei, Kadoma-shi, Osaka 571-0057 (JP); KASUGAI, Hideki, Kadoma-shi, Osaka 571-0057 (JP); KOMATSU, Toshiyuki, Kadoma-shi, Osaka 571-0057 (JP); OKUDA, Ichiro, Kadoma-shi, Osaka 571-0057 (JP)
(74) Representative: Novagraaf International SA
(86) International application number: PCT/JP2023/006241
(87) International publication number: WO 2023/162977

(57) **Abstract**

The present disclosure is aimed to reduce the uncomfortable feeling to be caused in a user. A photocosmetic device (100) includes a plurality of light source units (20), a housing (1), and a light exit surface (13). Each of the plurality of light source units (20) includes a light source (22). The plurality of light source units (20) are housed in the housing (1). Light of the light source (22) of each of the plurality of light source units (20) is emitted from the light exit surface (13) to an outside of the housing (1). The light exit surface (13) has a recessed shape recessed inward the housing (1).

## Description

### Technical Field

The present disclosure relates generally to photocosmetic devices. The present disclosure relates more specifically to a photocosmetic device configured to emit light to be illuminated on the skin.

### Background Art

Patent Literature 1 describes a hair removal device. The hair removal device includes a light emission unit having a substrate and a plurality of first LED dies. The plurality of first LED dies are mounted on the substrate on an area of at least 0.2 cm², and having a peak emission wavelength in the far red or infrared wavelength range of between 700 nm and 980 nm. Patent Literature 1 describes that LED dies are mounted on the substrate so that 8 times 8 = 64 LED dies are mounted on a 1 cm times 1 cm = 1cm² substrate area.

Patent Literature 1 further describes that hair follicles are located at around 1-3 mm below the skin surface, and that it is known that the coagulation needed for bringing a hair follicle into apoptosis (programmed cell death) is a function of both, temperature and time. Patent Literature 1 describes that the hair removal device is arranged to emit a treatment light pulse having a pulse length in the range of between 60 ms and 120 ms and that the first LED dies have a radiant flux such that a radiant fluence on the skin of a user in the range of between 3 J/cm² and 7 J/cm² is achieved by application of the treatment light pulse.

However, according to a usual photocosmetic device such as the hair removal device described in Patent Literature 1, an illumination of intense (high density) light of a light source onto the skin sufficient for the facilitation of the coagulation for bringing the hair follicle into apoptosis may cause an excessive increase of the light quantity at a surface of the skin, which may provide the user with uncomfortableness.

### Citation List

### Patent Literature

Patent Literature 1: JP 2019-509087 A

### Summary of Invention

The present disclosure is aimed to provide a photocosmetic device which can reduce the uncomfortable feeling to be caused in a user.

A photocosmetic device according to one aspect of the present disclosure includes a plurality of light source units, a housing, and a light exit surface. Each of the plurality of light source units includes a light source. The plurality of light source units are housed in the housing. Light of the light source of each of the plurality of light source units is emitted from the light exit surface to an outside of the housing. The light exit surface has a recessed shape recessed inward the housing.

### Brief Description of Drawings

FIG. 1 is a sectional view of a photocosmetic device according to embodiment 1;
FIG. 2 is a front view of the photocosmetic device;
FIG. 3 is a sectional view of a main part of the photocosmetic device;
FIG. 4 is a sectional view showing a usage state of the photocosmetic device;
FIG. 5 is a block diagram of the photocosmetic device;
FIG. 6 is a sectional view of a main part of a photocosmetic device according to a first variation of the embodiment 1;
FIG. 7 is a sectional view of a main part of a photocosmetic device according to a second variation of the embodiment 1;
FIG. 8 is a sectional view of a main part of a photocosmetic device according to a third variation of the embodiment 1;
FIG. 9 is a sectional view of a main part of a photocosmetic device according to a fourth variation of the embodiment 1;
FIG. 10 is a sectional view of a main part of a photocosmetic device according to a fifth variation of the embodiment 1;
FIG. 11 is a sectional view of a photocosmetic device according to a sixth variation of the embodiment 1;
FIG. 12 is a sectional view of a main part of a photocosmetic device according to a seventh variation of the embodiment 1;
FIG. 13 is a sectional view of a main part of a photocosmetic device according to an eighth variation of the embodiment 1;
FIG. 14 is a sectional view of a main part of a photocosmetic device according to a ninth variation of the embodiment 1;
FIG. 15 is a schematic view showing a usage state of a photocosmetic device according to a first comparative example;
FIG. 16 is a sectional view of a photocosmetic device according to embodiment 2;
FIG. 17 is a perspective view of a main part of the photocosmetic device;
FIG. 18 is a front view of the photocosmetic device;
FIG. 19 is a sectional view showing a usage state of the photocosmetic device;
FIG. 20 schematically illustrates the intensity distribution of light emitted from the photocosmetic device;
FIG. 21 is a schematic view showing how to use the photocosmetic device;
FIG. 22 is a perspective view of a main part of a photocosmetic device according to a first variation of the embodiment 2;
FIG. 23 is a sectional view of a main part of a photocosmetic device according to a second variation of the embodiment 2;
FIG. 24 is a sectional view of a main part of a photocosmetic device according to a third variation of the embodiment 2;
FIG. 25 is a perspective view of a main part of the photocosmetic device;
FIG. 26 is a sectional view of a main part of a photocosmetic device according to a fourth variation of the embodiment 2;
FIG. 27 is a sectional view of a main part of a photocosmetic device according to an alternative of the fourth variation of the embodiment 2;
FIG. 28 is a sectional view of a main part of a photocosmetic device according to a fifth variation of the embodiment 2;
FIG. 29 is a sectional view of a main part of a photocosmetic device according to a sixth variation of the embodiment 2;
FIG. 30 is a sectional view of a main part according to a seventh variation of the embodiment 2;
FIG. 31 is a sectional view of a main part of a photocosmetic device according to an eighth variation of the embodiment 2;
FIG. 32 is a schematic view showing how to use the photocosmetic device;
FIG. 33 is a sectional view of a photocosmetic device according to a second comparative example;
FIG. 34 is a schematic view showing how to use the photocosmetic device;
FIG. 35 is a perspective view of a photocosmetic device according to embodiment 3;
FIG. 36 is a front view of the photocosmetic device;
FIG. 37 is a sectional view of the photocosmetic device;
FIG. 38 is a schematic view showing a usage state of the photocosmetic device;
FIG. 39 is a sectional view of a main part of a photocosmetic device according to a first variation of the embodiment 3;
FIG. 40 is a sectional view of a main part of a photocosmetic device according to a second variation of the embodiment 3;
FIG. 41 is a sectional view of a photocosmetic device according to embodiment 4;
FIG. 42 is a sectional view showing a usage state of the photocosmetic device;
FIG. 43 is a sectional view of a main part of a photocosmetic device according to a first variation of the embodiment 4;
FIG. 44 is a sectional view of a main part of a photocosmetic device according to a second variation of the embodiment 4;
FIG. 45 is a sectional view of a main part of a photocosmetic device according to a third variation of the embodiment 4;
FIG. 46 is a block diagram of a photocosmetic device according to a fourth variation of the embodiment 4;
FIG. 47 is a sectional view of a main part of a photocosmetic device according to a fifth variation of the embodiment 4;
FIG. 48 is a sectional view of a main part of a photocosmetic device according to a sixth variation of the embodiment 4;
FIG. 49 is a view schematically illustrating a mechanism how the hair falls off from the skin by the illumination of light;
FIG. 50 is a view schematically illustrating a mechanism how the hair falls off from the skin by the illumination of light; and
FIG. 51 is a view schematically illustrating a mechanism how the hair falls off from the skin by the illumination of light.

### Description of Embodiments

Photocosmetic devices according to embodiments will now be described in detail with reference to the accompanying drawings. The drawings to be referred to in the following description of embodiments are all schematic representations. That is to say, the ratio of the dimensions (including thicknesses) of respective constituent elements illustrated on the drawings does not always reflect their actual dimensional ratio. Note that the embodiment and variations to be described below is only an exemplary one of various embodiments of the present disclosure and should not be construed as limiting. Any of the embodiments and the variations can be combined with each other. Moreover, the embodiments and variations may be readily modified in various manners depending on a design choice or any other factor as long as the advantages of the present disclosure are achievable.

### (1) Embodiment 1

### (1.1) Overview of Embodiment 1

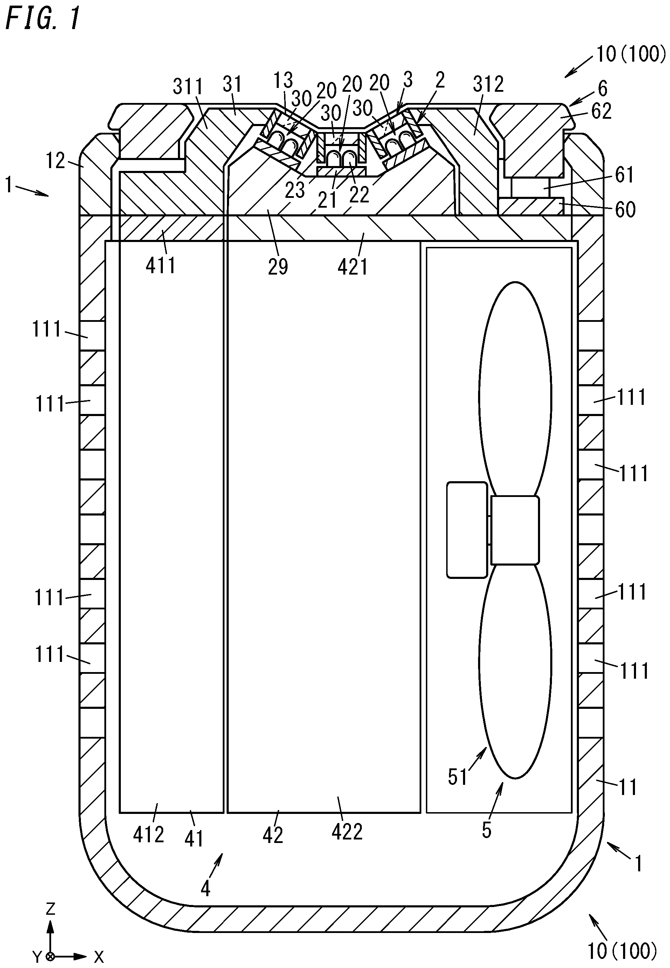

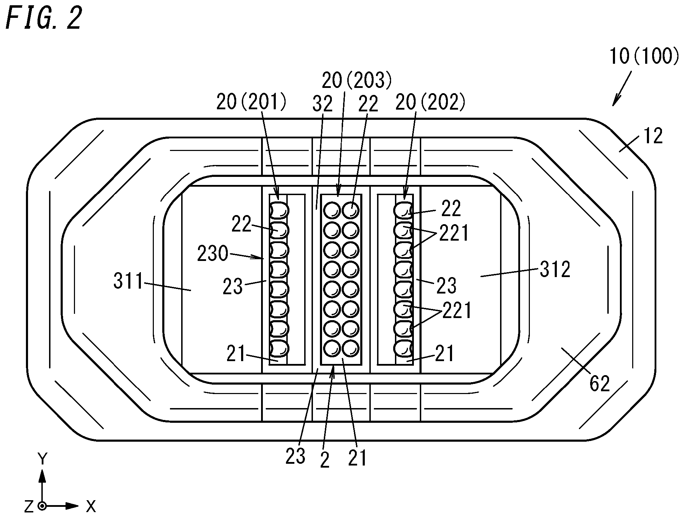

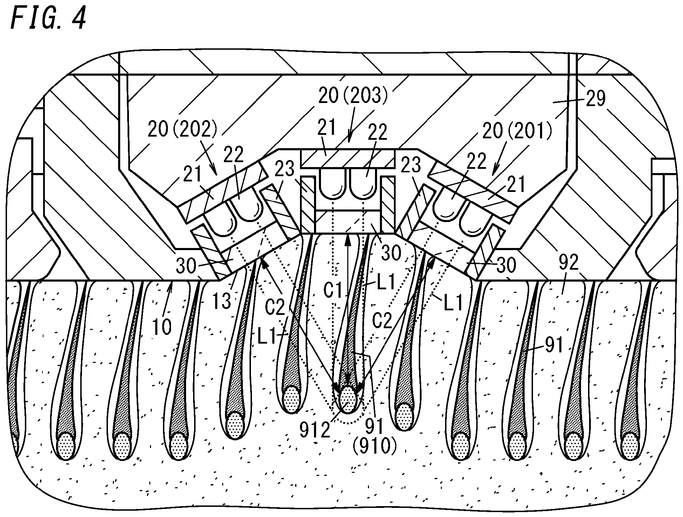

A photocosmetic device 100 of the present embodiment (see FIGS. 1, 2) is a device for illuminating light to a skin 92 (see FIG. 4) to cause absorption of the light in the skin 92 to treat the skin 92 with the function of the absorbed light.

Specifically, the photocosmetic device 100 of the present embodiment is a body hair treatment device for the hair control or the hair removal, which is configured to emit light to a region of the skin 92 with a hair 91 to facilitate the falling off of the hair 91 from the skin 92. According to the present disclosure, the "hair control" indicates a concept of at least temporary removing the hair 91 from a certain region of the skin 92. That is, the "hair control" tolerates the hair 91 grows up again from the certain region of the skin 92 from which the hair 91 is once removed. According to the present disclosure, the "hair removal" indicates a concept of removing the hair 91 from a certain region of the skin 92 and preventing the hair 91 from growing up again from this region (namely, indicates the permanent hair removal). Accordingly, the photocosmetic device 100 (body hair treatment device) of the present embodiment is a device for the hair control or the hair removal by means of the illumination of the light onto the skin 92.

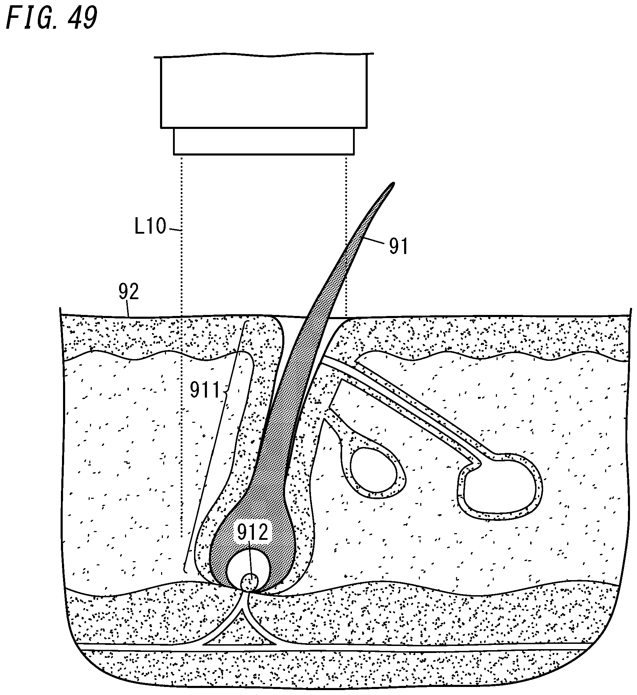

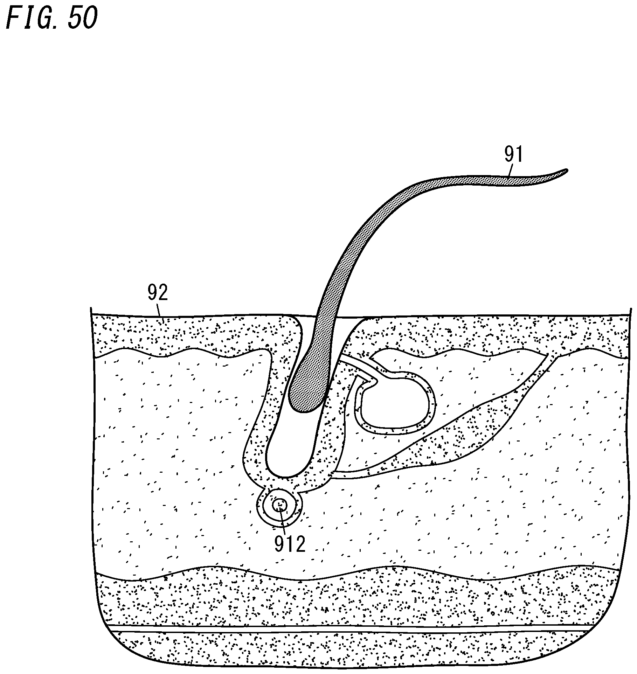

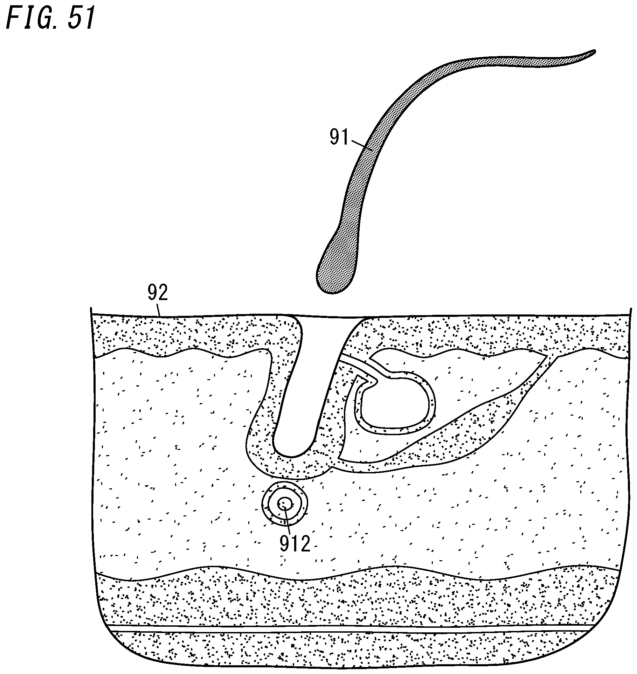

In regard to the hair control or the hair removal by the illumination of the light, the following mechanism is known. Specifically, when a region of the skin 92 with the hair 91 is irradiated with light L10 as shown in FIG. 49, the hair follicle 911 containing the melanin absorbs the irradiated light L10. The hair follicle 911 is heated with the energy of the light L10 thus absorbed, and the hair matrix 912 thereof is damaged with the heat. When the hair matrix 912 is damaged, the hair 91 leaves the hair matrix 912 according to the living body activity (see FIG. 50), and then is fallen off from the skin 92 (see FIG. 51).

Incidentally, around the surface layer of the skin 92 of animals (including the human), there is some substances that may absorb the light L10, such as the water, the vessel, and the like, in addition to the melanin contained in the hair follicle 911. Therefore, when the light L10 is illuminated on the skin 92 for the purpose of e.g., the hair control or the hair removal, at least part of the light L10 is absorbed in such the substances (water etc.) and generates the heat. The heat caused by the absorption of the light L10 may stimulate the nerves that detect pain and the like present in the skin 92, and a large stimulation on the nerves may cause an uncomfortable feeling in the user.

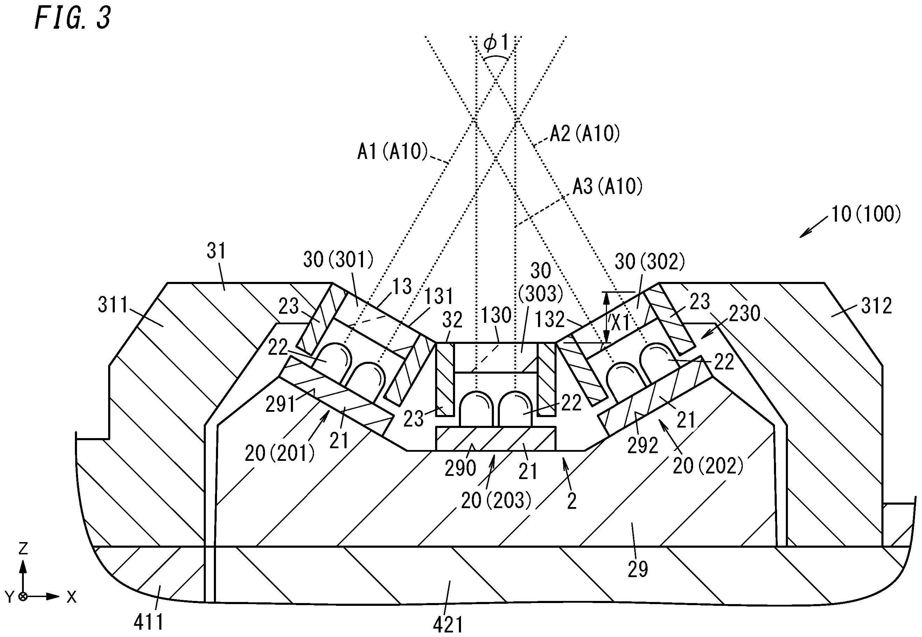

In this regard, according to the photocosmetic device 100 of the present embodiment, a light exit surface 13 from which light L1 of a plurality of light source units 20 is emitted to an outside of a housing 1 has a recessed shape recessed inward the housing 1, as shown in FIG. 3. Therefore, when the skin 92 is pressed against the light exit surface 13 of the photocosmetic device 100, the surface of the skin 92 is deformed from a flat shape to a shape deformed along the light exit surface 13 (i.e., a shape drawn into a space surrounded by the light exit surface 13) as shown in FIG. 4, for example. As a result, the hair matrix 912 of the hair 91 present in the skin 92 approaches the light exit surface 13.

As shown in FIG. 4, according to the photocosmetic device 100 of the present embodiment, the light L1 of the plurality of light source units 20 is emitted from the light exit surface 13 having the recessed shape toward the space surrounded by the light exit surface 13. Therefore, rays of the light L1 of the plurality of light source units 20 cross one another in an inside of the skin 92 which is drawn into the space surrounded by the light exit surface 13. Accordingly, the rays of the light L1 emitted from the plurality of light source units 20 can possibly cross one another in a desired region (e.g., a region around the hair matrix 912) inside the skin 92. This can facilitate the light L1 to be absorbed in the skin 92 in the region around the hair matrix 912 to efficiently damage the hair matrix 912, for example.

Moreover, according to the photocosmetic device 100 (body hair treatment device) of the present embodiment, the plurality of light source units 20 are arranged such that the rays of the light L1 of the light source units 20 cross one another at a position of the hair matrix 912 (bulb) of the hair 91 in the skin 92 in a state where the surface of the skin 92 is in contact with the light exit surface 13, as shown in FIG. 4. According to the photocosmetic device 100 of the present embodiment, therefore, the rays of the light L1 of the plurality of light source units 20 are gathered at the position of the hair matrix 912 in the skin 92. This allows the light L1 to be efficiently absorbed in the skin 92 in the region around the hair matrix 912 to efficiently damage the hair matrix 912.

According to the photocosmetic device 100 of the present embodiment, it is sufficient that total energy of the light L1 emitted from the plurality of light source units 20 reaches to an extent for damaging the hair matrix 912 to facilitate the falling off of the hair 91 at the position of the hair matrix 912 (where the rays of the light L1 cross one another). This allows a light source 22 of each of the light source units 20 to have a relatively small intensity of the light L1, which can reduce the amount of the light L1 to be absorbed in the substances (such as the water) in the skin 92. This can reduce the amount of the heat generated in regions (such as the region around the surface layer) of the skin 92 other than the position where the rays of the light L1 cross one another. It is therefore possible to reduce the uncomfortable feeling to be caused in a user.

### (1.2) Details of Embodiment 1

The photocosmetic device 100 of the present embodiment is described in detail below with reference to FIGS. 1 to 5. The photocosmetic device 100 of the present embodiment is the body hair treatment device for treating the body hair (hair 91) on the skin 92, as described above.

The following description is made based on one example where three axes are defined which are perpendicular to one another, namely the X-axis (first axis), the Y-axis (second axis) and the Z-axis (third axis). Specifically, the "X-axis" is defined as an axis along which the plurality of light source units 20 are arranged, and the "Z-axis" is defined as an axis along which the light source unit 20 and the light exit surface 13 are arranged. Note that the X-axis, the Y-axis and the Z-axis are all imaginary, and the arrows in the figures with the characters of "X", "Y", and "Z" are illustrated for the explanation purpose only and are not tangible.

In the description below, the positive and negative sides in the X-axis will be referred to as the "right" and "left", respectively, and the positive and negative sides in the Z-axis will be referred to as the "front" and "rear", respectively. However, these definitions of the directions do not limit the orientation of the photocosmetic device 100 when it is used.

According to the present disclosure, the "hair (body hair)" includes various hairs 91 protruding from the skin 92, namely various hairs on the skin 92, and may include various body hairs of the human such as hair on the head, beard or mustache, eyebrow, leg hair, nose hair, ear hair and the like. Moreover, the "hair (body hair)" used herein may include various hairs 91 protruding from the skin 92 of mammals such as dog or cat, or other animals. That is, the irradiation target of the body hair treatment device as an example of the photocosmetic device 100 of the present embodiment includes various skins 92 from which the hair 91 protrudes.

According to the present disclosure, the "user" indicates a subject who has the skin 92 to be irradiated with the light L1 from the photocosmetic device 100. The "user" is, for example, an animal including humans, mammals, and other animals. If the user is a human, the photocosmetic device 100 may be operated (held) by the user oneself, or may be operated by a person other than the user. If the user is an animal other than the human, the photocosmetic device 100 will be usually operated by a person other than the user.

According to the present disclosure, the "operator" indicates a person who operates the photocosmetic device 100. The operator may be the user oneself or a person other than the user.

According to the present disclosure, the feature "two members are thermally connected to each other" indicates any of the concepts: the two members are in contact with each other; and the two members are connected together via one or more other members (hereinafter, referred to as a "heat conduction member"). The heat conduction member has the heat transfer coefficient greater than the heat transfer coefficient of the air.

In the following, a hair control device 10 will be described as an example of the photocosmetic device 100 (body hair treatment device) of the present embodiment.

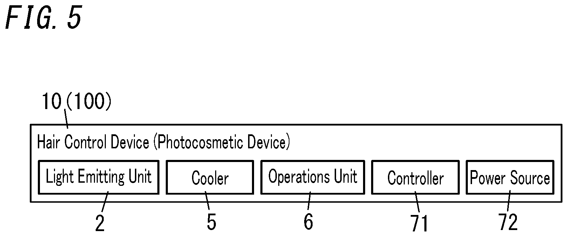

As shown in FIG. 1, the hair control device 10 includes the housing 1, the light exit surface 13, a light emitting unit 2, a light transmission member 3, a heat dissipation part 4, a cooler 5, an operations unit 6, a controller 71 (see FGI. 5), and a power source 72 (see FIG. 5).

As shown in FIG. 1, the housing 1 includes a first case 11 and a second case 12.

The first case 11 is made from resin, for example. The first case 11 has a tubular shape opened forward. The first case 11 has left and right side walls each of which is provided with a plurality of air vents 111 penetrating the side wall in the X-axis. The first case 11 is a part to be held by the operator when the hair control device 10 is used, for example.

The second case 12 is made from resin, for example. The second case 12 has a peripheral wall shape protruding forward from a periphery around a front opening of the first case 11. The second case 12 is coupled to the first case 11 with a coupling member such as screws, for example.

The housing 1 houses therein the light emitting unit 2, the light transmission member 3, the heat dissipation part 4, the cooler 5, the controller 71, and the power source 72. The operations unit 6 is held by the housing 1. As shown in FIG. 1, the heat dissipation part 4 and the cooler 5 are housed in the first case 11. The light emitting unit 2 and the light transmission member 3 are housed in the second case 12. The controller 71 and the power source 72 are housed in the first case 11. The operations unit 6 is held by the second case 12 with a part of the operations unit 6 exposed outward.

The light exit surface 13 is a surface from which the light of the light emitting unit 2 is emitted to an outside of the housing 1. According to the hair control device 10 of the present embodiment, the light exit surface 13 is positioned in a front surface of the housing 1 (a front surface of the second case 12).

As shown in FIG. 1, the light exit surface 13 has a recessed shape recessed inward the housing 1. Specifically, as shown in FIG. 3, the light exit surface 13 includes a bottom surface 130 having a flat plane shape perpendicular to the Z-axis, a first inclined surface 131 located on the left and inclined diagonally forward left with respect to the bottom surface 130, and a second inclined surface 132 located on the right and inclined diagonally forward right with respect to the bottom surface 130.

As shown in FIG. 1, the light emitting unit 2 includes a plurality of (three, in the embodiment) light source units 20, and a holding member 29.

As shown in FIGS. 2, 3, each of the plurality of light source units 20 includes a substrate 21, a light source 22, and a light blocking member 23.

The substrate 21 has a rectangular plate shape. The substrate 21 extends along the Y-axis.

The light source 22 is mounted on the substrate 21. The light source 22 is mounted on a surface on the front side of the substrate 21.

As shown in FIG. 2, the light source 22 includes a plurality of light emitting diodes (LEDs) 221. The LED 221 has so-called a shell-shape. In the LED 221, an LED device (semiconductor device) is enclosed in a case made from resin and having a shell shape.

The LED 221 has an emission wavelength falling within the range from 600 nm to 1000 nm. The LED 221 is a red LED or a near-infrared LED. An example of the emission wavelength of the LED 221 is about 850 nm. Another example of the emission wavelength of the LED 221 is about 950 nm. According to the present disclosure, the feature "emission wavelength of the LED 221" indicates a peak emission wavelength of the LED 221, that is, a wavelength exhibiting the maximum intensity in an emission spectrum of the LED 221.

The LED 221 is a monochromatic light source configured to emit a monochromatic light. That is, the LED 221 is configured to emit light having a relatively narrow bandwidth. The bandwidth of the emission light of the LED 221 may fall within the range from 60 nm to 100 nm, for example. According to the present disclosure, the feature "bandwidth of the emission light of the LED 221" indicates a full width at half maximum of the spectrum of the emission light of the LED 221.

The plurality of LEDs 221 have the same emission wavelengths as one another. According to the present disclosure, the feature "the plurality of LEDs 221 have the same emission wavelengths as one another" is not limited to a condition where the emission wavelengths (peak emission wavelengths) of the plurality of LEDs 221 are exactly identical to one another, but accepts a certain degree of tolerance such as the manufacturing errors, for example.

As shown in FIG. 2, the plurality of LEDs 221 are arranged in an array on the substrate 21. That is, the light source 22 includes an LED array including the plurality of LEDs 221 arranged in an array. In the embodiment, the light source 22 includes 16-pieces of LEDs 221. The 16-pieces of LEDs 221 are arranged in a 2x8 array viewed from the front, where there are two pieces along the X-axis and eight pieces along the Y-axis.

Each of the plurality of LEDs 221 is mounted on the substrate 21 such that it exhibits a light distribution property where the direction of the peak intensity is aligned with the normal direction of the substrate 21. In other words, each of the plurality of LEDs 221 has an optical axis along the normal direction of the substrate 21 on which the LED 221 is mounted.

In each light source unit 20, the light source 22 has an optical axis along the normal direction of the substrate 21. According to the present embodiment, the feature "optical axis of the light source 22 including the plurality of LEDs 221" indicates an imaginary axis (pillar shaped axis) defined by a region surrounded by the optical axes of the plurality of LEDs 221. According to the hair control device 10 of the present embodiment, a projection region of the optical axis of the light source 22 projected onto an imaginary plane perpendicular to the normal direction of the substrate 21 has a dimension (area) and a shape which are same as those of a region (rectangular region) surrounded by the optical axes of the plurality of LEDs 221 on the substrate 21.

According to the hair control device 10 of the present embodiment, each of the plurality of light source units 20 has an optical axis A10 (see FIG. 3). The optical axis A10 of the light source unit 20 indicates an imaginary line representing the ray of light emitted from the light source unit 20. According to the hair control device 10 of the present embodiment, the optical axis A10 of the light source unit 20 coincides with the optical axis of the light source 22. The optical axis A10 of the light source unit 20 is along the normal direction of the substrate 21.

As shown in FIG. 2, the light blocking member 23 has a shape surrounding the light source 22 when viewed in the normal direction of the substrate 21. The light blocking member 23 surrounds the light source 22 seamlessly when viewed in the normal direction of the substrate 21. According to the hair control device 10 of the present embodiment, the light blocking member 23 has a rectangular frame shape when viewed in the normal direction of the substrate 21. The light blocking member 23 has a wall shape extending along the normal direction of the substrate 21. Accordingly, the light blocking member 23 is like a wall surrounding the light source 22 (the plurality of LEDs 221) and having a rectangular frame shape when viewed in the normal direction of the substrate 21.

The light blocking member 23 is configured to reduce the light (leakage light) that is emitted from the light source 22 and goes to an outside of the substrate 21 (to an outside of the substrate 21 considered in an imaginary plane perpendicular to the normal direction of the substrate 21). Accordingly, the light blocking member 23 inhibits the light emitted from the light source 22 of the light source unit 20 including this light blocking member 23 from leaking to another light source unit 20.

The light blocking member 23 has an opening on a side where the light of the light source 22 is emitted (i.e., opened forward). The light of the light source 22 is emitted in the normal direction of the substrate 21 (emitted forward) through the opening.

The light blocking member 23 has the heat conduction property. The light blocking member 23 is made of metal, for example.

The light blocking member 23 has an inside surface on which a mirror is formed to reflect the light of the light source 22. The light emitted from the light source 22 and reached the light blocking member 23 is then reflected forward by the mirror. This can increase the amount of the light L1 emitted from the light source unit 20.

As shown in FIGS. 2, 3, according to the hair control device 10 of the present embodiment, the light blocking members 23 of the plurality of (three) light source units 20 are continuous at their front ends to form an integral body.

The (three) light blocking members 23 of the plurality of (three) light source units 20 form a light blocking part 230. According to the hair control device 10 of the present embodiment, the light blocking part 230 includes the three light blocking members 23 that are integrated together. The light blocking part 230 is configured to reduce light (leakage light) traveling from a light source 22 of one of the plurality of light source units 20 toward a light source 22 of at least one of the remainder of the plurality of light source units 20.

The holding member 29 has the heat conduction property. The holding member 29 may be made of metal or ceramic, for example.

The holding member 29 holds the substrates 21 of the plurality of (three) light source units 20. The holding member 29 holds the substrates 21 of the plurality of light source units 20 such that the plurality of (three) light source units 20 are arranged along the X-axis. As shown in FIG. 2, the plurality of (three) light source units 20 are arranged along the X-axis with their short-side axes of the respective substrates 21 are aligned one another when viewed from the front side.

Hereinafter, the three light source units 20 may be referred to as a first light source unit 201, a second light source unit 202, and a third light source unit 203, respectively, in order to distinguish the three light source units 20 from one another. As shown in FIG. 3, the first light source unit 201 is one of the three light source units 20 located on the left side (on the negative side in the X-axis). The second light source unit 202 is one of the three light source units 20 located on the right side (on the positive side in the X-axis). The third light source unit 203 is one of the three light source units 20 located on the center. The first light source unit 201 to the third light source unit 203 are arranged along the X-axis in the order of the first light source unit 201, the third light source unit 203, and the second light source unit 202.

As shown in FIG. 3, the holding member 29 has a front surface having a recessed shape whose center part in the X-axis is recessed rearward (towards the negative side in the Z-axis). The front surface of the holding member 29 includes a bottom surface 290 having a flat plane shape perpendicular to the Z-axis, a first inclined surface 291 extends diagonally forward left from a left end of the bottom surface 290, and a second inclined surface 292 extends diagonally forward right from a right end of the bottom surface 290.

In the hair control device 10 of the present embodiment, the bottom surface 290 in the front surface of the holding member 29 is parallel to the bottom surface 130 of the light exit surface 13. The first inclined surface 291 in the front surface of the holding member 29 is parallel to the first inclined surface 131 of the light exit surface 13. The second inclined surface 292 in the front surface of the holding member 29 is parallel to the second inclined surface 132 of the light exit surface 13.

The substrate 21 of the first light source unit 201 is located on the first inclined surface 291. The substrate 21 of the second light source unit 202 is located on the second inclined surface 292. The substrate 21 of the third light source unit 203 is located on the bottom surface 290.

An optical axis A1 (A10) of the first light source unit 201 is non-parallel to an optical axis A2 (A10) of the second light source unit 202. The optical axis A1 of the first light source unit 201 and the optical axis A2 of the second light source unit 202 cross each other on the outside of the housing 1.

The optical axis A1 of the first light source unit 201 is non-parallel to an optical axis A3 (A10) of the third light source unit 203. The optical axis A1 of the first light source unit 201 and the optical axis A3 of the third light source unit 203 cross each other on the outside of the housing 1.

The optical axis A2 of the second light source unit 202 is non-parallel to the optical axis A3 of the third light source unit 203. The optical axis A2 of the second light source unit 202 and the optical axis A3 of the third light source unit 203 cross each other on the outside of the housing 1.

According to the hair control device 10 of the present embodiment, the optical axes A10 of the plurality of (three) light source units 20 are non-parallel to one another. The optical axes A10 of the plurality of (three) light source units 20 cross one another at one point. As shown in FIG. 3, the optical axes A10 of the plurality of (three) light source units 20 cross one another on the outside of the housing 1 and in front of the light exit surface 13.

As shown in FIG. 4, the plurality of (three) light source units 20 are arranged such that the rays of the light L1 of the plurality of light source units 20 cross one another in a region around the hair matrix 912 (bulb) in the skin 92 in a state where the surface of the skin 92 is in contact with the light exit surface 13 (i.e., in a state where the skin 92 is drawn into the space surrounded by the light exit surface 13). As one example, the plurality of (three) light source units 20 are arranged such that the rays of the light L1 of the plurality of light source units 20 cross one another in the skin 92 at a position of a distance from the bottom surface 130 falling within the range from about 2 mm to 5 mm (e.g., about 4 mm) in a state where the surface of the skin 92 is in contact with the light exit surface 13. A crossing angle of the plurality of (three) light source units 20 (i.e., a crossing angle of the optical axes A10 thereof) are determined in consideration with the refractive index of the skin 92 and the like such that the rays of the light L1 of the plurality of light source units 20 cross one another in the region around the hair matrix 912 in the skin 92 in a state where the surface of the skin 92 is in contact with the light exit surface 13. A crossing angle between two light source units 20 having largest angle value (hereinafter, referred to as a crossing angle cp1 of the optical axes A10; see FIG. 3) among the crossing angles of the plurality of light source units 20 is set to fall within the range equal to or greater than 10° and equal to or smaller than 180°, for example. When the two light source units 20 are arranged such that the rays of the light L 1 cross each other in the skin 92 at the depth from the surface of the skin 92 falling within the range from about 2 mm to about 5 mm (e.g., about 4 mm) with the crossing angle ϕ1 of less than 10°, the light sources 22 of the two light source units 20 can include only a small number of LEDs 221. This may decrease the amount of light emitted from the light sources 22. The crossing angle ϕ1 of 180° or more may cause the difficulty to deform the skin 92 along the light exit surface 13 having the recessed shape. Moreover, for realizing the optical axes A10 crossing each other in the inside of the skin 92 with a large crossing angle ϕ1, the light source units 20 should be disposed apart from each other, which may increase the size of the device. The crossing angle cp1 may be smaller than 170°. The crossing angle ϕ1 may fall within the range equal to or greater than 50° and equal to or smaller than 160°. The crossing angle ϕ1 may fall within the range equal to or greater than 90° and equal to or smaller than 150°.

In an example, the light emitted from the light emitting unit 2 (i.e., the plurality of light source units 20) has the intensity such that the light density on the light exit surface 13 falls within the range from 15 W/cm² to 35 W/cm². The emission duration of the light emitted from the light emitting unit 2 falls within the range from 500 ms to 2000 ms, for example. The light having the intensity within the above range with the emission duration equal to or greater than 500 ms can provide a high effect on the hair 91 which is in a growth stage of an early stage of the anagen to the anagen. Moreover, the light having the intensity within the above range with the emission duration equal to or smaller than 2000 ms can advantageously suppress the excessive temperature increase in the skin 92 caused by the absorption of the light.

As shown in FIG. 1, the substrates 21 of the plurality of light source units 20 are out of contact and apart from one another in the axis (i.e., X-axis) along which the plurality of light source units 20 are arranged. A distance between substrates 21 of adjacent two light source units 20 is greater than a dimension of one LED 221, for example. Moreover, the light sources 22 of the plurality of light source units 20 are out of contact and apart from one another in the axis (i.e., X-axis) along which the plurality of light source units 20 are arranged. A distance between light sources 22 of adjacent two light source units 20 (a distance between LEDs 221 of the adjacent two light source units 20) may be greater than the dimension of one LED 221, for example. That is, the light sources 22 of the plurality of light source units 20 are arranged to be apart from one another. The plurality of light sources 22 are apart from one another, which can reduce the overlap of the rays of the light L1 of the plurality of light source units 20 on the light exit surface 13.

According to the hair control device 10 of the present embodiment, the light sources 22 of the plurality of light source units 20 are arranged to be apart from one another such that the rays of the light L1 of adjacent two light source units 20 do not substantially overlap each other on the light exit surface 13. According to the present embodiment, the feature "the rays of the light L1 of two light source units 20 do not substantially overlap each other on the light exit surface 13" indicates a state where the light L1 emitted from a focused one light source unit 20 of the two light source units 20 does not substantially reach a light transmission piece 30 (to be described later) arranged correspondingly to the other light source unit 20, for example.

When the overlap of the rays of the light L1 of the plurality of light source units 20 is small in the light exit surface 13, the intensity of light is relatively low in an entire of the surface of the skin 92 while the surface of the skin 92 is in contact with the light exit surface 13. This can suppress the increase in the temperature of the skin 92 around the surface layer caused by the absorption of the light. The uncomfortable feeling to be caused in a user can be reduced accordingly.

Moreover, since the hair control device 10 of the present embodiment includes the light blocking part 230 (the light blocking member 23), it is possible to further reduce the overlap of the rays of the light L 1 of the plurality of light source units 20 on the light exit surface 13.

The light transmission member 3 is disposed in front of the light emitting unit 2. The light exit surface 13 is at least partially constituted by a surface of the light transmission member 3. In the present embodiment, the surface (front surface) of the light transmission member 3 on the outer side of the housing 1 constitutes (a whole of) the light exit surface 13. The light transmission member 3 allows the light of the light sources 22 of the plurality of light source units 20 of the light emitting unit 2 to pass through. Note that the illustration of the light transmission member 3 is omitted in FIG. 2.

The light transmission member 3 is made of the material having the small absorptance at the wavelength of the light emitted from the light source 22. In other words, the light transmission member 3 is made of the material exhibiting small absorption loss with respect to the light of the light source 22. The light transmission member 3 is transparent at the wavelength of the light of the light source 22. The light transmission member 3 is made of the material having the small extinction coefficient at the wavelength of the light of the light source 22. It may be preferable that the light transmission member 3 is made of the material having the extinction coefficient of 0.0 at the wavelength of the light of the light source 22. The light transmission member 3 having the small absorptance of the light is less likely to absorb the light, which can suppress the increase in the temperature of the light transmission member 3. The skin 92 in contact with the light transmission member 3 will be less stimulated.

Furthermore, the light transmission member 3 is made of the material having the refractive index greater than that of the skin 92 of the human at the wavelength of the light of the light source 22 (i.e., at the emission wavelength of the light source 22). In the embodiment, the light transmission member 3 is made of the material having the refractive index of 1.7 or more at the emission wavelength of the light source 22. The refractive index is measured in compliance with the measuring method for refractive index of optical glass, defined by JIS B 7071, for example. The light transmission member 3 having the refractive index greater than the refractive index of the skin 92 can reduce the total reflection of the light at the boundary between the light transmission member 3 and the surface of the skin 92 when the light travels in the light transmission member 3 toward the skin 92.

The light transmission member 3 has the heat conduction property. The light transmission member 3 is made of the material having the thermal conductivity of 1 W/mK or more. The thermal conductivity is measured in compliance with the measurement method of thermal conductivity for fine ceramics by flash method, defined by JIS R 1611, for example. The light transmission member 3 having the heat conduction property can facilitate the cooling of the body in contact with the light transmission member 3.

The light transmission member 3 is made of the material having the refractive index of 1.7 or more at the wavelength of the light L1 of the light source 22 and having the thermal conductivity of 1 W/mK or more, for example. Specifically, the light transmission member 3 is made of at least one material selected from the group consisting of sapphire (Al₂O₃), zinc oxide (ZnO), zirconium oxide (ZrO₂), magnesium oxide (MgO), gallium nitride (GaN), aluminum nitride (AIN), and diamond. The light transmission member 3 may preferably be made of sapphire. The light transmission member 3 may be made of two or more materials. The refractive indices and the thermal conductivities (W/mK) of these materials are shown in the following Table 1.

The refractive index shown in the Table 1 is a value at a particular wavelength (specifically, d-line: 587.6 nm). Note that the value of the refractive index of the material varies depending on the wavelength of the light of the light source 22, due to the wavelength dispersion of the refractive index. For example, the value of the refractive index of the sapphire decreases as the increase in the wavelength. Specifically, the refractive index of the sapphire at the wavelength of 850 nm is 1.76, for example. The value of the refractive index of the sapphire increase as the decrease in the wavelength. The refractive index of the sapphire at the wavelength of 405 nm is 1.786, for example.

**Table 1**

| | Refractive Index | Thermal Conductivity (W/mK) |
|---|---|---|
| Sapphire | 1.768 | 42 |
| ZnO | 2.01 | 20 |
| ZrO₂ | 2.217 | 3 |
| MgO | 1.74 | 47 |
| GaN | 2.393 | 200 |
| AlN | 2.175 | 150 |
| Diamond | 2.417 | 1000 |

As shown in FIGS. 1, 3, the light transmission member 3 includes a plurality of (three) light transmission pieces 30. Each of the plurality of light transmission pieces 30 has a plate shape. The plurality of light transmission pieces 30 arranged to correspond (one-to-one) to the plurality of light source units 20. As shown in FIG. 3, each of the plurality of light transmission pieces 30 is positioned on the optical axis A10 of a corresponding light source unit 20. Each of the plurality of light transmission pieces 30 faces the light source 22 of the corresponding light source unit 20.

Each light transmission piece 30 is fixed to the light blocking member 23 of the corresponding light source unit 20 to occlude the front opening of the light blocking member 23 of the corresponding light source unit 20.

Hereinafter, in order to distinguish the three light transmission pieces 30 from one another, a light transmission piece 30 corresponding to the first light source unit 201 may be referred to as a first light transmission piece 301, a light transmission piece 30 corresponding to the second light source unit 202 may be referred to as a second light transmission piece 302, and a light transmission piece 30 corresponding to the third light source unit 203 may be referred to as a third light transmission piece 303, respectively.

According to the hair control device 10 of the present embodiment, a front surface of the first light transmission piece 301 serves as the first inclined surface 131 of the light exit surface 13, a front surface of the second light transmission piece 302 serves as the second inclined surface 132 of the light exit surface 13, and a front surface of the third light transmission piece 303 serves as the bottom surface 130 of the light exit surface 13. That is, the light exit surface 13 includes the front surface of the first light transmission piece 301 (the first inclined surface 131), the front surface of the second light transmission piece 302 (the second inclined surface 132), and the front surface of the third light transmission piece 303 (the bottom surface 130).

According to the hair control device 10 of the present embodiment, a depth X1 of the recessed space surrounded by the light exit surface 13 falls within the range of greater than 0 mm and equal to or smaller than 30 mm. According to the present disclosure, the feature "depth X1 of the recessed space surrounded by the light exit surface 13" indicates a depth of the surface, from which the light is actually emitted, of the housing 1. In one specific example, the "depth X1 of the recessed space surrounded by the light exit surface 13" indicates the dimension between a far end of the first inclined surface 131 away from the bottom surface 130 (or a far end of the second inclined surface 132 away from the bottom surface 130) and a bottom surface of the recessed space (i.e., the bottom surface 130) measured along the Z-axis (see FIG. 3). The depth X1 may preferably fall within the range of equal to or greater than 2 mm and equal to or smaller than 30 mm.

The depth X1 equal to or greater than the lower limit of the above range allows a plurality of rays of light to be emitted from the light exit surface 13 that are non-parallel to one another and thus allows the plurality of non-parallel rays of light to cross one another around the hair matrix 912, which can increase the amount of light focused onto the region around the hair matrix 912. The depth X1 greater than the upper limit of the above range may cause the difficulty in deforming the skin 92 along the light exit surface 13.

The front surface of each light transmission piece 30 intersects with the optical axis A10 of the corresponding light source unit 20. In the embodiment, the front surface of each light transmission piece 30 intersects with the optical axis A10 of the corresponding light source unit 20 at right angles. Specifically, as shown in FIG. 3, the front surface of the first light transmission piece 301 (i.e., the first inclined surface 131) intersects with the optical axis A1 of the first light source unit 201 (at right angles, in the embodiment). The front surface of the second light transmission piece 302 (i.e., the second inclined surface 132) intersects with the optical axis A2 of the second light source unit 202 (at right angles, in the embodiment). The front surface of the third light transmission piece 303 (i.e., the bottom surface 130) intersects with the optical axis A3 of the third light source unit 203 (at right angles, in the embodiment). In short, according to the hair control device 10 of the present embodiment, the optical axis A10 of the light source 22 of each of the plurality of light source units 20 intersects with the light exit surface 13 (at right angles, in the embodiment). This can increase the light extraction efficiency of the light from the light exit surface 13.

As shown in FIGS. 2, 3, a front end part of the light blocking member 23 is located between two light transmission pieces 30 adjacent in the X-axis. In the light exit surface 13, thus, the plurality of light transmission pieces 30 are partitioned by the light blocking member 23. Accordingly, the hair control device 10 of the present embodiment includes a partition 32 (the front end part of the light blocking member 23) that separates the plurality of light transmission pieces 30 from one another in the light exit surface 13. The partition 32 can reduce the overlap of the rays of the light L1 of the plurality of light source units 20 on the light exit surface 13.

As described above, the light transmission pieces 30 are fixed to the light blocking member 23. Accordingly, when the plurality of light blocking members 23 are positioned with respect to the plurality of light sources 22, the plurality of light transmission pieces 30 are also positioned with respect to the plurality of light sources 22.

The light transmission member 3 is held by a holder 31. The holder 31 is a part of the housing 1. According to the hair control device 10 of the present embodiment, the holder 31 holds the light blocking part 230 (the plurality of light blocking members 23 integrated together) to thereby hold the light transmission member 3 (the plurality of light transmission pieces 30) indirectly.

The holder 31 has the heat conduction property. The holder 31 is made of metal, for example. The holder 31 that holds the light transmission member 3 has the heat conduction property, which can further facilitate the cooling of the body in contact with the light transmission member 3.

As shown in FIG. 1, the holder 31 includes a first holding arm 311 and a second holding arm 312. As shown in FIGS. 2, 3, the first holding arm 311 is coupled to the left end of the light blocking part 230 (the left end of the light blocking member 23 of the first light source unit 201), and the second holding arm 312 is coupled to the right end of the light blocking part 230 (the right end of the light blocking member 23 of the second light source unit 202), whereby the light blocking part 230 is held by the holder 31.

The light transmission member 3 (the plurality of light transmission pieces 30) is thermally connected to the holder 31 via the light blocking part 230. This allows the heat of the body in contact with the light transmission member 3 to transfer to the holder 31 via the light blocking part 230 (the light blocking member 23).

As shown in FIG. 1, the heat dissipation part 4 includes a first heat dissipation part 41 and a second heat dissipation part 42. The first heat dissipation part 41 is designed to primarily dissipate the heat of the light transmission member 3. The second heat dissipation part 42 is designed to primarily dissipate the heat of the light transmission member 3 and the light emitting unit 2.

The first heat dissipation part 41 includes a first coupler 411 and a first heat dissipation member 412.

The first coupler 411 has the heat conduction property. The first coupler 411 is made of metal, for example.

The first coupler 411 has a plate shape having the thickness along the Z-axis and extending along the Y-axis. The first coupler 411 is disposed inside the housing 1 to occlude the left part of the front opening of the first case 11. The first holding arm 311 of the holder 31 is provided on a front surface of the first coupler 411.

The first heat dissipation member 412 has the heat conduction property. The first heat dissipation member 412 is made of metal, for example.

The first heat dissipation member 412 includes a plurality of first heat dissipation fins. Each of the plurality of first heat dissipation fins has a plate shape extending rearward from a rear surface of the first coupler 411. The plurality of first heat dissipation fins are coupled together with the first coupler 411. The plurality of first heat dissipation fins are disposed in an inside space of the first case 11. The plurality of first heat dissipation fins are arranged along the Y-axis. A gap is formed between two first heat dissipation fins adjacent to each other in the Y-axis.

The first heat dissipation member 412 is thermally connected to the light transmission member 3 via the first coupler 411, the first holding arm 311 and the light blocking part 230. The heat in the light transmission member 3 is transferred to the plurality of first heat dissipation fins of the first heat dissipation member 412 via the light blocking part 230, the first holding arm 311 and the first coupler 411, and then is emitted to the outside from the plurality of first heat dissipation fins (through the air vents 111 of the first case 11, for example).

The second heat dissipation part 42 includes a second coupler 421 and a second heat dissipation member 422.

The second coupler 421 has the heat conduction property. The second coupler 421 is made of metal, for example.

The second coupler 421 has a plate shape having the thickness along the Z-axis. The second coupler 421 is disposed inside the housing 1 to occlude the center and right part of the front opening of the first case 11. The holding member 29 of the light emitting unit 2 and the second holding arm 312 of the holder 31 are provided on a front surface of the second coupler 421.

The second heat dissipation member 422 has the heat conduction property. The second heat dissipation member 422 is made of metal, for example.

The second heat dissipation member 422 includes a plurality of second heat dissipation fins. Each of the plurality of second heat dissipation fins has a plate shape extending rearward from a left part of a rear surface of the second coupler 421. The plurality of second heat dissipation fins are coupled together with the second coupler 421. The plurality of second heat dissipation fins are disposed in the inside space of the first case 11. The plurality of second heat dissipation fins are arranged along the Y-axis. A gap is formed between two second heat dissipation fins adjacent to each other in the Y-axis.

The first heat dissipation fin and the second heat dissipation fin are located at the same position as each other in the Y-axis. The gap of the first heat dissipation fins and the gap of the second heat dissipation fins are located at the same position as each other in the Y-axis.

The second heat dissipation member 422 is thermally connected to the substrates 21 of the plurality of light source units 20 via the second coupler 421 and the holding member 29. The heat generated in the light sources 22 of the light source units 20 is transferred to the plurality of second heat dissipation fins of the second heat dissipation member 422 via the holding member 29 and the second coupler 421, and then is emitted to the outside from the plurality of second heat dissipation fins (through the air vents 111 of the first case 11, for example).

The second heat dissipation member 422 is thermally connected to the light transmission member 3 via the second coupler 421, the second holding arm 312 and the light blocking part 230. The heat in the light transmission member 3 is transferred to the plurality of second heat dissipation fins of the second heat dissipation member 422 via the light blocking part 230, the second holding arm 312 and the second coupler 421, and then is emitted to the outside from the plurality of second heat dissipation fins (through the air vents of the first case 11, for example).

As described above, the heat in the light transmission member 3 is emitted to the outside via the first heat dissipation member 412 and the second heat dissipation member 422. Therefore, the heat of the body in contact with the light transmission member 3 is taken away by the light transmission member 3 and thus the body is cooled. That is, the light transmission member 3 has a cooling capability for facilitating the cooling of the body in contact with the light transmission member 3.

According to the hair control device 10 of the present embodiment, the surface (front surface) of the light transmission member 3 constitutes the light exit surface 13. The hair control device 10 is adapted to be used in a state where the front surface of the light transmission member 3 (i.e., the light exit surface 13) is in contact with the skin 92 (see FIG. 4). In the usage state of the hair control device 10, therefore, the skin 92 which is in contact with the front surface of the light transmission member 3 (i.e., the light exit surface 13) is cooled by the heat of the skin 92 being taken away by the light transmission member 3. That is, the light transmission member 3 has a cooling capability for facilitating the cooling of the skin 92 in contact with the light transmission member 3. The light transmission member 3 having the cooling capability can suppress the increase in the temperature of the skin 92 around the surface layer. The nerves that detect the pain in the skin 92 will be less stimulated, and the uncomfortable feeling to be caused in a user can be reduced accordingly.

As shown in FIG. 1, the cooler 5 is disposed inside the first case 11. In the present embodiment, the cooler 5 includes a cooling fan 51. The cooling fan 51 is disposed in the first case 11 and arranged side-by-side with the second heat dissipation member 422 and the first heat dissipation member 412 in the X-axis.

The cooling fan 51 is disposed to send out the wind leftward (flowing to the negative side in the X-axis). The wind sent out from the cooling fan 51 reaches the second heat dissipation member 422 and the first heat dissipation member 412 to take the heat away from the second heat dissipation member 422 and the first heat dissipation member 412. This can facilitate the cooling of the second heat dissipation member 422 and the first heat dissipation member 412. As a result, the light transmission member 3 thermally connected with the first heat dissipation member 412 and the second heat dissipation member 422 is cooled. In short, the cooler 5 is disposed to cool the light transmission member 3.

The hair control device 10 including the cooler 5 can further suppress the increase in the temperature of the skin 92 around the surface layer. The uncomfortable feeling to be caused in a user can be further reduced accordingly.

The cooler 5 is controlled by the controller 71 such that the temperature of the surface of the skin 92 in contact with the light transmission member 3 falls within a predetermined temperature range, for example. The predetermined temperature range is a range of -5°C to 35°C, for example. Making the temperature of the surface of the skin 92 equal to or greater than -5°C can reduce the uncomfortable feeling due to the low temperature state of the skin 92. Making the temperature of the surface of the skin 92 equal to or smaller than 35°C can reduce the uncomfortable feeling due to the high temperature state of the skin 92.

The hair control device 10 may include a temperature sensor configured to measure the temperature of the skin 92. The controller 71 may be configured to operate the cooler 5 based on the measurement result of the temperature sensor. It should be noted that the hair control device 10 can make the temperature of the surface of the skin 92 fall within the above predetermined temperature range without the temperature sensor by, for example, preliminary setting the operation condition of the cooler 5 (such as the rotation speed of the cooling fan 51) appropriately or allowing the operator to turn the cooler 5 on and off by a switch and the like.

The operations unit 6 is configured to be operated by the operator. As shown in FIG. 1, the operations unit 6 includes a substrate 60 located on the front surface of the second coupler 421, a switch 61 mounted on a front surface of the substrate 60, and a push button 62 disposed in front of the switch 61. The push button 62 is configured to be operated by the operator to move rearward to push the switch 61.

As shown in FIG. 2, the push button 62 is formed in a peripheral wall shape surrounding the plurality of light source units 20 of the light emitting unit 2. As shown in FIG. 1, in a part of the front surface of the push button 62 positionally corresponding to the light exit surface 13 in the X-axis, a recess is formed. The recess is recessed rearward (toward the negative side in the Z-axis) along the recessed shape of the light exit surface 13.

As shown in FIG. 1, the push button 62 is held by the housing 1 such that the front surface of the push button 62 is located on the front side of the front surface of the housing 1 (the front surface of the second case 12). When the hair control device 10 is pressed against the skin 92, a part of the skin 92 around a region in contact with the light transmission member 3 comes to contact with the front surface of the push button 62 and pushes the push button 62 rearward. As a result, the switch 61 is pressed by the push button 62. According to the configuration where the push button 62 is pushed by the skin 92 when the skin 92 comes in contact with the light transmission member 3, it is possible to prevent the light emitting unit 2 from emitting the light at an unwanted timing.

The controller 71 is configured to control the operation of the hair control device 10. The controller 71 is configured to control the operations of the light emitting unit 2 and the cooler 5.

The controller 71 is implemented as a microcontroller including one or more processors and one or more memories. The microcontroller performs the function of the controller 71 by making the one or more processors execute a program stored in the one or more memories. The program may be stored in advance in the memories, or may be downloaded via a telecommunications line or distributed after having been stored in a non-transitory storage medium such as a memory card. In other words, the program is a program for making the one or more processors function as the controller 71.

The controller 71 controls the operations of the light emitting unit 2 and the cooler 5 in accordance with the operation given to the operations unit 6, for example. The controller 71 is configured to make the light emitting unit 2 start emitting the light and make the cooling fan 51 start operating when the switch 61 is pushed in accordance with a push operation given to the push button 62, for example. The controller 71 is configured to, when the push operation given to the switch 61 from the push button 62 is released, stop the light emitting unit 2 emitting the light, and then stop the cooling fan 51 operating after a predetermined time (which may be 0 second), for example.

The power source 72 supplies the electric power to the light emitting unit 2, the cooler 5 and the controller 71. The power source 72 supplies the DC power to the light emitting unit 2, the cooler 5 and the controller 71. The power source 72 may be a battery, for example. The power source 72 may include a rechargeable secondary battery and a charging circuit configured to convert an AC power supplied from an external AC power supply into a DC power to charge the secondary battery. However, the power source 72 is not limited thereto, but may include a power source circuit including an AC/DC converter configured to convert an AC power supplied from an external AC power supply into a DC power.

### (1.3) Advantage in Embodiment 1

Advantages in the photocosmetic device 100 of the present embodiment will be described while comparing with a photocosmetic device 200 of a first comparative example.

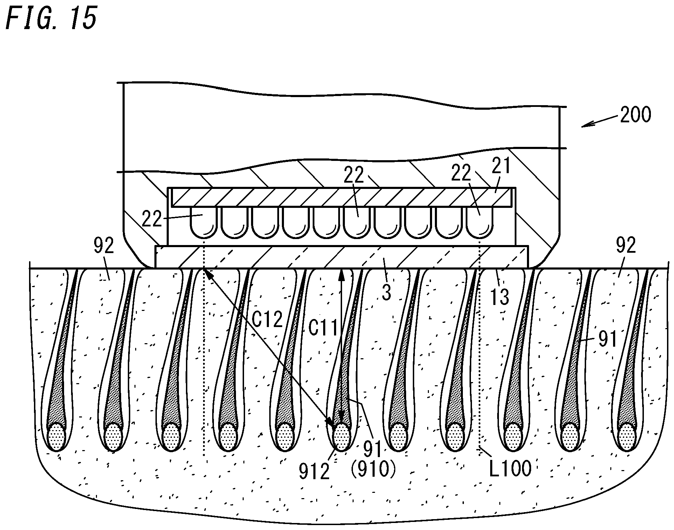

As shown in FIG. 15, the photocosmetic device 200 of the first comparative example includes a plurality of light sources 22. According to the photocosmetic device 200 of the first comparative example, a light exit surface 13 has a planar shape. According to the photocosmetic device 200 of the first comparative example, light L100 of the plurality of light sources 22 is emitted planarly from the light exit surface 13.

The following description is made focusing on one hair 91 (a hair 910 shown in FIG. 15) among a lot of hairs 91 of the skin 92. It is assumed a case where the hair matrix 912 of this hair 910 is irradiated with light to be damaged in order to facilitate the falling off of this hair 910 from the skin 92.

As shown in FIG. 15, according to the photocosmetic device 200 of the first comparative example, the light emitted from a light source 22 positioned in front of the hair 910 among the plurality of light sources 22 travels in the skin 92 by a distance C11 before reaching the hair matrix 912 of the hair 910. The distance C11 corresponds to a depth of the hair matrix 912 of the hair 91 in the skin 92 (about 2 mm to 5 mm).

According to the photocosmetic device 200 of the first comparative example, the light emitted from a light source 22 positioned apart from the hair 910 in the left or right needs to travel in the skin 92 by a distance C12 greater than the distance C11 (C12>C11), before reaching the hair matrix 912 of the hair 910. The intensity of the light gradually attenuates while the light traveling in the skin 92, by being absorbed in substances present in the skin 92, such as the water. Therefore, the light emitted from the light source 22 positioned apart from the hair 910 tends to provide less damage to the hair matrix 912 of the hair 910. Moreover, the light absorbed in the substances such as the water in the skin 92 is converted into the heat, which may stimulate the nerves that detect pain in the skin 92 to provide the uncomfortable feeling to the user.

In contrast, according to the photocosmetic device 100 of the present embodiment, the light exit surface 13 has the recessed shape, and thus the skin 92 is irradiated with the light L1 while the skin 92 is deformed so as to be drawn into the space surrounded by the light exit surface 13, as shown in FIG. 4. That is, according to the photocosmetic device 100 of the present embodiment, the skin 92 is irradiated with the light L1 in a state where the hair matrix 912 of the hair 910 approaches near the bottom surface 130 of the light exit surface 13. In this state, the light L1 emitted from a light source unit 20 positioned in front of the hair 910 (i.e., the third light source unit 203) travels in the skin 92 by a distance C1 before reaching the hair matrix 912 of the hair 910. The distance C1 corresponds to a depth of the hair matrix 912 in the skin 92 (about 2 mm to 5 mm). Moreover, according to the photocosmetic device 100 of the present embodiment, the light L1 emitted from a light source unit 20 positioned apart from the hair 910 in the left or right (i.e., the first light source unit 201 or the second light source unit 202) travels in the skin 92 before reaching the hair matrix 912 of the hair 910 by a distance C2, which is substantially the same as the distance C1 (C2 ≈ C1). Accordingly, the photocosmetic device 100 of the present embodiment can efficiently provide the damage to the hair matrix 912 of the hair 910 not only by the light L1 emitted from the light source unit 20 positioned in front of the hair 910 but also the light L1 emitted from the light source unit 20 positioned apart from the hair 910. Accordingly, the photocosmetic device 100 of the present embodiment can efficiently provide the damage to the hair matrix 912 of the hair 910 by the light L1 emitted from (all of) the plurality of light source units 20.

Moreover, according to the photocosmetic device 100 of the present embodiment, the plurality of light source units 20 are arranged such that the rays of the light L 1 of the plurality of light source units 20 cross one another at a position of the hair matrix 912 (bulb) of the focused one hair 910 in the skin 92 in a state where the surface of the skin 92 is in contact with the light exit surface 13. This can further efficiently provide the damage to the hair matrix 912 of the hair 910.

According to the photocosmetic device 100 of the present embodiment, it is sufficient that total energy of the light L1 emitted from the plurality of light source units 20 reaches to an extent for damaging the hair matrix 912 to facilitate the falling off of the focused hair 910 at the position of the hair matrix 912 of the focused one hair 910. This can decrease the intensity of the light emitted from the light source 22 of each light source unit 20. This can reduce the amount of the heat generated in regions of the skin 92 other than the position of the hair matrix 912 of the hair 910 caused by the absorption of the light L1, which can reduce the uncomfortable feeling to be caused in a user.

The photocosmetic device 100 of the present embodiment includes the light blocking part 230. This can reduce the leakage light (the light traveling from a light source 22 of one of the plurality of light source units 20 toward a light source 22 of another light source unit 20) and thus can improve the energy efficiency.

The photocosmetic device 100 of the present embodiment includes the light transmission member 3 (the plurality of light transmission pieces 30) that has the surface (front surface) constituting the light exit surface 13 and that allows the light of the light sources 22 of the plurality of light source units 20 to pass through. Incidentally, in a comparative device that does not include the light transmission member 3 and there is no object in the light exit surface 13 and when the device is pushed toward the skin 92 to locate the skin 92 in the light exit surface 13, the surface layer of the skin 92 may be entered into a space surrounded by the light blocking member 23. The amount of the skin 92 enters into the space varies depending on the way how the device is pushed hard to the skin 92. According to this device, the degree of the deformation of the skin 92 may vary depending on the way how to use the device, and thus the distances C1, C2 that the light L1, which is emitted from the light source unit 20, travels in the skin 92 before reaching the hair matrix 912 of the hair 910 may also vary depending on the way how to use the device. Contrary to this, according to the photocosmetic device 100 of the present embodiment, the surface (front surface) of the light transmission member 3 constitutes the light exit surface 13 (a surface to be in contact with the skin 92). This can equalize the degree of deformation of the skin 92 any time it is used, and can uniquely set the distances C1, C2 that the light L1, which is emitted from the light source unit 20, travels in the skin 92 before reaching the hair matrix 912 of the hair 910 even in a repeated use (i.e., without fluctuating the distances in each use).

According to the photocosmetic device 100 of the present embodiment, the light transmission member 3 has the heat conduction property. This can facilitate the cooling of the skin 92 and can reduce the uncomfortable feeling to be caused in the user. Moreover, the cooler 5 that cools the light transmission member 3 can further facilitate the cooling of the skin 92, which can further reduce the uncomfortable feeling to be caused in the user.

Moreover, according to the photocosmetic device 100 of the present embodiment, the light transmission member 3 is made of the material that is less likely to absorb the light having the emission wavelength of the light source 22. This can suppress the increase in the temperature of light transmission member 3 caused by the absorption of the light. The uncomfortable feeling to be caused in the user can be reduced accordingly.

### (1.4) Variations of Embodiment 1

Variations of the embodiment 1 will be described. In the following description, the embodiment 1 described above may be referred to as a "basic example (of the embodiment 1)". In the description of the variations, components that are substantially the same as those of a basic aspect may be appropriately omitted. The basic example and the variations may be readily modified depending on a design choice or any other factor, without departing from a true spirit and scope of the present disclosure.

### (1.4.1) First Variation

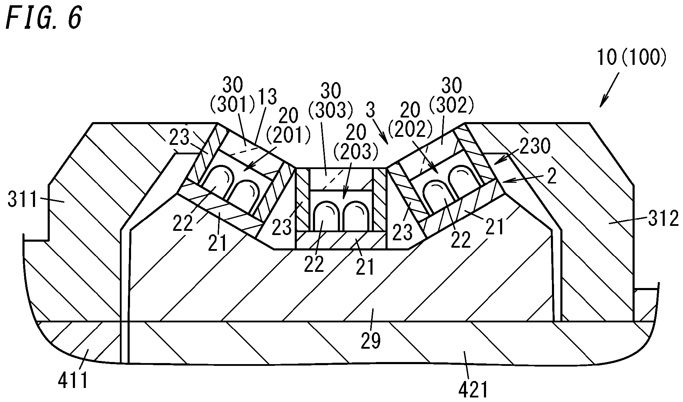

A hair control device 10 (photocosmetic device 100) of the present variation will be described with reference to FIG. 6. The hair control device 10 of the present variation differs from the hair control device 10 of the basic example in a structure of a light blocking part 230.

As shown in FIG. 6, according to the hair control device 10 of the present variation, light blocking members 23 of a plurality of light source units 20, which constitute the light blocking part 230, are in contact with surfaces (where light sources 22 are mounted) of corresponding substrates 21. This can further reduce leakage of light compared with a case where a gap is formed between a light blocking member 23 and a substrate 21.

According to the hair control device 10 of the present variation, the light blocking member 23 having the heat conduction property is in contact with (thermally connected with) the substrate 21. This allows the heat generated in the light source 22 (LEDs 221) to transfer to a holder 31 via the light blocking member 23, which can efficiently facilitate the cooling of the light source 22.

### (1.4.2) Second Variation

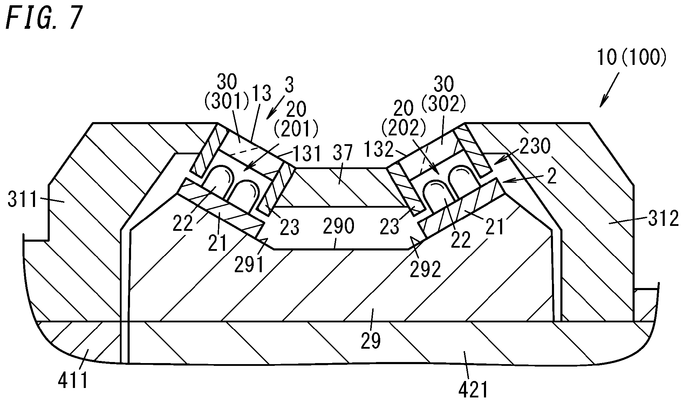

A hair control device 10 (photocosmetic device 100) of the present variation will be described with reference to FIG. 7. The hair control device 10 of the present variation differs from the hair control device 10 of the basic example in a configuration of a light emitting unit 2.

As shown in FIG. 7, according to the hair control device 10 of the present variation, the light emitting unit 2 includes a first light source unit 201 disposed on a first inclined surface 291 and a second light source unit 202 disposed on a second inclined surface 292. According to the hair control device 10 of the present variation, the light emitting unit 2 does not include a third light source unit 203 (see FIG. 3).

According to the hair control device 10 of the present variation, a light transmission member 3 includes, as a plurality of light transmission pieces 30, a first light transmission piece 301 arranged correspondingly to the first light source unit 201 and a second light transmission piece 302 arranged corresponding to the second light source unit 202. According to the hair control device 10 of the present variation, the light transmission member 3 does not include a third light transmission piece 303 (see FIG. 3) corresponding to the third light source unit 203. Between a light blocking member 23 of the first light source unit 201 and a light blocking member 23 of the second light source unit 202, a light blocking plate 37 is disposed. The light blocking plate 37 is made of metal, for example. A plurality of the light blocking members 23 are integrated together with the light blocking plate 37 to form a light blocking part 230.

The hair control device 10 of the present variation also can reduce the uncomfortable feeling caused in the user, as with the hair control device 10 of the basic example.

The number of light source units 20 included in the light emitting unit 2 is not limited to two or three, but may be four or more.

### (1.4.3) Third Variation

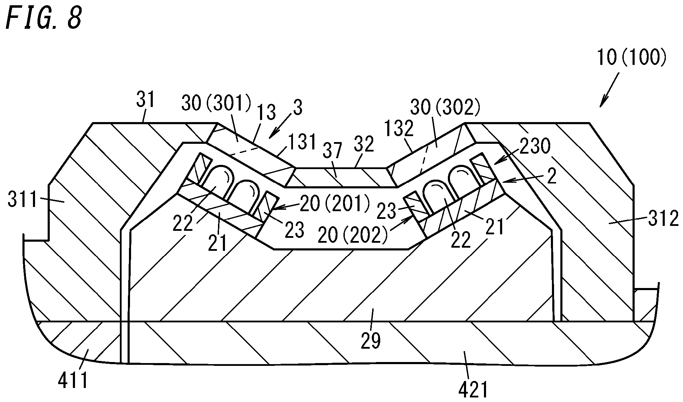

A hair control device 10 (photocosmetic device 100) of the present variation will be described with reference to FIG. 8. The hair control device 10 of the present variation differs from the hair control device 10 of the second variation in a structure of a light blocking part 230.

As shown in FIG. 8, according to the hair control device 10 of the present variation, a light blocking member 23 of a first light source unit 201 is a wall having a rectangular frame shape, and is fixed to a surface of a substrate 21 (where a light source 22 is mounted) without gap therebetween and surrounds the light source 22 (a plurality of LEDs 221). The light blocking member 23 of the first light source unit 201 is apart from a light transmission member 3 (first light transmission piece 301) and a holder 31.

Moreover, according to the hair control device 10 of the present variation, a light blocking member 23 of a second light source unit 202 is a wall having a rectangular frame shape, and is fixed to a surface of a substrate 21 (where a light source 22 is mounted) without gap therebetween and surrounds the light source 22 (a plurality of LEDs 221). The light blocking member 23 of the second light source unit 202 is apart from the light transmission member 3 (second light transmission piece 302) and the holder 31.

According to the hair control device 10 of the present variation, the light transmission member 3 (the first light transmission piece 301 and the second light transmission piece 302) are directly coupled to the holder 31 (a first holding arm 311 and a second holding arm 312) and are held therewith. Between the first light transmission piece 301 and the second light transmission piece 302, a light blocking plate 37 is disposed. According to the hair control device 10 of the present variation, the light blocking plate 37 serves as a partition 32.

The hair control device of the present variation can reduce the leakage of light by the light blocking part 230.

Note that in each of the light source units 20, the light blocking member 23 may cover a side surface of the substrate 21, and further be in contact with a holding member 29.

### (1.4.4) Fourth Variation

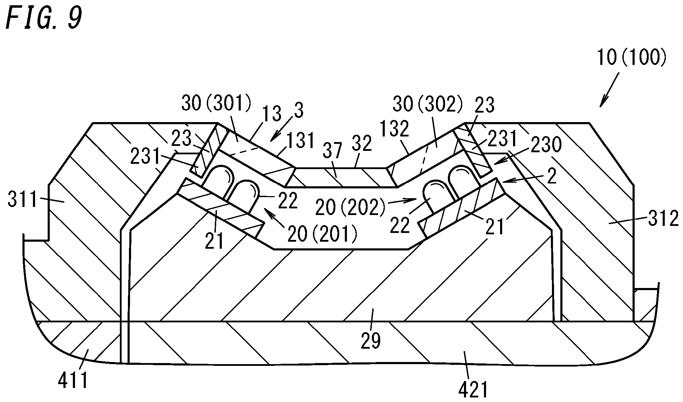

A hair control device 10 (photocosmetic device 100) of the present variation will be described with reference to FIG. 9. The hair control device 10 of the present variation differs from the hair control device 10 of the second variation in a structure of a light blocking part 230.

As shown in FIG. 9, according to the hair control device 10 of the present variation, a light blocking member 23 of a first light source unit 201 includes only an outer wall part 231 that covers an outer (left) side of a light transmission member 3 (first light transmission piece 301). The outer wall part 231 is coupled to and fixed to a first holding arm 311 of a holder 31.

According to the hair control device 10 of the present variation, a light blocking member 23 of a second light source unit 202 includes only an outer wall part 231 that covers an outer (right) side of the light transmission member 3 (second light transmission piece 302). The outer wall part 231 is coupled to and fixed to a second holding arm 312 of the holder 31.

Between the first light transmission piece 301 and the second light transmission piece 302, a light blocking plate 37 is disposed.

The hair control device 10 of the present variation can reduce leakage of light by the light blocking part 230.

### (1.4.5) Fifth Variation

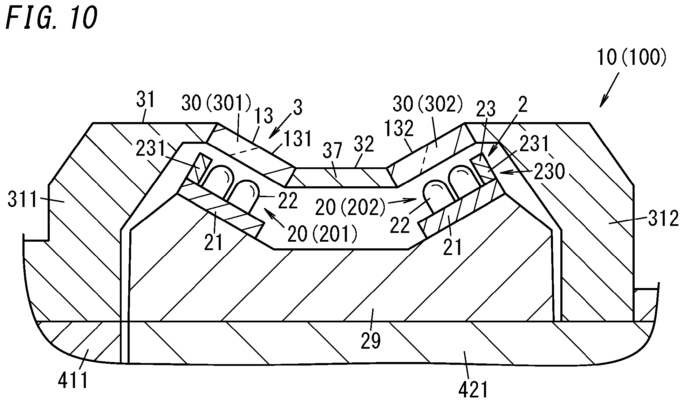

A hair control device 10 (photocosmetic device 100) of the present variation will be described with reference to FIG. 10. The hair control device 10 of the present variation differs from the hair control device 10 of the third variation in a structure of a light blocking part 230.

As shown in FIG. 10, according to the hair control device 10 of the present variation, a light blocking member 23 of a first light source unit 201 includes only an outer wall part 231 disposed on an outer (left) side of a substrate 21. The outer wall part 231 is in contact with and fixed to the substrate 21.

According to the hair control device 10 of the present variation, a light blocking member 23 of a second light source unit 202 includes only an outer wall part 231 disposed on an outer (right) side of a substrate 21. The outer wall part 231 is in contact with and fixed to the substrate 21.

The hair control device 10 of the present variation can reduce leakage of light by the light blocking part 230.

### (1.4.6) Sixth Variation

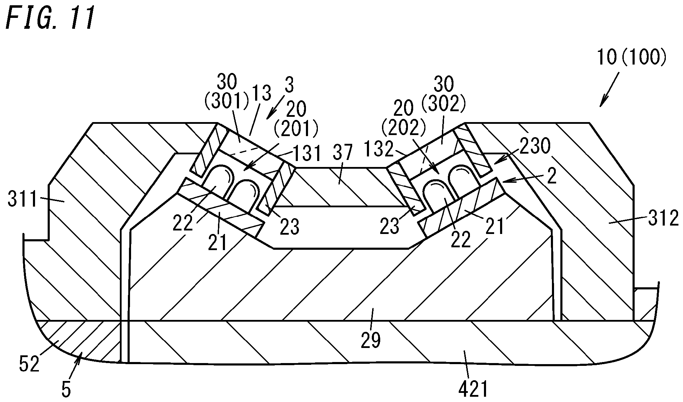

A hair control device 10 (photocosmetic device 100) of the present variation will be described with reference to FIG. 11. The hair control device 10 of the present variation differs from the hair control device 10 of the second variation in including a Peltier device 52 in place of a cooling fan 51.

As shown in FIG. 11, according to the hair control device 10 of the present variation, the Peltier device 52 is disposed at a position where a first coupler 411 is disposed in the hair control device 10 of the second variation. The Peltier device 52 is disposed such that the "cold" side is directed forward (and on which a first holding arm 311 is disposed).

According to the hair control device 10 of the present variation, the Peltier device 52 can cool a light transmission member 3 (a first light transmission piece 301 and a second light transmission piece 302) to facilitate the cooling of the skin 92 that is in contact with the light transmission member 3. The uncomfortable feeling to be caused in a user can be reduced.

Alternatively, the cooler 5 may include both the cooling fan 51 and the Peltier device 52. The cooling efficiency of the light transmission member 3 and the light emitting unit 2 can be further improved.

The Peltier device 52 may be provided to any of the hair control devices 10 of the basic example, the first variation, and the third to fifth variations.

### (1.4.7) Seventh Variation

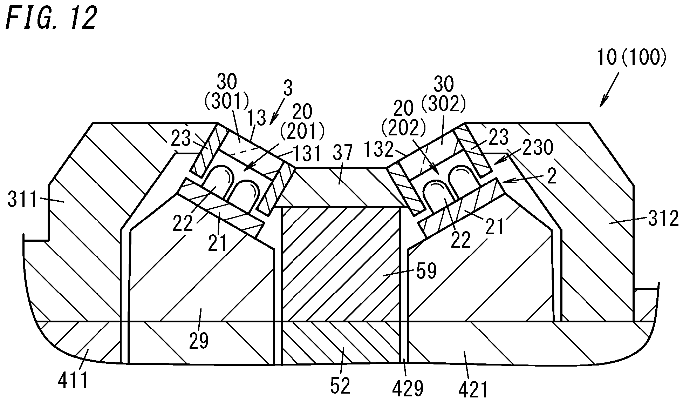

A hair control device 10 (photocosmetic device 100) of the present variation will be described with reference to FIG. 12. The hair control device 10 of the present variation differs from the hair control device 10 of the sixth variation in a location of a Peltier device 52.

As shown in FIG. 12, according to the hair control device 10 of the present variation, the Peltier device 52 is disposed in an inside of a hole 429 formed in a center of a second coupler 421. The Peltier device 52 is connected to a light blocking plate 37 via a coupling part 59.

According to the hair control device 10 of the present variation, a light transmission member 3 (a first light transmission piece 301 and a second light transmission piece 302) can be cooled by the Peltier device 52 to facilitate the cooling of the skin 92 in contact with the light transmission member 3. The uncomfortable feeling to be caused in a user can be reduced.

### (1.4.8) Eighth Variation

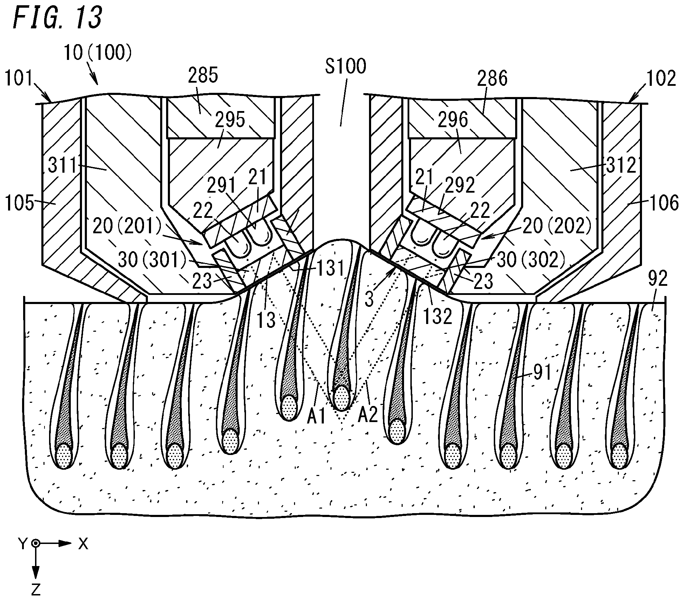

A hair control device 10 (photocosmetic device 100) of the present variation will be described with reference to FIG. 13. The hair control device 10 of the present variation differs from the hair control device 10 of the second variation in a configuration of a housing 1 (second case 12), a light emitting unit 2, a light transmission member 3 etc.

As shown in FIG. 13, according to the hair control device 10 of the present variation, the light emitting unit 2 includes, as a plurality of light source units 20, a first light source unit 201 and a second light source unit 202. Moreover, according to the hair control device 10 of the present variation, the housing 1 (specifically, the second case 12) includes a first housing part 105 in which the first light source unit 201 is housed and a second housing part 106 in which the second light source unit 202 is housed. According to the hair control device 10 of the present variation, a light exit surface 13 having a recessed shape includes: a first inclined surface 131 allowing light of a light source 22 of the first light source unit 201 to be emitted to an outside of the first housing part 105; and a second inclined surface 132 allowing light of a light source 22 of the second light source unit 202 to be emitted to an outside of the second housing part 106. The first housing part 105 and the second housing part 106 are disposed separately (separated) from each other in a direction in which the first light source unit 201 and the second light source unit 202 are arranged.

Specifically, the hair control device 10 of the present variation includes a first light source block 101 and a second light source block 102.

The first light source block 101 includes a first supporting plate 285, a first holding member 295, the first light source unit 201, a first light transmission piece 301, a first holding arm 311, and the first housing part 105. The first supporting plate 285 has a plate shape and extends along the Y-axis. The first holding member 295 has an inclined base shape extending along the Y-axis, and is supported on the first supporting plate 285. The first holding member 295 has a first inclined surface 291 inclined toward the second light source block 102. A substrate 21 of the first light source unit 201 is disposed on the first inclined surface 291. The first light transmission piece 301 is held by the first holding arm 311 to be positioned on an optical axis A1 of the first light source unit 201. A front surface of the first light transmission piece 301 serves as the first inclined surface 131 of the light exit surface 13. The first housing part 105 has a peripheral wall shape and surrounds the first holding member 295, the first light source unit 201 and the first holding arm 311.

The second light source block 102 includes a second supporting plate 286, a second holding member 296, the second light source unit 202, a second light transmission piece 302, a second holding arm 312, and the second housing part 106. The second supporting plate 286 has a plate shape and extends along the Y-axis. The second holding member 296 has an inclined base shape extending along the Y-axis, and is supported on the second supporting plate 286. The second holding member 296 has a second inclined surface 292 inclined toward the first light source block 101. A substrate 21 of the second light source unit 202 is disposed on the second inclined surface 292. The second light transmission piece 302 is held by the second holding arm 312 to be positioned on an optical axis A2 of the second light source unit 202. A front surface of the second light transmission piece 302 serves as the second inclined surface 132 of the light exit surface 13. The second housing part 106 has a peripheral wall shape and surrounds the second holding member 296, the second light source unit 202 and the second holding arm 312.

As shown in FIG. 13, in the hair control device 10 of the present variation, the first light transmission piece 301 and the second light transmission piece 302 are disposed separately from each other in the direction along the X-axis (in which the first light source unit 201 and the second light source unit 202 are arranged) and disconnected from each other. As shown in FIG. 13, there is a space S100 between the first housing part 105 and the second housing part 106. According to the hair control device 10 of the present variation, therefore, the space S 100 is formed between the first light source block 101 and the second light source block 102.

According to the hair control device 10 of the present variation, the housing 1 is divided into the first housing part 105 in which the first light source unit 201 is housed and the second housing part 106 in which the second light source unit 202 is housed. This can reduce light (leakage light) traveling from a light source 22 of one of the first light source unit 201 and the second light source unit 202 toward a light source 22 of the other.

According to the hair control device 10 of the present variation, the space S100 is formed between the first housing part 105 and the second housing part 106 as described above. According to this structure, a part of the skin 92 is introduced into the space S 100 when the skin 92 is pressed against the light exit surface 13 of the photocosmetic device 100. As a result, the skin 92 is less likely to be pushed back by the light exit surface 13, and thus the skin 92 can be further closely in contact with the light exit surface 13.

Note that the first housing part 105 and the second housing part 106 may be in contact with each other (i.e., a distance therebetween may be 0).

### (1.4.9) Ninth Variation

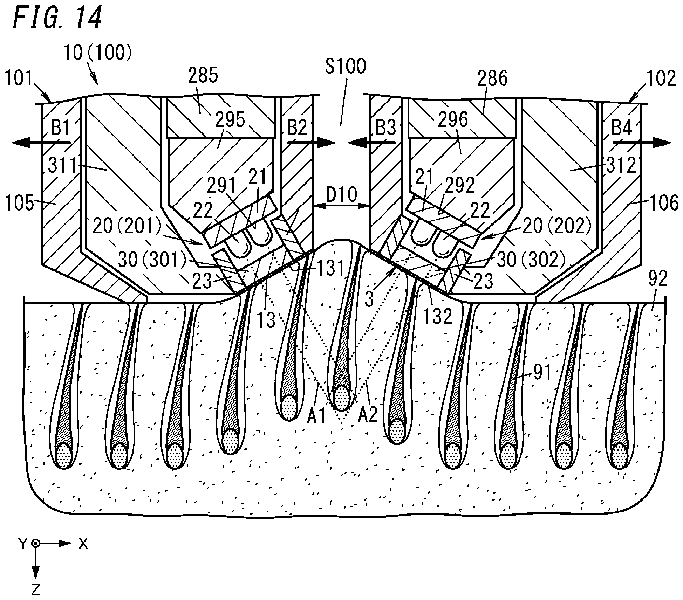

A hair control device 10 (photocosmetic device 100) of the present variation will be described with reference to FIG. 14. The hair control device 10 of the present variation differs from the hair control device 10 of the eighth variation in that at least one of a first housing part 105 or a second housing part 106 (i.e., at least one of a first light source block 101 or a second light source block 102) is movable in a direction (direction along the X-axis) in which the first light source unit 201 and the second light source unit 202 are arranged.

According to the hair control device 10 of the present variation, as indicated by imaginal arrows B1 to B4 shown in FIG. 14, each of the first light source block 101 and the second light source block 102 is movable along the X-axis. The hair control device 10 of the present variation includes a moving mechanism configured to move the first light source block 101 and the second light source block 102 in the direction in which the first light source block 101 and the second light source block 102 are arranged. In an example, the moving mechanism may include a hinge mechanism pivotably supporting the first light source block 101 and the second light source block 102 around a rotation shaft. In another example, the moving mechanism may include a sliding mechanism configured to move (slide) the first light source block 101 and the second light source block 102 along the X-axis. The moving mechanism may be configured to be manually operated by the operator to move the first light source block 101 and the second light source block 102. Additionally or alternatively, the moving mechanism may include a motor configured to be driven in response to an operation given to a button by the operator to move the first light source block 101 and the second light source block 102. Either one of the first light source block 101 or the second light source block 102 may be movable, or else both of the first light source block 101 and the second light source block 102 may be movable, whichever is appropriate.

The hair control device 10 of the present variation can pinch the skin 92 between the first light source block 101 and the second light source block 102 by, e.g., firstly the light exit surface 13 being brought into contact with the skin 92 in a state where the first light source block 101 and the second light source block 102 are apart from each other and a distance D10 therebetween are set to be relatively large, and then the first light source block 101 and/or the second light source block 102 being moved to minimize the distance D10 between the first light source block 101 and the second light source block 102. This allows the skin 92 to be further closely in contact with the light exit surface 13.

The hair control device 10 of the present variation may include a stopper mechanism configured to keep the distance D10 between the first housing part 105 and the second housing part 106 greater than 0 even when the first light source block 101 and the second light source block 102 are approached closest each other. That is, the hair control device 10 may further include the stopper mechanism configured to restrict the movement of at least one of the first housing part 105 or the second housing part 106 such that a gap remains between the first housing part 105 and the second housing part 106 even in a state where the first housing part 105 and the second housing part 106 are approached closest each other. The stopper mechanism may include a protrusion protruding from one of the first housing part 105 and the second housing part 106 toward the other. The stopper mechanism may include a spring provided in the hinge mechanism and configured to give an elastic force to move the first housing part 105 and the second housing part 106 away from each other. The stopper mechanism may include a structure that restricts the movable range of the sliding mechanism. The stopper mechanism can prevent the skin 92 from being tightly caught between the first housing part 105 and the second housing part 106. The uncomfortable feeling to be caused in a user can be reduced. However, this should not be construed as limiting. Alternatively, the hair control device 10 may be configured such that the first housing part 105 and the second housing part 106 come in contact with each other without gap therebetween when the first light source block 101 and the second light source block 102 are approached closest each other.

According to the present variation, the light exit surface 13 can be in contact with the skin 92 more closely, which can increase the treatment efficiency for treating the skin 92.

### (1.4.10) Other Variations

In one variation, a housing 1 does not have to house therein an entire of a plurality of light source units 20. A housing 1 may house a light source unit 20 with a part of a light source unit 20 (e.g., a part of a substrate 21) protruding from the housing 1, for example.

In one variation, a photocosmetic device 100 may not include a light blocking part 230.

In one variation, a light source unit 20 may include an optical member configured to change the direction of the optical path of light of the light source 22 by the reflection or the refraction, such as a mirror, a half mirror, or a prism. In this case, an optical axis A10 of the light source unit 20 may not coincide with an optical axis of the light source 22. For example, a plurality of light source units 20 may be arranged such that the optical axes of the light sources 22 are parallel to one another. Even in this case, the optical axes A10 of the plurality of light source units 20 may be able to cross one another on an outside of the light exit surface 13 of the housing 1, by means of the optical member that reflects or refracts the light of the light source 22 in each of the plurality of light source units 20.

In one variation, a light transmission member 3 (light transmission piece 30) may have a shape serving as a lens.

In one variation, a photocosmetic device 100 may not include a light transmission member 3.

In one variation, an operations unit 6 may include a switch provided on a side surface of a first case 11, for example.

In one variation, a photocosmetic device 100 may further include a suction mechanism configured to suck the skin 92.

### (Embodiment 2)

### (2.1) Overview of Embodiment 2

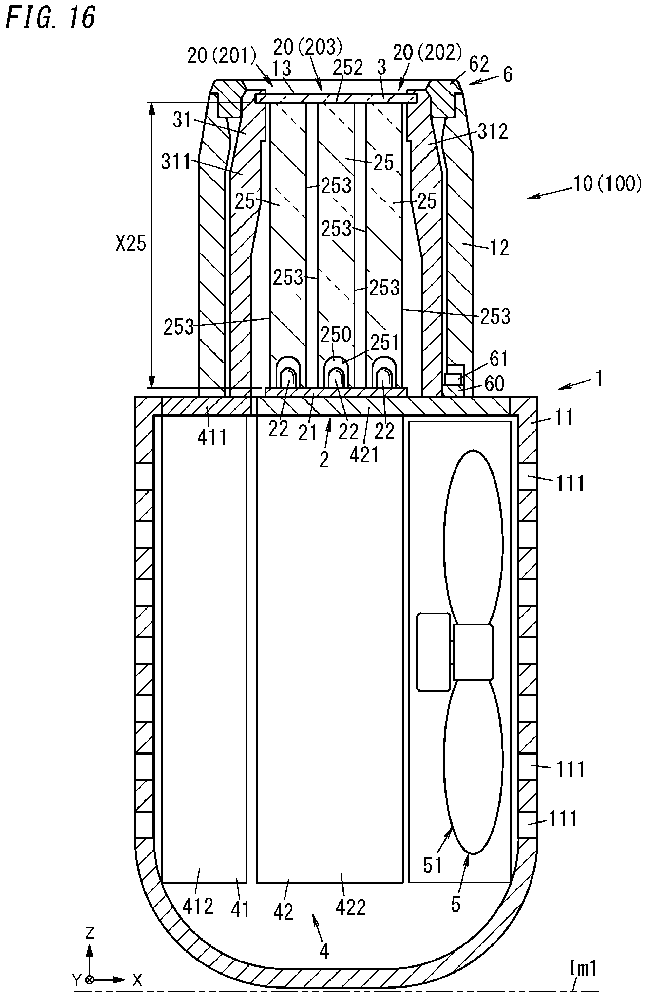

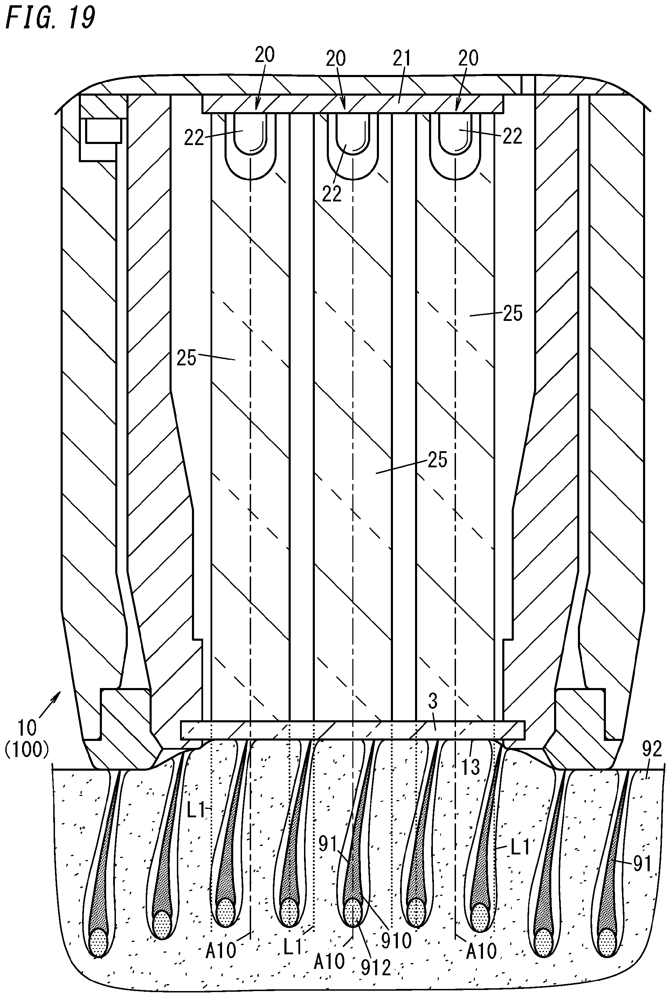

A photocosmetic device 100 of the present embodiment (see FIGS. 16 to 18) is a device for illuminating light to a skin 92 (see FIG. 19) to cause absorption of the light in the skin 92 to treat the skin 92 with the function of the absorbed light.

Incidentally, according to Patent Literature 1, the hair removal device includes a head section for emission of treatment light pulses and a handle section for holding of the hair removal device by a user's hand. A control element is arranged at the handle section for at least switching ON/OFF the hair removal device. The head section has an exit opening through which the treatment light pulses will be emitted during operation. A substrate with a plurality of LED dies mounted on the substrate is arranged closely behind the exit opening or the substrate is arranged with a certain distance of about or less than 10 mm to the exit opening inside of the head section. An exit window made from a material being essentially transparent to the light to be emitted by the LED dies covers the exit opening. A substrate mounted LED die array is mounted on a heat sink to convey away excess heat generated by the LED dies in operation. A fan is arranged close to the heat sink to support the heat dissipation away from the heat sink.

In the hair removal device described in Patent Literature 1, the substrate and the heat sink are arranged closely behind the exit opening or with a distance of about or less than 10 mm in the head section. This structure requires a space enough to arrange the heat sink near the exit opening, which increase the size of the structure around the exit opening. As a result, this structure may disturb the user to see the position of the skin to be illuminated by the treatment light, which may worsen the handleability of the device.

In order for the efficient treatment (e.g., facilitation of falling off of the hair 91 for hair control or hair removal) of the skin 92 with the light irradiation, it is desirable that sufficient amount of light of the photocosmetic device 100 reaches a part in the skin 92 desired to absorb the light (a part around the hair matrix 912 of the hair 910 to be removed; see FIG. 19). For this purpose, it is preferable that the ray of the light of the photocosmetic device 100 strikes a desired region (e.g., a region with the hair 910 to be removed) of the surface of the skin 92.

In this regard, according to the photocosmetic device 100 of the present embodiment, each of a plurality of light source units 20 includes a light guide member 25 configured to guide light emitted from the light source 22 toward a light exit surface 13, as shown in FIG. 16. This allows the light source 22 to be located apart from the light exit surface 13 inside the housing 1, which can simplify the structure around the light exit surface 13 of the photocosmetic device 100. The size of the part of the photocosmetic device 100 around the light exit surface 13 can be reduced accordingly. As a result, the operator who operates the photocosmetic device 100 can see the position to be irradiated with the treatment light without being disturbed by the part of the photocosmetic device 100 around the light exit surface 13. The light exit surface 13 thus can be easily positioned to a desired position of the skin 92. The handleability of the device can be improved.

### (2.2) Details of Embodiment 2

As shown in FIG. 16, the hair control device 10 includes a housing 1, the light exit surface 13, a light emitting unit 2, a light transmission member 3, a heat dissipation part 4, a cooler 5, an operations unit 6, a controller 71 (see FIG. 5) and a power source 72 (see FIG. 5). The cooler 5, the operations unit 6, the controller 71 and the power source 72 have substantially same configurations as those of the hair control device 10 of the embodiment 1 and thus the explanations thereof will be omitted.

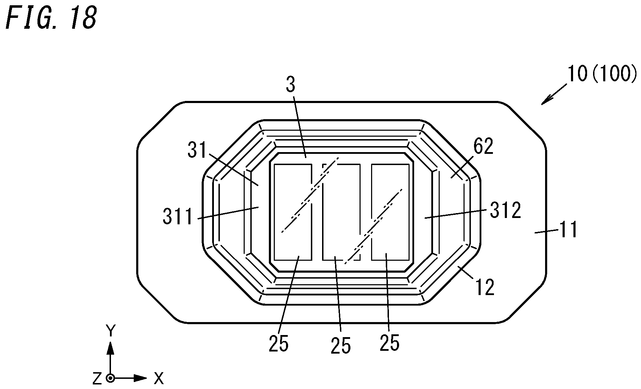

The housing 1 includes a first case 11 and a second case 12. The first case 11 has a tubular shape opened forward. When viewed from the front side, a dimension of a front opening of the first case 11 measured along the X-axis is smaller than a dimension of the outer periphery of the first case 11 measured along the X-axis. The second case 12 protrudes from the first case 11 along a first axis (Z-axis). As shown in FIG. 16, the maximum dimension of the first case 11 measured along a second axis (X-axis) perpendicular to the first axis (Z-axis) is greater than the maximum dimension of the second case 12 measured along the second axis. As shown in FIG. 18, an area of a first projection region is greater than an area of a second projection region. The first projection region is a region enclosed by a first projection line obtained by projecting a circumference of the first case 11 on an imaginary plane Im1 (see FIG. 16) perpendicular to the first axis. The second projection region is a region enclosed by a second projection line obtained by projecting a circumference of the second case 12 on the imaginary plane Im1.

According to the hair control device 10 of the present embodiment, the second case 12 has a far end (front end) away from the first case 11 in the first axis (Z-axis). The light exit surface 13 is located at the far end of the second case 12.

As shown in FIG. 16, the light emitting unit 2 includes the plurality of (three, in the embodiment) light source units 20 (a first light source unit 201 to a third light source unit 203), and a substrate 21.

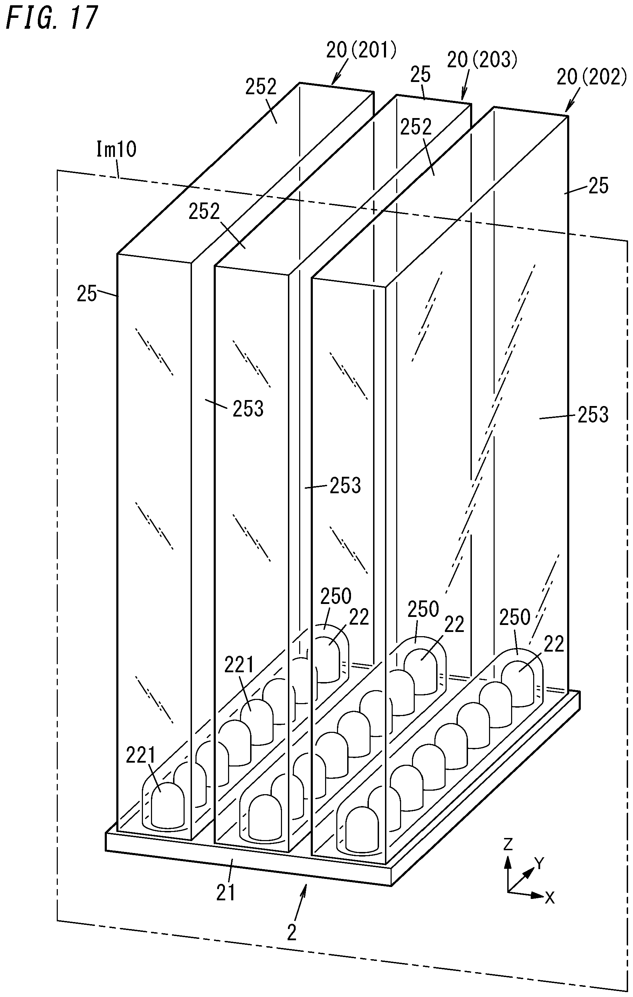

The substrate 21 has a rectangular plate shape. As shown in FIG. 17, the substrate 21 has a plate shape extending along the X-axis and the Y-axis.

As shown in FIG. 16, each of the plurality of light source units 20 includes a light source 22 and a light guide member 25.

The light source 22 is mounted on the substrate 21. The light source 22 is mounted on a surface on the front side of the substrate 21. The light sources 22 of the plurality of light source units 20 are mounted on the substrate 21 in common. The light sources 22 of the plurality of light source units 20 are arranged along the second axis (X-axis).

As shown in FIG. 17, the light source 22 includes a plurality of LEDs 221.

As shown in FIG. 17, in each of the plurality of light source units 20, the plurality of LEDs 221 are arranged in a line along one axis (Y-axis). Specifically, the light source 22 includes eight-pieces of LEDs 221, for example. In each light source unit 20, the eight-pieces of LEDs 221 are arranged in a line along the one axis (Y-axis).

Each of the plurality of LEDs 221 is mounted on the substrate 21 such that it exhibits a light distribution property where the direction of the peak intensity is aligned with the normal direction of the substrate 21. In other words, each of the plurality of LEDs 221 has an optical axis along the normal direction of the substrate 21. In each light source unit 20, the light source 22 has an optical axis along the normal direction of the substrate 21. According to the present embodiment, the feature "optical axis of the light source 22 including the plurality of LEDs 221" indicates an imaginary plane (planar axis) including the optical axes of the plurality of LEDs 221.

As shown in FIG. 16, the light guide member 25 is disposed in front of the light source 22. In each of the plurality of light source units 20, the light guide member 25 is configured to guide the light emitted from a corresponding light source 22 toward the light exit surface 13.

The material of the light guide member 25 includes quartz or sapphire, for example. The light guide member 25 may be made from resin, such as polymer resin. The polymer resin used for the material of the light guide member 25 may be Polymethylmethacrylate, for example.

The light guide member 25 has a plate shape extending along the Y-axis and the Z-axis. The light guide member 25 has a thickness along the X-axis. For example, the light guide member 25 has a length X25 along the first axis (Z-axis) falling within the range equal to or greater than 10 mm and equal to or smaller than 50 mm. The length X25 of the light guide member 25 may be equal to or greater than 20 mm, or may be equal to or greater than 30 mm.

In each light source unit 20, the light guide member 25 of a single member is disposed to cover the front side of the plurality of (eight-pieces of) LEDs 221 of the light source 22. The light guide member 25 has a recess 250 in a rear surface (surface facing the light source 22). The light source 22 is disposed inside the recess 250. As shown in FIG. 17, the recess 250 has a groove shape extending along the Y-axis. The plurality of (eight-pieces of) LEDs 221 are arranged in the recess 250. A rear end of the light guide member 25 (part of a rear surface other than the recess 250) is fixed to a front surface of the substrate 21. The recess 250 of the light guide member 25 and the light source 22 (LEDs 221) are not contacted with each other, and a gap is provided between them.

As shown in FIG. 16, in each of the plurality of light source units 20, the light guide member 25 has a light incident surface 251 and a light emerging surface 252.

The light incident surface 251 is a surface of the light guide member 25 through which the light of the light source 22 enters. In the embodiment, the light incident surface 251 is constituted by a surface of the recess 250 of the light guide member 25. The light incident surface 251 faces the light source 22.

As shown in FIG. 16, in the hair control device 10 of the present embodiment, the light incident surface 251 has a lens surface. As shown in FIG. 17, the light incident surface 251 has a shape of a cylindrical concave lens surface. The light incident surface 251 having the lens surface can appropriately adjust a shape of the light of the light source 22 when the light enters the light guide member 25 through the light incident surface 251.

The light emerging surface 252 is a surface of the light guide member 25 from which the light entered through the light incident surface 251 is emerged to the light exit surface 13. As shown in FIG. 16, the light emerging surface 252 faces the light exit surface 13. The light emerging surface 252 adjacently faces the light transmission member 3 having the light exit surface 13. In the embodiment, the light emerging surface 252 is in contact with the light transmission member 3.

As shown in FIG. 17, in the hair control device 10 of the present embodiment, the light emerging surface 252 has a flat plane shape. The light emerging surface 252 is in parallel to a rear surface of the light transmission member 3. This can reduce the loss (such as the loss caused in the reflection) caused when the light emitted from the light emerging surface 252 of the light guide member 25 enters the light transmission member 3 through the rear surface of the light transmission member 3.

The light guide member 25 has a pair of side surfaces 253 that intersect with a thickness axis (along the X-axis) of the light guide member 25. The pair of side surfaces 253 intersect with the light emerging surface 252. In the embodiment, each of the pair of side surfaces 253 intersects with the light emerging surface 252 at right angles. The pair of side surfaces 253 are parallel to each other.

The light guide member 25 has the constant cross sectional shapes along one axis (axis along which the plurality of LEDs 221 are arranged; Y-axis). In a region facing the light source 22, cross sectional shapes of the light guide member 25 in any imaginary planes Im 10 (imaginary planes Im10 intersecting with each of the light incident surface 251, the light emerging surface 252 and the pair of side surfaces 253; see FIG. 17) perpendicular to the one axis are same as each other.

In the hair control device 10 of the present embodiment, each of the plurality of light source units 20 has an optical axis A10 (see FIG. 19). In the hair control device 10 of the present embodiment, the optical axis A10 of the light source unit 20 coincides with the optical axis of the light source 22. The optical axis A10 of the light source unit 20 is along the normal direction of the light emerging surface 252 of the light guide member 25. In the hair control device 10 of the present embodiment, the optical axes A10 of the plurality of light source units 20 are parallel to one another.

The light guide members 25 of the first light source unit 201 to the third light source unit 203 have the common shapes to each other.

As shown in FIG. 16, according to the plurality of light source units 20, the light sources 22 are out of contact and apart from each other in the axis (i.e., X-axis) along which the plurality of light source units 20 are arranged. A distance between the light sources 22 of adjacent two light source units 20 (a distance between LEDs 221 of the adjacent two light source units 20) may be greater than a dimension of one LED 221, for example. Moreover, according to the plurality of light source units 20, the light guide members 25 are out of contact and apart from each other in the axis (i.e., X-axis) along which the plurality of light source units 20 are arranged. A distance between the light guide members 25 of adjacent two light source units 20 may be smaller than a dimension of one LED 221, for example. In short, the light sources 22 of the plurality of light source units 20 are apart from one another, and a plurality of light guide members 25 are apart from one another. This can reduce the overlap of the rays of the light L1 (see FIG. 19) of the plurality of light source units 20 on the light exit surface 13.

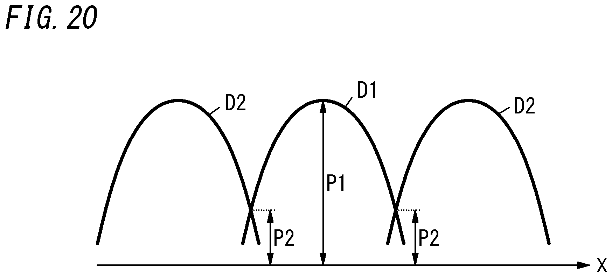

According to the hair control device 10 of the present embodiment, the light sources 22 of the plurality of light source units 20 are arranged to be apart from one another such that the rays of the light L1 of adjacent two light source units 20 do not substantially overlap each other on the light exit surface 13. According to the present embodiment, the feature "the rays of the light L1 of two light source units 20 do not substantially overlap each other on the light exit surface 13" indicates, for example, a condition that a ratio of intensities (P1/P2) is equal to or greater than 3 (see FIG. 20). The intensity P1 is an intensity at a peak in the intensity distribution D1 of the light L1 emitted from an interested one light source unit 20 of the two light source units 20. The intensity P2 is an intensity of the light L1 at a point where the intensity in the intensity distribution D1 of the light L1 emitted from the interested one light source unit 20 is the same as the intensity in the intensity distribution D2 of the light L1 emitted from the other light source unit 20. The ratio of intensities (P1/P2) defined above may be equal to or greater than 5, or equal to or greater than 10, or equal to or greater than 20.

When the overlap of the rays of the light L1 of the plurality of light source units 20 is small in the light exit surface 13, the intensity of light is relatively low in an entire of the surface of the skin 92 while the surface of the skin 92 is in contact with the light exit surface 13. This can suppress the increase in the temperature of the skin 92 around the surface layer caused by the absorption of the light. The uncomfortable feeling to be caused in a user can be reduced accordingly.

The light transmission member 3 is disposed in front of the light emitting unit 2. The light transmission member 3 is located in front of the light emerging surfaces 252 of the light guide members 25 of the plurality of light source units 20. In the present embodiment, the light transmission member 3 of a single plate covers a whole of the plurality of light source units 20 from the front side. The light transmission member 3 allows the light of the plurality of light source units 20 to pass through.

As shown in FIG. 19, the hair control device 10 of the present embodiment is adapted to be used in a state where the surface of the light transmission member 3 (i.e., the light exit surface 13) is in contact with the surface of the skin 92.

As shown in FIGS. 16, 18, a holder 31 has a hollow tubular shape extending along the Z-axis. The light transmission member 3 is held on the front end (an end on the positive side in the Z-axis) of the holder 31.

As shown in FIG. 16, the holder 31 includes a left side wall 311 disposed on a front surface of a first coupler 411 of a first heat dissipation part 41 of the heat dissipation part 4. A first heat dissipation member 412 of the first heat dissipation part 41 is thermally connected to the light transmission member 3 via the first coupler 411 and the holder 31. The heat in the light transmission member 3 is transferred to a plurality of first heat dissipation fins of the first heat dissipation member 412 via the holder 31 and the first coupler 411, and then is emitted to the outside from the plurality of first heat dissipation fins (through air vents 111 of the first case 11, for example).

The holder 31 further includes a right side wall 312 disposed on a front surface of a second coupler 421 of a second heat dissipation part 42 of the heat dissipation part 4. A second heat dissipation member 422 of the second heat dissipation part 42 is thermally connected to the substrate 21 via the second coupler 421. The heat generated in the light sources 22 of the light source units 20 is transferred to a plurality of second heat dissipation fins of the second heat dissipation member 422 via the second coupler 421, and then is emitted to the outside from the plurality of second heat dissipation fins (through the air vents 111 of the first case 11, for example).

The second heat dissipation member 422 is also thermally connected to the light transmission member 3, via the second coupler 421 and the holder 31. The heat in the light transmission member 3 is transferred to the plurality of second heat dissipation fins of the second heat dissipation member 422 via the holder 31 and the second coupler 421, and then is emitted to the outside from the plurality of second heat dissipation fins (through the air vents 111 of the first case 11, for example).

### (2.3) Advantage in Embodiment 2

Advantages in the photocosmetic device 100 of the present embodiment will be described while comparing with a photocosmetic device 300 of a second comparative example.

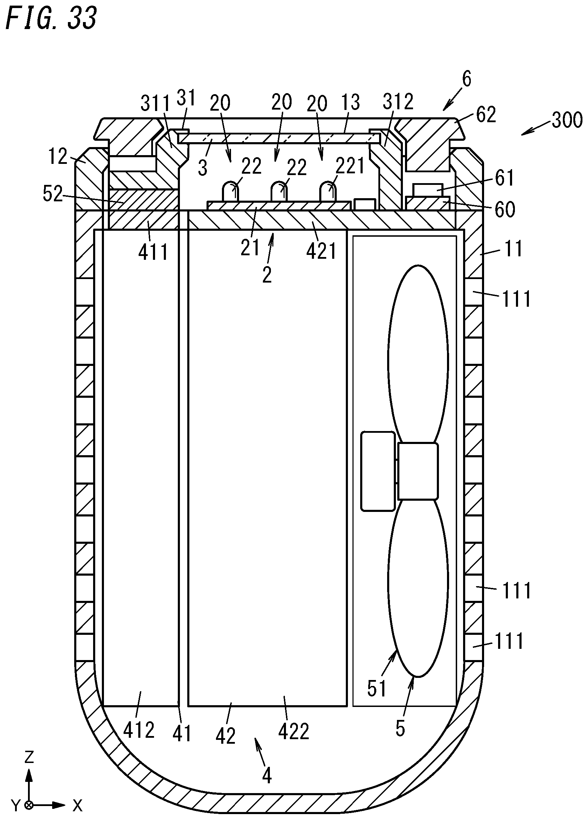

As shown in FIG. 33, in the photocosmetic device 300 of the second comparative example, each light source unit 20 does not include a light guide member 25, but a light source 22 (LEDs 221) directly faces a light transmission member 3. In the photocosmetic device 300 of the second comparative example, dimensions measured along the Z-axis of a holder 31 and a second case 12 are smaller than those in the photocosmetic device 100 of the embodiment. According to the photocosmetic device 300 of the second comparative example, a distance along the Z-axis between the light source 22 and the light transmission member 3 may be smaller than 10 mm, for example.

According to the photocosmetic device 300 of the second comparative example, a Peltier device 52 is disposed between a left side wall 311 of the holder 31 and a first coupler 411. The Peltier device 52 is disposed such that the "cold" side is directed forward (and on which the holder 31 is disposed). According to the photocosmetic device 300 of the second comparative example, the distance between the light source 22 and the light transmission member 3 is comparatively small, and thus the hot gas (hot air) heated by the heat generated in the light source 22 can easily reach the light transmission member 3. The photocosmetic device 300 of the second comparative example may be configured to cool the light transmission member 3 by the Peltier device 52 in order to suppress the increase in the temperature of the light transmission member 3.

According to the photocosmetic device 300 of the second comparative example, each of the plurality of light source units 20 (light sources 22) emits a ray of light having a certain degree of width (intensity distribution) in the X-axis. According to the photocosmetic device 300 of the second comparative example, in order for the light of the plurality of light source units 20 to be received by the light transmission member 3 sufficiently, the light transmission member 3 has a dimension along the X-axis greater than that of the photocosmetic device 100 of the embodiment.

As described above, in the photocosmetic device 300 of the second comparative example, various components (including the light source 22, the Peltier device 52, and the heat dissipation part 4) are arranged near the light transmission member 3 including the light exit surface 13. Moreover, according to the photocosmetic device 300 of the second comparative example, the light transmission member 3 has the dimension along the X-axis greater than that in the photocosmetic device 100 of the embodiment. As a result, a maximum dimension along the X-axis of the second case 12 of the photocosmetic device 300 according to the second comparative example is greater than that of the photocosmetic device 100 according to the embodiment. Specifically, according to the photocosmetic device 300 of the second comparative example, the maximum dimension along the X-axis of the second case 12 is substantially equal to a maximum dimension along the X-axis of the first case 11.

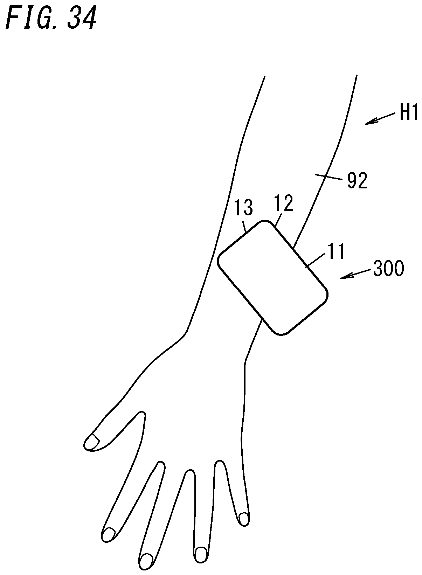

As schematically shown in FIG. 34, the photocosmetic device 300 of the second comparative example is adapted to be used in a state where the light exit surface 13 faces the skin 92 of the user H1. However, according to the photocosmetic device 300 of the second comparative example, the maximum dimension of the second case 12 measured along the X-axis is comparatively large as described above. It is therefore difficult to see the position of the light exit surface 13 (the light transmission member 3) located in the front surface of the second case 12 when the photocosmetic device 300 is viewed laterally (e.g., viewed in the direction along the X-axis). It is therefore difficult for the operator to locate the light exit surface 13 on a desired region of the skin 92.

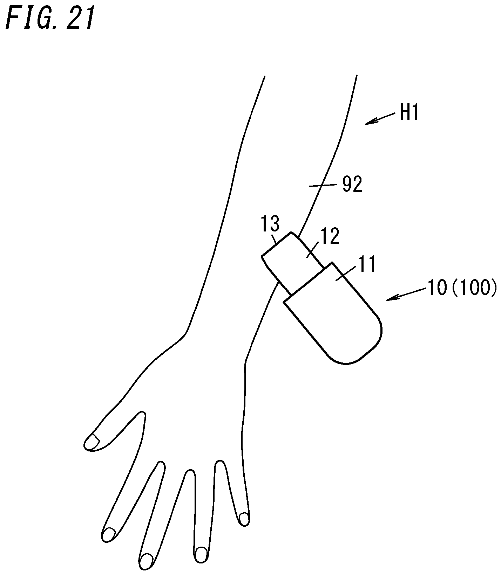

Compared to this, according to the photocosmetic device 100 of the present embodiment, the light source units 20 includes the light guide members 25. A distance between the light source 22 and the light transmission member 3 can be increased by means of the light guide member 25. According to this, the dimension of the front end of the second case 12 measured along the X-axis can be minimized up to the dimension of the light exit surface 13 along the X-axis, for example. This allows the operator who operates the photocosmetic device 100 to easily locate the light exit surface 13 on a desired region of the skin 92 when the photocosmetic device 100 is used in a state where the light exit surface 13 faces the skin 92 of the user H1 (see FIG. 21). The photocosmetic device 100 of the present embodiment thus can have the improved handleability.

Moreover, the light guide member 25 guides the light of the light source 22 to the light transmission member 3, which can reduce the dimension along the X-axis of the light transmission member 3. Moreover, since the light guide member 25 guides the light of the light source 22 to the light transmission member 3, the rays of the light L1 emitted from the plurality of light source units 20 are less overlapped one another on the light exit surface 13. Moreover, since the light emerging surface 252 of the light guide member 25 faces the light transmission member 3, it is easy to grasp a region in the light exit surface 13 from which the light of the light source units 20 is emitted. This can improve the handleability of the device.

According to the photocosmetic device 100 of the present embodiment, the light guide member 25 has the length along the Z-axis falling within the range equal to or greater than 10 mm and equal to or smaller than 50 mm. With regard to the length of the light guide member 25, the visibility of the light exit surface 13 can be improved according to the increase in the length of the light guide member 25 (since the light transmission member 3 can be more apart from the light source 22). On the other hand, the loss of the light emitted from the light source 22 and reaching the light transmission member 3 can be reduced as the length of the light guide member 25 decreases. The length of the light guide member 25 may be appropriately determined in consideration with the visibility and the loss of the light.

### (2.4) Variations of Embodiment 2

Variations of the embodiment 2 will be described. In the following description, the embodiment 2 described above may be referred to as a "basic example (of the embodiment 2)". In the description of the variations, components that are substantially the same as those of a basic aspect may be appropriately omitted. The basic example and the variations may be readily modified depending on a design choice or any other factor, without departing from a true spirit and scope of the present disclosure.

### (2.4.1) First Variation

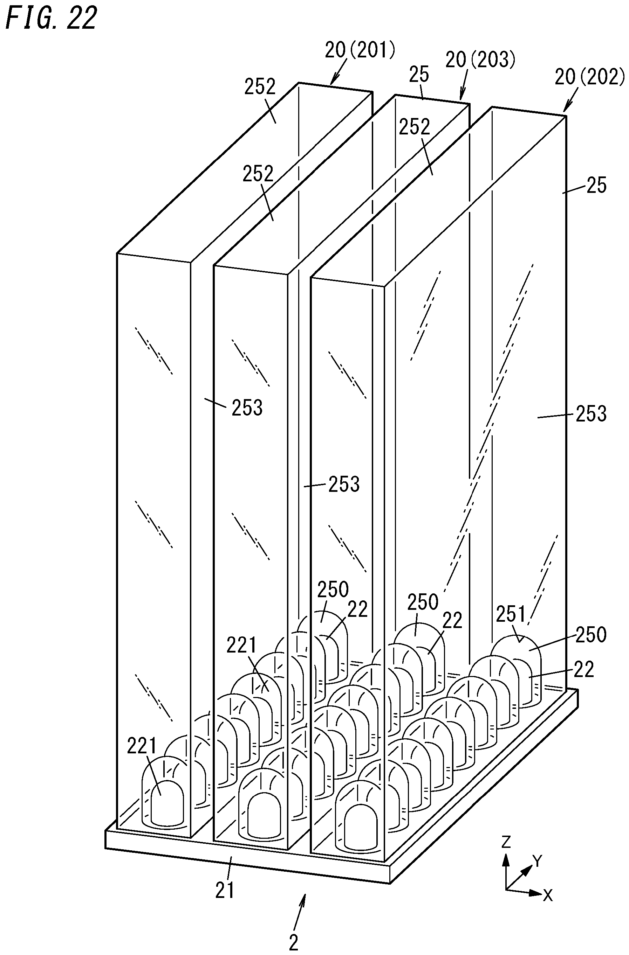

A hair control device 10 (photocosmetic device 100) of the present variation will be described with reference to FIG. 22. The hair control device 10 of the present variation differs from the hair control device 10 of the basic example in a structure of a light guide member 25.

As shown in FIG. 22, according to the hair control device 10 of the present variation, the light guide member 25 includes a plurality of (eight) recesses 250 corresponding one-to-one to a plurality of (eight-pieces of) LEDs 221. The recess 250 has a shape corresponding to a shape of the LED 221. That is, the recess 250 has a shell shape. The surface of the light guide member 25 forming the recess 250 constitutes a light incident surface 251.

According to the hair control device 10 of the present variation, the light incident surface 251 of the light guide member 25 has a lens surface having the shape corresponding to the shape of the recess 250. The light incident surface 251 has a shape of a concave lens surface. The light incident surface 251 having the lens surface can appropriately adjust a shape of the light of the light source 22 when the light enters the light guide member 25 through the light incident surface 251.

In an alternative example, the recess 250 of the light guide member 25 and the light source 22 (LED 221) may be in close contact with each other without gap.

### (2.4.2) Second Variation

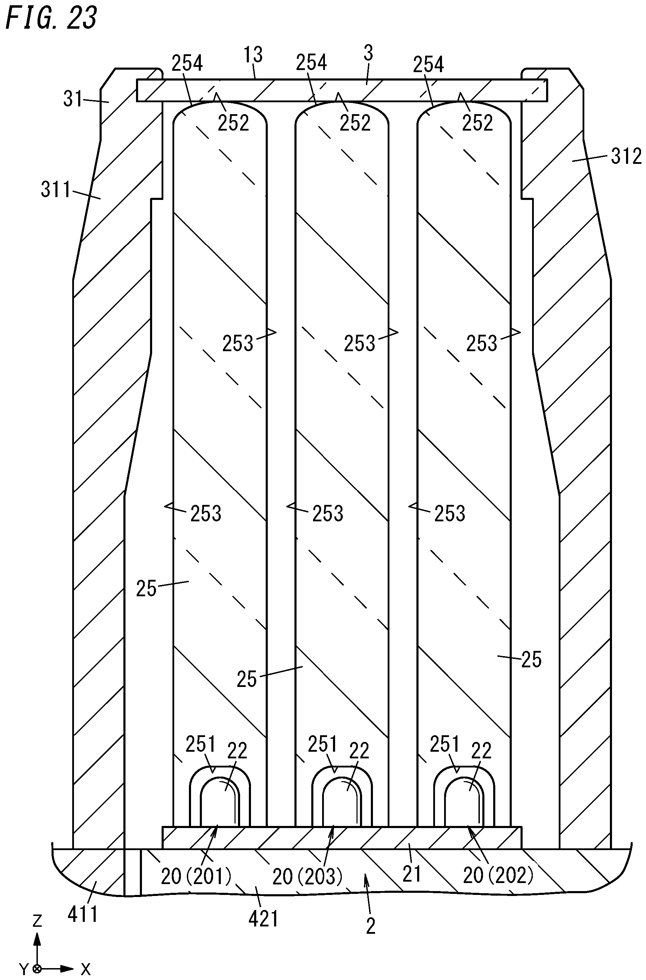

A hair control device 10 (photocosmetic device 100) of the present variation will be described with reference to FIG. 23. The hair control device 10 of the present variation differs from the hair control device 10 of the basic example in a structure of a light guide member 25.

As shown in FIG. 23, according to the hair control device 10 of the present variation, a light emerging surface 252 of a light guide member 25 in each of a plurality of light source units 20 has a lens surface 254. In each light guide member 25, cross sectional shapes of the light emerging surface 252 in any imaginary planes Im10 (see FIG. 17) perpendicular to one axis (Y-axis) are the same as each other. The light emerging surface 252 has a shape of a symmetric cylindrical convex lens surface (when viewed in the Y-axis).

The properties (such as the curvatures) of the shape of the light incident surface 251 and the shape of the light emerging surface 252 are determined such that the light of the light source 22 is collimated when it is emitted from the light emerging surface 252. That is, the light source unit 20 emits the collimated light. The rays of the collimated light emitted from the plurality of light source units 20 are parallel to one another.

According to the hair control device 10 of the present variation, the plurality of light source units 20 are apart from one another in the X-axis and the light emerging surface 252 of each of the plurality of light source units 20 has the lens surface 254, which can further reduce the overlap of the rays of the light L1 of adjacent two light source units 20 on the light exit surface 13. Moreover, since the shapes of the light emerging surfaces 252 of the light guide members 25 of the light source units 20 are set such that the rays of the collimated light emitted from the light emerging surfaces 252 of the plurality of light source units 20 are parallel to one another, it is possible to further reduce the overlap of the rays of the light L1 of adjacent two light source units 20 on the light exit surface 13. This can suppress the increase in the temperature of the skin 92 around the surface layer caused by the absorption of the light L1 while the surface of the skin 92 is in contact with the light exit surface 13. The uncomfortable feeling to be caused in a user can be reduced accordingly.

### (2.4.3) Third Variation

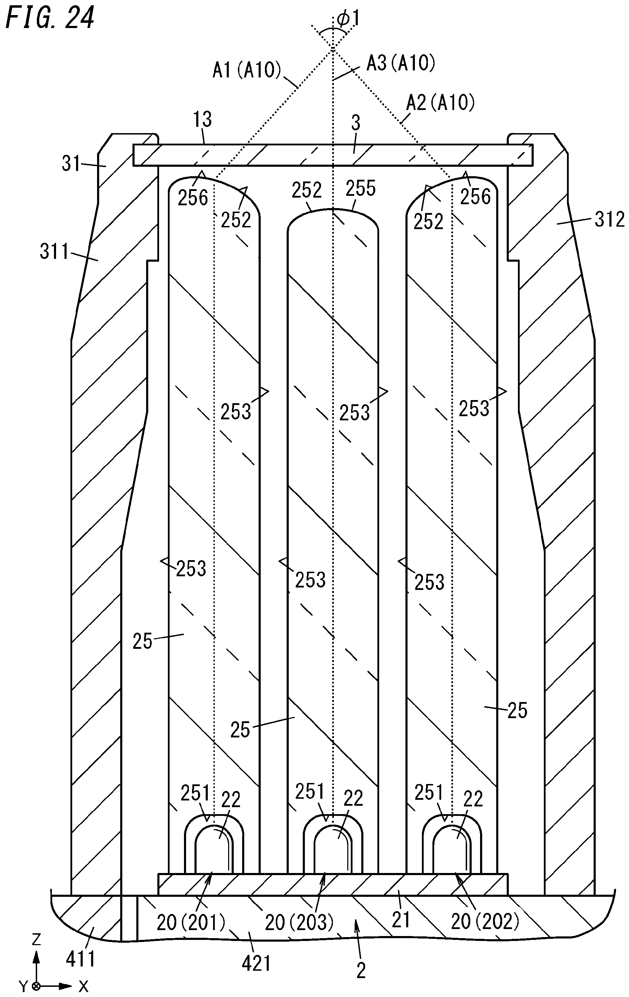

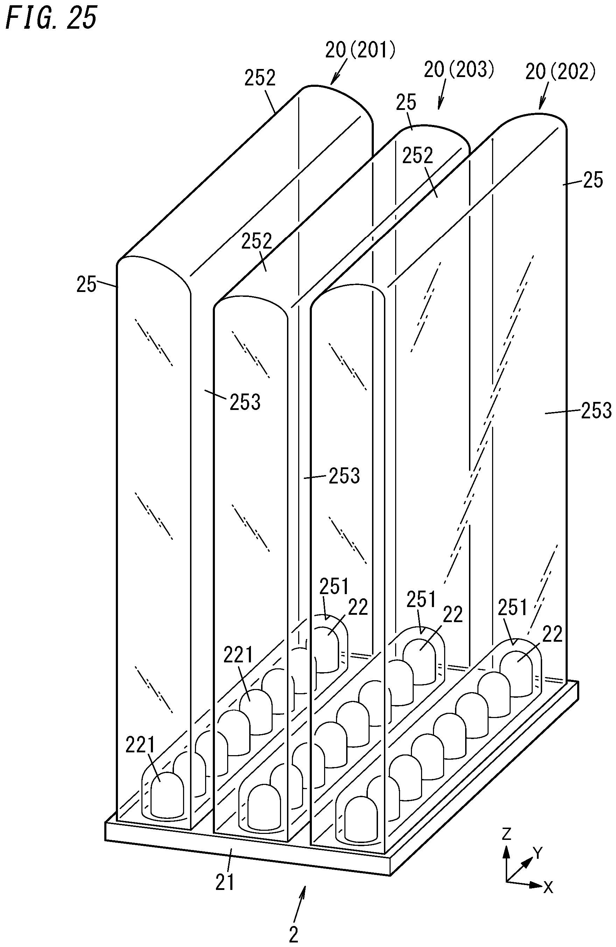

A hair control device 10 (photocosmetic device 100) of the present variation will be described with reference to FIGS. 24, 25. The hair control device 10 of the present variation differs from the hair control device 10 of the second variation in a structure of a light guide member 25.

According to the hair control device 10 of the present variation, light emerging surfaces 252 of the light guide members 25 of a plurality of light source units 20 are formed such that rays of light L1 of the plurality of light source units 20 cross one another on an outside of a housing 1. The light emerging surfaces 252 of the light guide members 25 of the plurality of light source units 20 respectively have lens shapes such that the rays of the light L1 of the plurality of light source units 20 cross one another on the front side of a light exit surface 13.

Specifically, as shown in FIGS. 24, 25, in a third light source unit 203, which is the center one of the plurality of (three) light source units 20 arranged along the X-axis, the light emerging surface 252 (lens surface 255) of the light guide member 25 has a lens shape of a symmetric cylindrical convex lens surface, as with the light source unit 20 of the second variation.

On the other hand, in each of a first light source unit 201 and a second light source unit 202, each of which is one of the plurality of (three) light source units 20 arranged along the X-axis and which are located on the left side and the right side, the light emerging surface 252 (lens surface 256) of the light guide member 25 has a lens shape of an asymmetric cylindrical convex lens surface, as shown in FIGS. 24, 25. In the first light source unit 201, the light emerging surface 252 of the light guide member 25 has a shape to refract the ray of the light of the light source 22 inward (toward the right side). In the second light source unit 202, the light emerging surface 252 of the light guide member 25 has a shape to refract the ray of the light of the light source 22 inward (toward the left side). The light guide member 25 of each of the first light source unit 201 and the second light source unit 202 has a dimension (length) along a first axis (Z-axis) greater than a dimension along the first axis (Z-axis) of the light guide member 25 of the third light source unit 203.

The shape of the light emerging surface 252 of the light guide member 25 of the first light source unit 201 and the shape of the light emerging surface 252 of the second light source unit 202 are symmetric. Therefore, the shapes of the light emerging surfaces 252 of the light guide members 25 of the plurality of (three) light source units 20 are symmetric in total.

As shown in FIG. 24, according to the hair control device 10 of the present variation, an optical axis A1 of the first light source unit 201 does not coincide with an optical axis of the light source 22 of the first light source unit 201, but extends diagonally inward (rightward) and forward from the light emerging surface 252. Also, an optical axis A2 of the second light source unit 202 does not coincide with an optical axis of the light source 22 of the second light source unit 202, but extends diagonally inward (leftward) and forward from the light emerging surface 252. On the other hand, an optical axis A3 of the third light source unit 203 coincides with an optical axis of the light source 22 of the third light source unit 203, and extends forward along the Z-axis from the light emerging surface 252. The optical axes A10 of the plurality of light source units 20 cross one another on an outside of the housing 1 (specifically, in front of the light exit surface 13).

Accordingly, the rays of the light L1 from the plurality of (three) light source units 20 cross one another on a front side of the light exit surface 13.

In the usage state of the hair control device 10 of the present variation where the light exit surface 13 is in contact with the surface of the skin 92, the rays of the light L1 of the plurality of light source units 20 cross one another inside the skin 92. This can facilitate the rays of the light L1 of the plurality of light source units 20 to cross one another inside the skin 92 around the hair matrix 912. This facilitates the absorption of the light L1 in the skin 92 in the region around the hair matrix 912 to efficiently damage the hair matrix 912.

According to the hair control device 10 of the present variation, properties of the lens shapes of the light emerging surfaces 252 of the light guide members 25 of the plurality of light source units 20 are determined such that the rays of the light L1 of the plurality of light source units 20 cross one another inside the skin 92 around the hair matrix 912 (bulb) in a state where the surface of the skin 92 is in contact with the light exit surface 13. For example, the properties of the lens shapes of the light emerging surfaces 252 of the light guide members 25 of the plurality of light source units 20 are determined such that the rays of the light L1 of the plurality of light source units 20 cross one another inside the skin 92 of the depth from the surface falling within the range of about 2 mm to 5 mm (e.g., about 4 mm) in a state where the surface of the skin 92 is in contact with the light exit surface 13. The properties of the lens shapes of the light emerging surfaces 252 of the light guide members 25 of the plurality of light source units 20 may be determined also in consideration with the refractive index of the skin 92 and the like. A crossing angle between two light source units 20 having largest angle value (hereinafter, referred to as a crossing angle cp1 of the optical axes A10; see FIG. 24) among the crossing angles of the plurality of light source units 20 is set to fall within the range equal to or greater than 10° and smaller than 180°, for example. The crossing angle cp1 may be smaller than 170°. The crossing angle ϕ1 may fall within the range equal to or greater than 50° and equal to or smaller than 160°. The crossing angle ϕ1 may fall within the range equal to or greater than 90° and equal to or smaller than 150°.

The hair control device 10 of the present variation can efficiently provide the damage to the hair matrix 912.

### (2.4.4) Fourth Variation

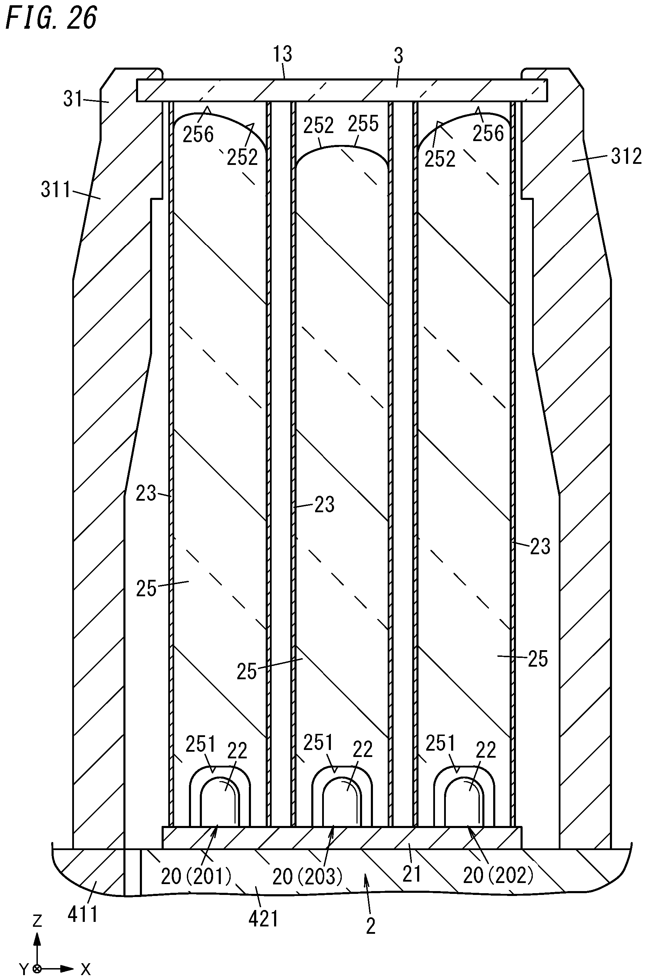

A hair control device 10 (photocosmetic device 100) of the present variation will be described with reference to FIG. 26. The hair control device 10 of the present variation differs from the hair control device 10 of the third variation in a structure of light source units 20.

As shown in FIG. 26, according to the hair control device 10 of the present variation, each of the plurality of light source units 20 further includes a light blocking member 23. The light blocking member 23 is configured to reduce the light (leakage light) emitted from a light source 22 and going toward at least one of remainder of the plurality of light source units 20.

Specifically, in each of the plurality of light source units 20, the light blocking member 23 has a shape surrounding the light guide member 25 when viewed in the normal direction of a substrate 21. The light blocking member 23 surrounds the light guide member 25 seamlessly when viewed in the normal direction of the substrate 21. According to the hair control device 10 of the present variation, the light blocking member 23 has a rectangular frame shape when viewed in the normal direction of the substrate 21. The light blocking member 23 has a wall shape extending along the normal direction of the substrate 21. The light blocking member 23 is provide to an entire length of the light guide member 25 along the Z-axis. Accordingly, in each of the plurality of light source units 20, the light blocking member 23 surrounds side surfaces (four side surfaces) of the light guide member 25. The light blocking member 23 is like a wall surrounding the light guide member 25 and having a rectangular frame shape when viewed in the normal direction of the substrate 21.

The light blocking member 23 is configured to reduce the amount of light (leakage light) emerging outward from the side surfaces of the light guide member 25, among the light emitted from the light source 22 and entered the light guide member 25. Accordingly, the light blocking member 23 prevents the light, which is emitted from the light source 22 of the light source unit 20 including this light blocking member 23, from leaking to another light source unit 20.

The light blocking member 23 has an opening on a side of the light emerging surface 252 of the light guide member 25 (i.e., opened forward). The light emerging from the light guide member 25 is emitted through the opening.

A mirror may be formed on an inside surface of the light blocking member 23 to reflect the light of the light source 22. This can increase the amount of the light L1 emitted from the light source unit 20.

The light blocking member 23 is fixed to the substrate 21, for example. The light blocking member 23 may have the heat conduction property. The light blocking member 23 may be made of metal, for example. When the light blocking member 23 has the heat conduction property and is thermally connected to (e.g., in contact with) the substrate 21, the heat generated in the light source 22 (LEDs 221) can be emitted outward through the light blocking member 23, which can facilitate the cooling of the light source 22.

The light blocking member 23 may be thermally connected to the light transmission member 3 or may be in contact with the light transmission member 3. When the light blocking member 23 has the heat conduction property and is thermally connected to (e.g., in contact with) the light transmission member 3, the light blocking member 23 can facilitate the cooling of the light transmission member 3.

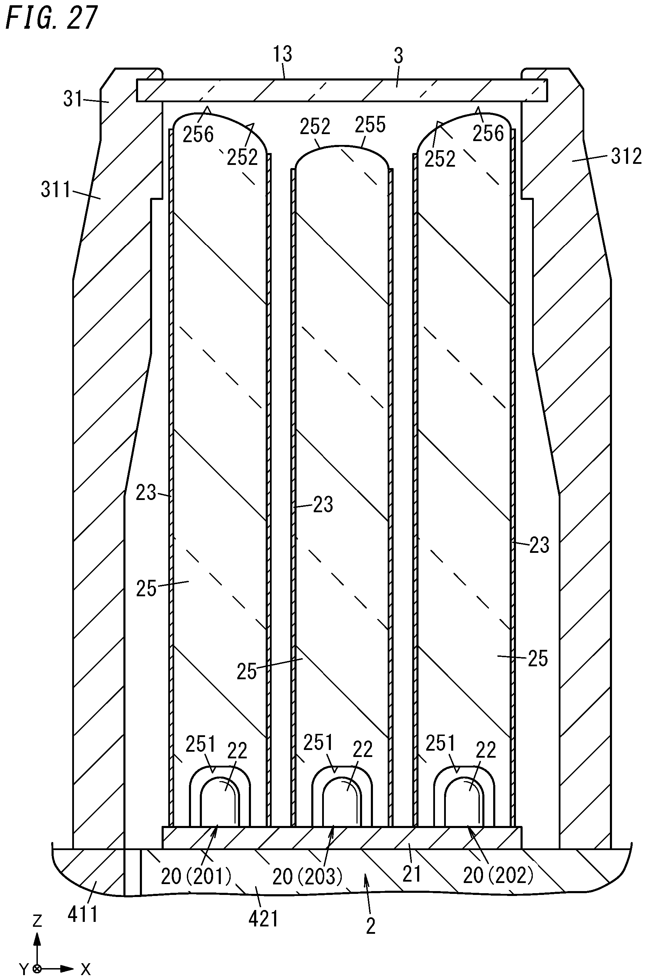

In an alternative example, the light blocking member 23 may not be provided to the entire length of the light guide member 25 along the Z-axis. As shown in FIG. 27, the light blocking member 23 may surround the light guide member 25 such that a part of the light guide member 25 corresponding to the light emerging surface 252 having the lens shape is exposed outward when viewed in the X-axis, for example.

### (2.4.5) Fifth Variation

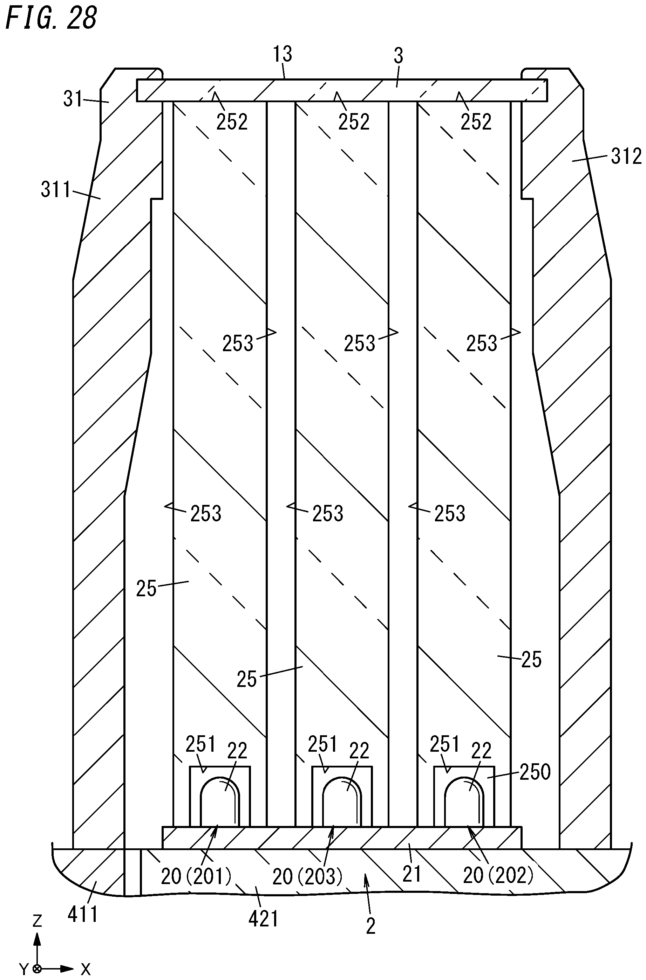

A hair control device 10 (photocosmetic device 100) of the present variation will be described with reference to FIG. 28. The hair control device 10 of the present variation differs from the hair control device 10 of the basic example in a configuration of a light guide member 25.

As shown in FIG. 28, according to the hair control device 10 of the present variation, the light guide member 25 has a recess 250 having a groove shape with a rectangular bottom surface and rectangular side surfaces. That is, according to the hair control device 10 of the present variation, a light incident surface 251 of the light guide member 25 is not a lens surface. The surface of the light guide member 25 forming the recess 250 constitutes the light incident surface 251.

The hair control device 10 of the present variation can have the improved handleability.

### (2.4.6) Sixth Variation

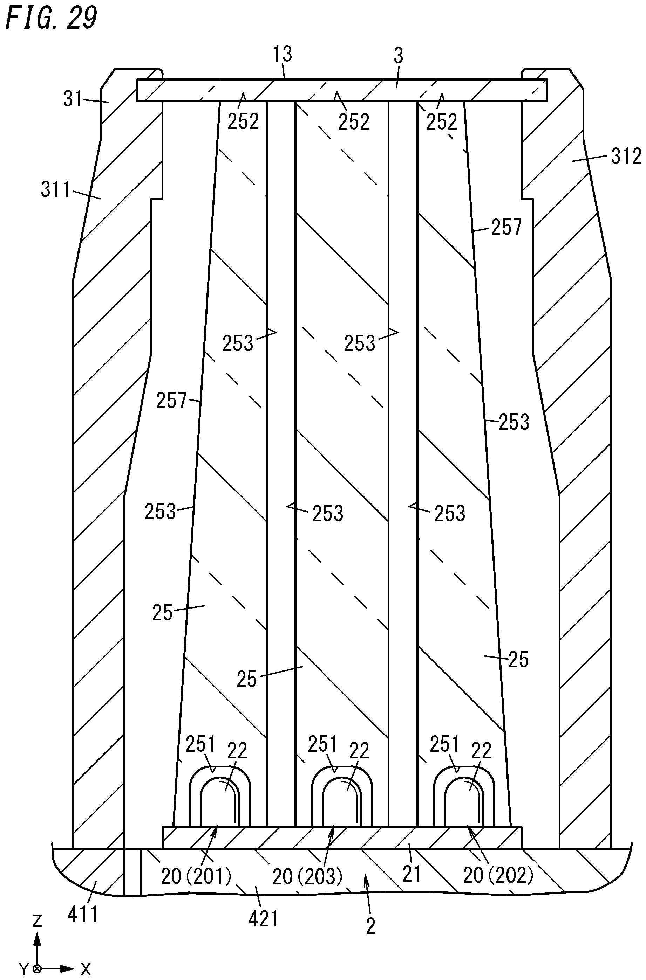

A hair control device 10 (photocosmetic device 100) of the present variation will be described with reference to FIG. 29. The hair control device 10 of the present variation differs from the hair control device 10 of the basic example in a structure of a light guide member 25.

According to the hair control device 10 of the present variation, in each of a first light source unit 201 and a second light source unit 202, which are respectively located on the left side and the right side of the plurality of (three) light source units 20 arranged along the X-axis, a light guide member 25 has a pair of side surfaces that are non-parallel to each other, as shown in FIG. 29.

Specifically, the light guide member 25 of the first light source unit 201 has an outer (left) side surface 253 that is an inclined surface 257 inclined such that the front side (positive side in the Z-axis) of the inclined surface 257 positions on the inner side (right side). In the first light source unit 201, the light guide member 25 has the thickness (dimension in the X-axis) gradually decreasing toward the front end. In other words, the light guide member 25 of the first light source unit 201 has a tapered shape of which cross-sectional area decreases from a light incident surface 251 to a light emerging surface 252.

The light guide member 25 of the second light source unit 202 has an outer (right) side surface 253 that is an inclined surface 257 inclined such that the front side (positive side in the Z-axis) of the inclined surface 257 positions on the inner side (left side). In the second light source unit 202, the light guide member 25 has the thickness (dimension in the X-axis) gradually decreasing toward the front end. In other words, the light guide member 25 of the second light source unit 202 has a tapered shape of which cross-sectional area decreases from a light incident surface 251 to a light emerging surface 252.

Since the light guide member 25 has the tapered shape, the dimension of a second case 12 can be relatively small at the front end. This can reduce the size of the front end of the hair control device 10 where the light exit surface 13 is provide, which allows the operator to easily position the light exit surface 13 on a desired region of the surface of the skin 92.

Note that a side surface of the light guide member 25 crossing with the Y-axis may be an inclined surface inclined such that the front side (positive side in the Z-axis) of the inclined surface positions on the inner side (right side).

### (2.4.7) Seventh Variation

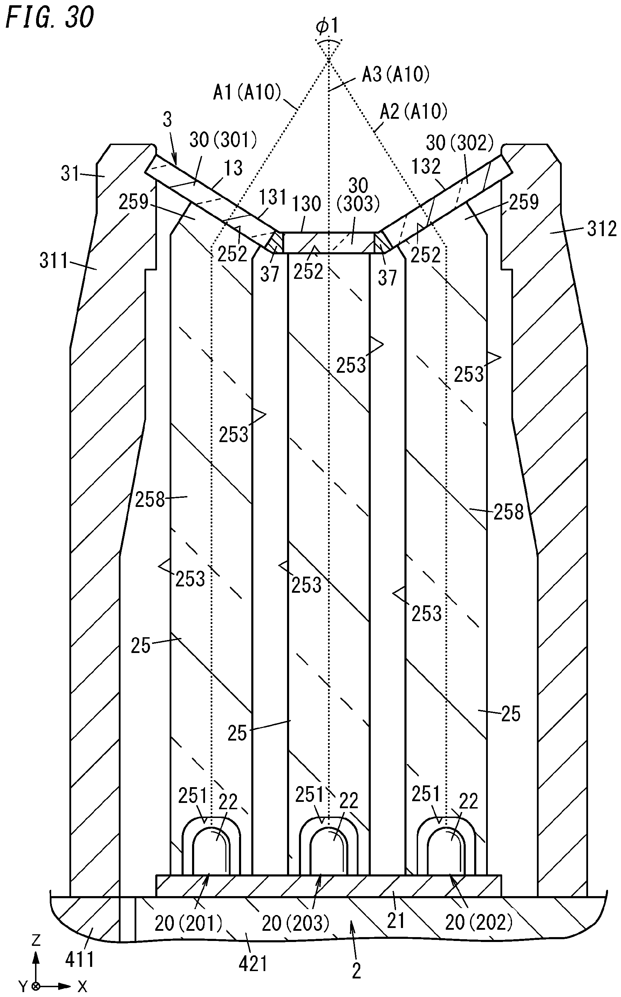

A hair control device 10 (photocosmetic device 100) of the present variation will be described with reference to FIG. 30. The hair control device 10 of the present variation differs from the hair control device 10 of the basic example in a configuration of a housing 1 (second case 12), a light exit surface 13, a light emitting unit 2, a light transmission member 3 etc.

As shown in FIG. 30, according to the hair control device 10 of the present variation, the light exit surface 13 has a recessed shape recessed inward the housing 1.

Specifically, according to the hair control device 10 of the present variation, each of a light guide member 25 of a first light source unit 201 and a light guide member 25 of a second light source unit 202 includes a first portion 258 and a second portion 259.

According to the first light source unit 201, the first portion 258 of the light guide member 25 has a cuboid shape extending along an optical axis of a light source 22 (extending along the Z-axis). The second portion 259 of the light guide member 25 of the first light source unit 201 has a cuboid shape extending diagonally right forward from the front end of the first portion 258. Accordingly, in the first light source unit 201, the light guide member 25 has a light emerging surface 252 (front surface of the second portion 259) of an inclined surface inclined inward. An optical axis A1 of the first light source unit 201 does not coincide with the optical axis of the light source 22 of the first light source unit 201, but extends diagonally inward (rightward) and forward from the light emerging surface 252.

According to the second light source unit 202, the first portion 258 of the light guide member 25 has a cuboid shape extending along an optical axis of ta light source 22 (extending along the Z-axis). The second portion 259 of the light guide member 25 of the second light source unit 202 has a cuboid shape extending diagonally left forward from the front end of the first portion 258. Accordingly, in the second light source unit 202, the light guide member 25 has a light emerging surface 252 (front surface of the second portion 259) of an inclined surface inclined inward. An optical axis A2 of the second light source unit 201 does not coincide with the optical axis of the light source 22 of the second light source unit 202, but extends diagonally inward (leftward) and forward from the light emerging surface 252.

According to the hair control device 10 of the present variation, the light transmission member 3 includes a plurality of (three) light transmission pieces 30 (a first light transmission piece 301 to a third light transmission piece 303). The plurality of light transmission pieces 30 are arranged to correspond (one-to-one) to the plurality of light source units 20. The plurality of light transmission pieces 30 are held by a holder 31 such that each of the plurality of light transmission pieces 30 faces (or is in contact with) the light emerging surface 252 of the light guide member 25 of a corresponding light source unit 20. Between adjacent light transmission pieces 30, a light blocking plate 37 is disposed.

The light exit surface 13 includes a bottom surface 130 having a flat plane shape perpendicular to the Z-axis, a first inclined surface 131 located on the left and inclined diagonally forward left with respect to the bottom surface 130, and a second inclined surface 132 located on the right and inclined diagonally forward right with respect to the bottom surface 130. A front surface of the first light transmission piece 301 constitutes the first inclined surface 131. A front surface of the second light transmission piece 302 constitutes the second inclined surface 132. A front surface of the third light transmission piece 303 constitutes the bottom surface 130.

According to the hair control device 10 of the present variation including the recessed shaped light exit surface 13, the skin 92 is irradiated with the light L1 in a state where the skin 92 is deformed so as to be introduced into the space surrounded by the light exit surface 13, as with the hair control device 10 of the embodiment 1. This can reduce the uncomfortable feeling to be caused in the user. The hair control device 10 of the present variation has the advantage of reducing the uncomfortable feeling to be caused in the user as with the hair control device 10 of the embodiment 1, in addition to the advantage of improving the handleability as with the hair control device 10 of the embodiment 2.

### (2.4.8) Eighth Variation

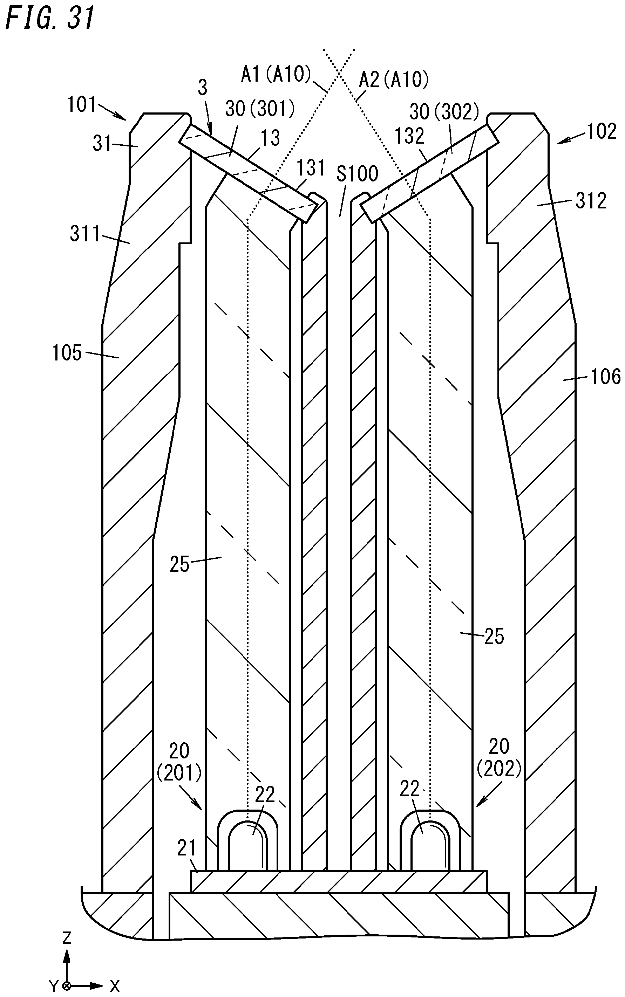

A hair control device 10 (photocosmetic device 100) of the present variation will be described with reference to FIG. 31. The hair control device 10 of the present variation differs from the hair control device 10 of the seventh variation in a configuration of a housing 1 (second case 12), a light emitting unit 2, a light transmission member 3 etc.

As shown in FIG. 31, according to the hair control device 10 of the present variation, the light emitting unit 2 does not include a third light source unit 203, and also the light transmission member 3 does not include a third light transmission piece 303. Moreover, according to the hair control device 10 of the present variation, the housing 1 (specifically, the second case 12) includes a first housing part 105 in which a first light source unit 201 is housed and a second housing part 106 in which a second light source unit 202 is housed. The first housing part 105 and the second housing part 106 are disposed separately (separated) from each other in a direction in which the first light source unit 201 and the second light source unit 202 are arranged.

Specifically, the hair control device 10 of the present variation includes a first light source block 101 and a second light source block 102.

The first light source block 101 includes the first light source unit 201, a first light transmission piece 301, and the first housing part 105. The first housing part 105 includes a left side wall 311 of a holder 31, has a peripheral wall shape surrounding the first light source unit 201, and holds the first light transmission piece 301 such that the first light transmission piece 301 is positioned on an optical axis A1 of the first light source unit 201. A front surface of the first light transmission piece 301 serves as a first inclined surface 131 of a light exit surface 13.

The second light source block 102 includes the second light source unit 202, a second light transmission piece 302, and the second housing part 106. The second housing part 106 includes a right side wall 312 of the holder 31, has a peripheral wall shape surrounding the second light source unit 202, and holds the second light transmission piece 302 such that the second light transmission piece 302 is positioned on an optical axis A2 of the second light source unit 202. A front surface of the second light transmission piece 302 serves as a second inclined surface 132 of the light exit surface 13.

As shown in FIG. 31, in the hair control device 10 of the present variation, the first light transmission piece 301 and the second light transmission piece 302 are disposed separately from each other in the direction along the X-axis (in which the first light source unit 201 and the second light source unit 202 are arranged) and disconnected from each other. As shown in FIG. 31, there is a space S100 between the first housing part 105 and the second housing part 106. A distance (dimension in the X-axis) between the first housing part 105 and the second housing part 106 may fall within the range from 0.1 mm to 10 mm, or may fall within the range from 0.5 mm to 5 mm, or may be about 2 mm, for example. According to the hair control device 10 of the present variation, therefore, the space S100 is formed between the first light source block 101 and the second light source block 102.

According to the hair control device 10 of the present variation, the housing 1 is divided into the first housing part 105 in which the first light source unit 201 is housed and the second housing part 106 in which the second light source unit 202 is housed. This can reduce light (leakage light) traveling from a light source 22 of one of the first light source unit 201 and the second light source unit 202 toward a light source 22 of the other.

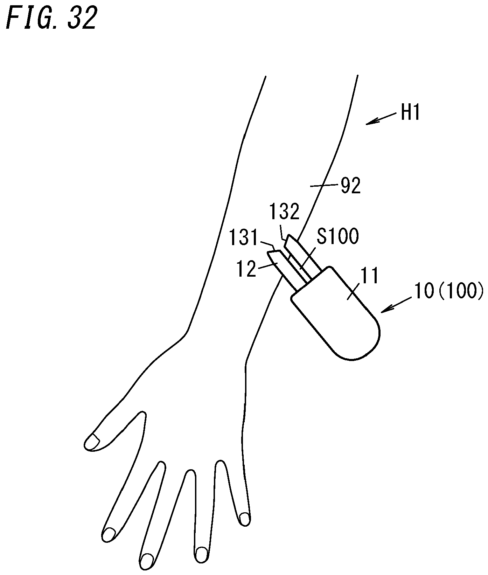

According to the hair control device 10 of the present variation, the space S100 is formed between the first housing part 105 and the second housing part 106. This allows the operator to see the position to be irradiated with the treatment light through the space S100 as schematically shown in FIG. 32, which allows the operator to easily position the light exit surface 13 to a desired region of the skin 92. The hair control device 10 of the present variation can have the further improved handleability.

### (2.4.9) Other Variations

In one variation, a housing 1 does not have to house therein an entire of a plurality of light source units 20. A housing 1 may house a light source unit 20 with a part of a light source unit 20 (e.g., a part of a light blocking member 23) protruding from the housing 1, for example.

In one variation, a light transmission member 3 may have a shape serving as a lens.

In one variation, a photocosmetic device 100 may not include a light transmission member 3.

In one variation, an operations unit 6 may include a switch provided on a side surface of a first case 11, for example.

In one variation, a cooler 5 may includes a Peltier device 52.

In one variation, a light emitting unit 2 may include one, two, four or more light source units 20. A light source 22 of a light source unit 20 may include only one LED 221 or two or more LEDs 221.

In one variation, a light source 22 of a light source unit 20 is not limited to an LED 221 but may include a xenon lamp or a semiconductor laser.

### (3) Embodiment 3

### (3.1) Overview of Embodiment 3

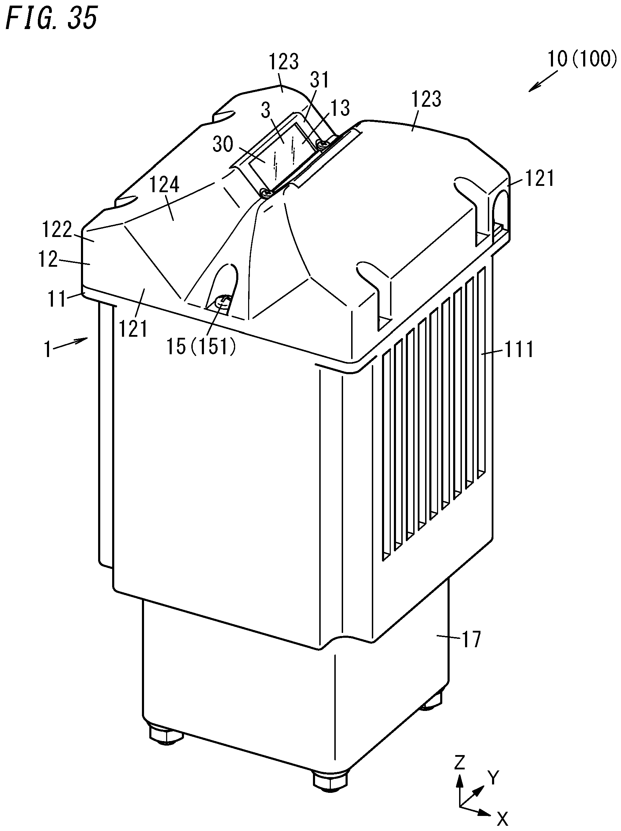

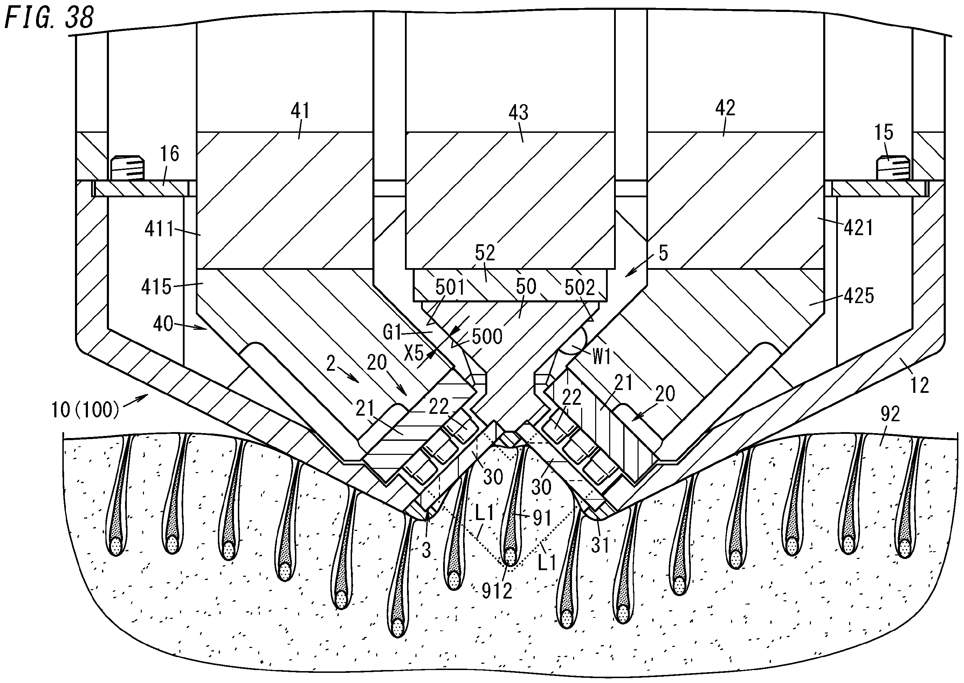

A photocosmetic device 100 of the present embodiment (see FIGS. 35 to 37) is a device for illuminating light to a skin 92 (see FIG. 38) to cause absorption of the light in the skin 92 to treat the skin 92 with the function of the absorbed light.

JP2000-153003A (hereinafter, referred to as a "reference document") discloses a cooling probe for laser beauty culture instrument. In a laser cosmetic device for performing cosmetic treatment by irradiating a laser beam from a tip of a probe to be brought into contact with a skin surface, the tip of the probe is made of a good thermal conductor, and a cooling means that utilizes the thermoelectric heat absorption phenomenon is connected to the tip of the probe.

In the cooling probe, a frame-shaped head part protruding a conical surface is integrally attached to the side face of a cylindrical case. Concerning the head part, a recessed part is formed at the center of the front, for which the top end of the conical surface is notched, and a spherical lens is installed at the deepest part thereof. At the back of the spherical lens, a hole is bored and a laser diode is inserted and fitted. The base part of the head part is surrounded with Peltier elements as cooling means utilizing the heat absorption phenomenon of thermoelectricity and the outside of Peltier elements is covered with a heat sink for absorbing heat exchanged by the Peltier elements.

A photocosmetic device such as the laser beauty culture instrument of the reference document may be desired to balance the cooling of the skin and the cooling of the light source.

That is, around the surface layer of the skin 92 of animals (including the human), there is some substances that may absorb the light L10, such as the water, the vessel, and the like, in addition to the melanin contained in the hair follicle 911. Therefore, when the light L10 is illuminated on the skin 92 for the purpose of e.g., the hair control or the hair removal, at least part of the light L10 is absorbed in such the substances (water etc.) and generates the heat. The heat caused by the absorption of the light L10 may stimulate the nerves that detect pain present in the skin 92 and the like, and a large stimulation on the nerves may cause an uncomfortable feeling in the user.

Cooling of the skin 92 is known as one example method to lessen the stimulation on the nerves of the skin 92. However, this method requires a means for cooling the skin 92.

Moreover, in order to illuminate the light L10 on the skin 92, a light source that can emit the light L10 is required. The light source usually emits the light L10 by converting electric energy into light energy. When the electric energy is converted into the light energy, part of the electric energy is inevitably converted into the thermal energy to generate the heat. As a result of which the temperature of the light source may be excessively increased by the generated heat, which may decrease the luminous efficacy of the light source. To avoid this, the photocosmetic device 100 is required to include a means for cooling the light source.

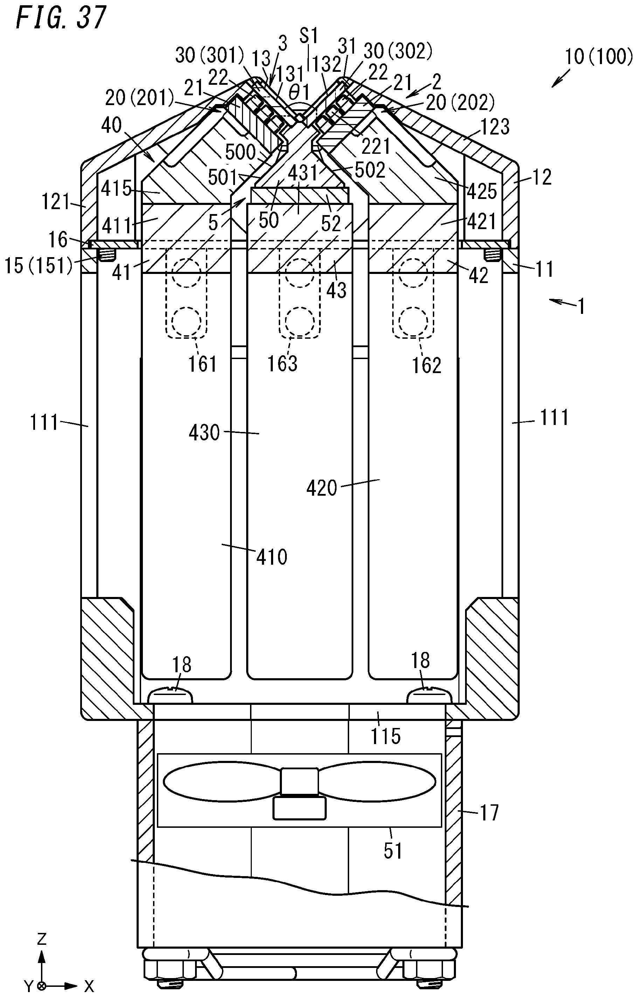

As shown in FIG. 37, the photocosmetic device 100 of the present embodiment includes a light emitting unit 2, a heat dissipator 40 (a first heat dissipation part 41 and a second heat dissipation part 42), a light transmission member 3, a heat conductor 50, and a cooler 5. The light emitting unit 2 is configured to emit light to be illuminated on the skin 92 (see FIG. 38). The heat dissipator 40 is thermally connected to the light emitting unit 2. The heat dissipator 40 is configured to dissipate the heat generated in the light emitting unit 2. The light transmission member 3 allows the light of the light emitting unit 2 to pass through. The heat conductor 50 is thermally connected to the light transmission member 3. The heat of the light transmission member 3 is transmitted to the heat conductor 50. The cooler 5 is disposed to cool the heat conductor 50. In the photocosmetic device 100, a predetermined surface 500, which is at least part of a surface of the heat conductor 50, faces the heat dissipator 40 with a gap between the predetermined surface 500 and the heat dissipator 40.

The photocosmetic device 100 of the present embodiment can facilitate the cooling of the light transmission member 3, since the heat in the light transmission member 3 is transferred to the heat conductor 50 which is cooled by the cooler 5. It is accordingly possible to facilitate the cooling of the body (such as the skin 92) in contact with the light transmission member 3. Moreover, according to the photocosmetic device 100 of the present embodiment, the heat generated in the light emitting unit 2 is transferred to the heat dissipator 40 and then is dissipated from the heat dissipator 40. The cooling of the light emitting unit 2 also can be facilitated.

Incidentally, in the photocosmetic device 100 of the present embodiment, when the heat conductor 50 is cooled by the cooler 5, a dew condensation water (water droplet W1; see FIG. 38) may possibly appear on the surface of the heat conductor 50 by the dew condensation. In this regard, according to the photocosmetic device 100 of the present embodiment, the predetermined surface 500 of the heat conductor 50 faces the heat dissipator 40 with a gap therebetween. Accordingly, the water droplet W1 of the dew condensation water appeared on the predetermined surface 500 can be brought into contact with the heat dissipator 40. While in contact with the heat dissipator 40 of which temperature is increased by the heat of the light emitting unit 2, the dew condensation water is heated by the heat dissipator 40 and vaporized, and takes the heat (heat of vaporization) away from the heat dissipator 40. This can facilitate the cooling of the heat dissipator 40 and also facilitate the cooling of the light emitting unit 2 thermally connected to the heat dissipator 40.

Accordingly, the photocosmetic device 100 of the present embodiment can balance the cooling of the skin and the cooling of the light source. Moreover, the photocosmetic device 100 of the present embodiment can facilitate the cooling of the light emitting unit 2.

### (3.2) Details of Embodiment 3

In the following, a hair control device 10 will be described as an example of the photocosmetic device 100 (body hair treatment device) of the present embodiment.

As shown in FIGS. 35 to 37, the hair control device 10 of the present embodiment includes a housing 1, the light emitting unit 2, the light transmission member 3, the heat dissipation part 4, the cooler 5, an operations unit 6, a controller 71 (see FIG. 5), and a power source 72 (see FIG. 5).

The housing 1 houses therein the light emitting unit 2, the light transmission member 3, the heat dissipation part 4, the cooler 5, the controller 71, and the power source 72. The operations unit 6 is provided to an outer peripheral surface of the housing 1, for example.

As shown in FIGS. 35 to 37, the housing 1 includes a first case 11, a second case 12, and a third case 17.

The first case 11 is made from resin, for example. As shown in FIGS. 35 to 37, the first case 11 has a rectangular tube shape opened forward. The first case 11 includes left and right side walls in which a plurality of air vents 111 are formed to penetrate the side wall along the X-axis. The first case 11 has an opening 115 in a rear surface.

The second case 12 is made from resin, for example. The second case 12 is disposed on the front side of the first case 11 to cover the front opening of the first case 11. As shown in FIG. 35, the second case 12 includes a peripheral wall 122, a pair of front walls 123, and a bottom wall 124. The peripheral wall 122 includes four side walls 121. The peripheral wall 122 has a rectangular frame shape and protrudes from a periphery around the front opening of the first case 11. The peripheral wall 122 is formed of the four side walls 121. Each of the pair of front walls 123 has a substantially trapezoid shape (hexagon near the isosceles trapezoid shape) when viewed from the front side. The pair of front walls 123 extend diagonally forward from front ends of left and right side walls 121 among the four side walls 121. The bottom wall 124 has a groove shape recessed rearward. The bottom wall 124 interconnects between the remaining two side walls 121 among the four side walls 121 and front ends of the pair of front walls 123 together.

The second case 12 and the first case 11 are coupled together with screws 15. Specifically, between the first case 11 and the second case 12, a fixing plate 16 (see FIG. 37) is disposed which has a rectangular frame shape when viewed from the front side and in which a plurality of screw holes are formed. The screws 15 include first screws 151 having heads on the front side and second screws having heads on the rear side. The second screws are provided on four corners of a front plate of the second case 12. The first screws 151 are inserted from the front side of the first case 11 into through holes formed in the first case 11, and are screwed into the screw holes of the fixing plate 16. The second screws are inserted from the rear side of the second case 12 into through holes formed in four corners of the front plate of the second case 12, and are screwed into the screws holes of the fixing plate 16. As a result, the first case 11 and the second case 12 are coupled together via the fixing plate 16.

The third case 17 is made from resin, for example. As shown in FIGS. 35, 37, the third case 17 has a rectangular tube shape opened forward and rearward. The third case 17 is a bit smaller than the first case 11 when viewed in the Z-axis. The third case 17 is disposed on the rear side of the first case 11 such that a front opening of the third case 17 is continuous with the rear opening 115 of the first case 11. The third case 17 and the first case 11 are coupled together with screws 18.

The hair control device 10 includes a light exit surface 13 (light exit part). The light exit surface 13 is a surface from which the light of the light emitting unit 2 is emitted to the outside of the housing 1. According to the hair control device 10 of the present embodiment, the light exit surface 13 is positioned in the front surface of the housing 1 (the front surface of the second case 12). When viewed from the front side, the light exit surface 13 is located at the center of the front surface of the housing 1 (the center of the front surface of the second case 12).

As shown in FIGS. 35, 37, the light exit surface 13 has a recessed shape (concave shape) recessed inward the housing 1. The light exit surface 13 includes a first inclined surface 131 and a second inclined surface 132. The first inclined surface 131 and the second inclined surface 132 face each other in the X-axis. The first inclined surface 131 and the second inclined surface 132 cross each other by a crossing angle θ1. The crossing angle θ1 is set to have an angle value falling within the range greater than 0° and smaller than 180°. The crossing angle θ1 may have an angle value falling within the range equal to or greater than 30° and equal to or smaller than 120°. The crossing angle θ1 may have an angle value falling within the range equal to or greater than 30° and equal to or smaller than 60°. The crossing angle θ1 may have an angle value falling within the range equal to or greater than 60° and equal to or smaller than 120°. The crossing angle θ1 may have an angle value falling within the range equal to or greater than 60° and equal to or smaller than 80°. The crossing angle θ1 may have an angle value falling within the range equal to or greater than 80° and equal to or smaller than 120°. The crossing angle θ1 may have an angle value falling within the range equal to or greater than 90° and equal to or smaller than 120°. As one example, according to the hair control device 10 of the present embodiment, the crossing angle θ1 is 90°.

As shown in FIG. 37, a recessed space S1 is defined as a space surrounded by the light exit surface 13 (between the first inclined surface 131 and the second inclined surface 132). As shown in FIG. 38, the recessed space S1 is a space into which the skin 92 is introduced when the hair control device 10 is used. The recessed space S1 is also a space to which the light is emitted from the hair control device 10 when the hair control device 10 is used. According to the hair control device 10 of the present embodiment, the recessed space S1 includes a V-shaped groove space surrounded by the light exit surface 13 when viewed in the Y-axis. A bottom angle of the V-shaped groove is the same as the crossing angle θ1. When the bottom angle of the groove of the recessed space S1 is greater than 0° and smaller than 180°, the skin 92 can be introduced into the recessed space S1. When the bottom angle of the groove of the recessed space S1 falls within the range equal to or greater than 30° and equal to or smaller than 120°, the surface of the skin 92 can be easily positioned near the light exit surface 13. According to the bottom angle within this range, the amount of the light illuminated from the first inclined surface 131 and the second inclined surface 132 can be increased at the hair follicle 911 by 1.8 times to 4.5 times compared to a conventional planar light irradiation (corresponding to a case where θ1= 180°). The irradiation efficiency thus can be increased. Moreover, when the bottom angle of the groove of the recessed space S1 falls within the range equal to or greater than 80° and equal to or smaller than 120°, the amount of the light can be increased at the hair follicle 911 by 1.8 times to 2.5 times compared to the planar light irradiation to increase the effect accordingly. Furthermore, when the bottom angle of the groove of the recessed space S1 falls within the range equal to or greater than 60° and equal to or smaller than 80°, the relation between the first inclined surface 131 and the second inclined surface 132 approaches the near parallel state, and the amount of the light illuminated from the two light source units 20 can be increased at the hair follicle 911 by 3.4 times at the maximum compared to the planar light irradiation to increase the effect accordingly. The bottom angle may preferably fall within the range equal to or greater than 30° and equal to or smaller than 60°, which can increase the amount of the light by 4.5 times at the maximum to increase the effect accordingly.

As shown in FIG. 37, the light emitting unit 2 includes a plurality of (in the embodiment, two) light source units 20.

Each of the plurality of light source units 20 includes a substrate 21 and a light source 22.

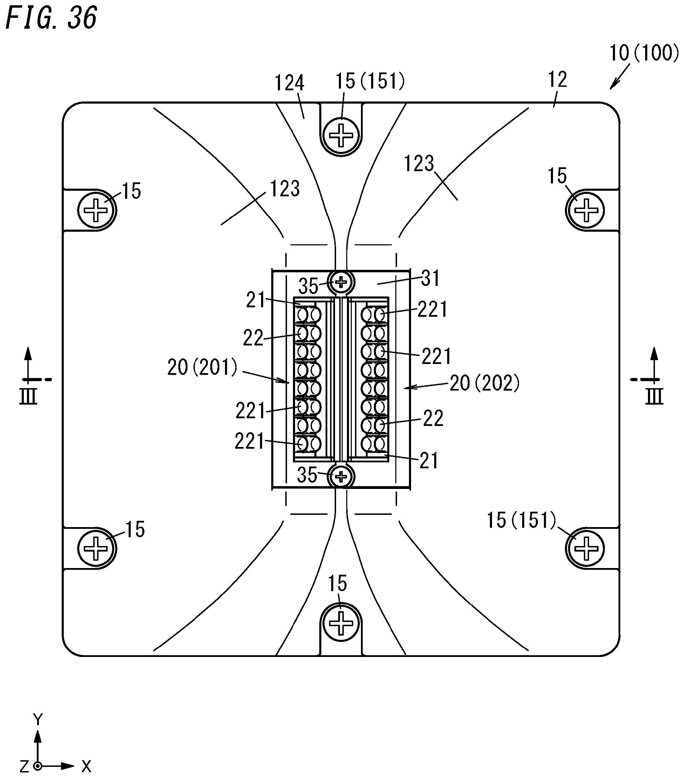

As shown in FIGS. 36, 37, the substrate 21 has a rectangular plate shape. The substrate 21 extends along the Y-axis.

The light source 22 is mounted on the substrate 21. The light source 22 is mounted on a surface on the front side of the substrate 21.

As shown in FIGS. 36, 37, the light source 22 includes a plurality of LEDs 221. That is, the light emitting unit 2 includes, as the light source 22, the plurality of LEDs 221.

As shown in FIG. 36, the plurality of LEDs 221 are arranged in an array on the substrate 21. In the embodiment, the light source 22 includes 24-pieces of LEDs 221. The 24-pieces of LEDs 221 are arranged in a 3x8 array viewed from the front, where there are three pieces along the X-axis and eight pieces along the Y-axis.

Each of the plurality of LEDs 221 is mounted on the substrate 21 such that it exhibits a light distribution property where the direction of the peak intensity is aligned with the normal direction of the substrate 21. In other words, each of the plurality of LEDs 221 has an optical axis along the normal direction of the substrate 21.

According to the hair control device 10 of the present embodiment, each of the plurality of light source units 20 has an optical axis. According to the hair control device 10 of the present embodiment, the optical axis of the light source unit 20 coincides with an optical axis of the light source 22. The optical axis of the light source unit 20 is along the normal direction of the substrate 21.

Hereinafter, the two light source units 20 may be referred to as a first light source unit 201 and a second light source unit 202, respectively, in order to distinguish the two light source units 20 from each other. As shown in FIG. 37, the first light source unit 201 is one of the two light source units 20 located on the left side (on the negative side in the X-axis). The second light source unit 202 is the other of the two light source units 20 located on the right side (on the positive side in the X-axis).

The substrate 21 of the first light source unit 201 is parallel to the first inclined surface 131 of the light exit surface 13. In the X-Y plane, the substrate 21 of the first light source unit 201 is inclined with respect to the X-axis by a predetermined angle (in the embodiment, 135°). The light L1 (see FIG. 38) of the first light source unit 201 is emitted to an outside of the housing 1 (specifically, to the recessed space S1) from the first inclined surface 131 of the light exit surface 13.

The substrate 21 of the second light source unit 202 is parallel to the second inclined surface 132 of the light exit surface 13. In the X-Y plane, the substrate 21 of the second light source unit 202 is inclined with respect to the X-axis by a predetermined angle (in the embodiment, 45°). The light L1 of the second light source unit 202 is emitted to the outside of the housing 1 (specifically, to the recessed space S1) from the second inclined surface 132 of the light exit surface 13.

As shown in FIG. 38, according to the hair control device 10 of the present embodiment, the plurality of light source units 20 (the first light source unit 201 and the second light source unit 202) are arranged such that the rays of the light L1 of the plurality of light source units 20 cross each other in a region around the hair matrix 912 (bulb) in the skin 92 in a state where the surface of the skin 92 is in contact with the light exit surface 13.

The light transmission member 3 is disposed in front of the light emitting unit 2. The light exit surface 13 is at least partially constituted by a surface of the light transmission member 3. In the present embodiment, the surface (front surface) of the light transmission member 3 on the outer side of the housing 1 constitutes (a whole of) the light exit surface 13. The light transmission member 3 allows the light of the light sources 22 of the plurality of light source units 20 of the light emitting unit 2 to pass through. Note that the illustration of the light transmission member 3 is omitted in FIG. 36.

As shown in FIG. 37, the light transmission member 3 includes a plurality of (two) light transmission pieces 30 (a first light transmission piece 301 and a second light transmission piece 302). Each of the plurality of light transmission pieces 30 has a plate shape. The plurality of light transmission pieces 30 are arranged to correspond (one-to-one) to the plurality of light source units 20. Each of the plurality of light transmission pieces 30 is disposed to face the light source 22 (a plurality of LEDs 221) of a corresponding light source unit 20 to increase the amount of light. That is, the light sources 22 of the light emitting unit 2 (light source units 20) are arranged to face the light transmission member 3 (light transmission pieces 30). This can reduce the distance from the light source 22 to the light exit surface 13 compared to a case where the light source 22 does not face the light transmission member 3.

A front surface of the first light transmission piece 301 serves as the first inclined surface 131 of the light exit surface 13, and a front surface of the second light transmission piece 302 serves as the second inclined surface 132 of the light exit surface 13. That is, the light exit surface 13 includes the front surface of the first light transmission piece 301 (the first inclined surface 131), and the front surface of the second light transmission piece 302 (the second inclined surface 132). The first light transmission piece 301 allows the light of the light source 22 of the first light source unit 201 to pass through. The second light transmission piece 302 allows the light of the light source 22 of the second light source unit 202 to pass through. The front surface of the first light transmission piece 301 and the front surface of the second light transmission piece 302 forms the bottom of the V-shaped groove of the recessed space S1. The first light transmission piece 301 and the second light transmission piece 302 are arranged such that the recess (recessed space S1) is formed by the first light transmission piece 301 and the second light transmission piece 302.

The recessed space S1 has a depth falling within the range greater than 0 mm and equal to or smaller than 30 mm, for example. According to the present disclosure, the depth of the recessed space S1 indicates a depth of the surface, from which the light is actually emitted, of the housing 1. In one specific example, the depth of the recessed space S1 is a dimension of the first inclined surface 131 (or the second inclined surface 132) measured along the Z-axis. The depth of the recessed space S1 may fall within the range equal to or greater than 2 mm and equal to or smaller than 30 mm.

The front surface of each light transmission piece 30 intersects with the optical axis of the corresponding light source unit 20 (at right angles, in the embodiment). Specifically, the front surface of the first light transmission piece 301 (i.e., the first inclined surface 131) intersects with the optical axis of the first light source unit 201 (at right angles, in the embodiment). The front surface of the second light transmission piece 302 (i.e., the second inclined surface 132) intersects with the optical axis of the second light source unit 202 (at right angles, in the embodiment). In short, according to the hair control device 10 of the present embodiment, the optical axis of the light emitting unit 2 intersects with the light exit surface 13 (at right angles, in the embodiment).

The light transmission member 3 is held by a holder 31. According to the hair control device 10 of the present embodiment, the holder 31 has a V-shaped plate shape when viewed laterally, and has openings into which the first light transmission piece 301 and the second light transmission piece 302 are respectively fitted. The holder 31 is fixed to the heat conductor 50 (see FIG. 37) with screws 35 (see FIG. 36).

As described above, according to the hair control device 10 of the present embodiment, the surface (front surface) of the light transmission member 3 constitutes the light exit surface 13. The hair control device 10 is adapted to be used in a state where the skin 92 introduced into the recessed space S1 is in contact with the front surface of the light transmission member 3 (the light exit surface 13) (see FIG. 38). In the usage state of the hair control device 10, therefore, the skin 92 in contact with the front surface of the light transmission member 3 (the light exit surface 13) is to be cooled by the heat of the skin 92 being taken away by the light transmission member 3. That is, the light transmission member 3 has a cooling capability for facilitating the cooling of the skin 92 in contact with the light transmission member 3. The light transmission member 3 having the cooling capability can suppress the increase in the temperature of the skin 92 around the surface layer. The nerves that detect the pain in the skin 92 will be less stimulated, and the uncomfortable feeling to be caused in a user can be reduced accordingly.

As shown in FIG. 37, the heat dissipation part 4 includes a first heat dissipation part 41, a second heat dissipation part 42, and a third heat dissipation part 43.

The first heat dissipation part 41 is coupled to the first light source unit 201. The first heat dissipation part 41 is designed to primarily dissipate the heat of the first light source unit 201.

The second heat dissipation part 42 is coupled to the second light source unit 202. The second heat dissipation part 42 is designed to primarily dissipate the heat of the second light source unit 202.

The first heat dissipation part 41 and the second heat dissipation part 42 constitute a heat dissipator 40 configured to dissipate heat generated in the light emitting unit 2 (the first light source unit 201 and the second light source unit 202). That is, the heat dissipator 40 includes the first heat dissipation part 41 configured to dissipate heat generated in the first light source unit 201 and the second heat dissipation part 42 configured to dissipate heat generated in the second light source unit 202.

The third heat dissipation part 43 is coupled to a Peltier device 52 of the cooler 5. The third heat dissipation part 43 is designed to primarily dissipate the heat of the cooler 5 (Peltier device 52).

The first heat dissipation part 41 has the heat conduction property. The first heat dissipation part 41 is made of metal, for example. The first heat dissipation part 41 includes a plurality of first heat dissipation fins 410 (first heat dissipation member 412; see FIG. 1), a first coupler 411, and a first supporting base part 415.

The first coupler 411 has a cuboid shape extending along the Y-axis. The first coupler 411 is disposed in a left part of the front opening of the first case 11 in the X-axis.

Each of the plurality of first heat dissipation fins 410 has a plate shape protruding rearward from a rear surface of the first coupler 411. The plurality of first heat dissipation fins 410 are coupled together with the first coupler 411. The plurality of first heat dissipation fins 410 and the first coupler 411 are formed integrally.

The plurality of first heat dissipation fins 410 are disposed in an inside space of the first case 11. The plurality of first heat dissipation fins 410 are arranged along the Y-axis. A gap is formed between two first heat dissipation fins 410 adjacent to each other in the Y-axis.

The plurality of first heat dissipation fins 410 and the first coupler 411 are fixed to a protruding piece 161 (see FIG. 37) of the fixing plate 16 with screws, thereby being held by the housing 1.

The first supporting base part 415 is disposed on the front side of the first coupler 411. The first supporting base part 415 and the first coupler 411 are coupled together with screws, for example.

The first supporting base part 415 supports the first light source unit 201. The first supporting base part 415 supports the substrate 21 of the first light source unit 201 and to thereby support the first light source unit 201. A front surface of the first supporting base part 415 is parallel to the first inclined surface 131 of the light exit surface 13. The first light source unit 201 is disposed on the front surface of the first supporting base part 415. The first light source unit 201 (the substrate 21) is in contact with the first supporting base part 415. The first supporting base part 415 is thermally connected to the first light source unit 201.

The second heat dissipation part 42 has the heat conduction property. The second heat dissipation part 42 is made of metal, for example. The second heat dissipation part 42 includes a plurality of second heat dissipation fins 420 (second heat dissipation member 422; see FIG. 1), a second coupler 421, and a second supporting base part 425.

The second coupler 421 has a cuboid shape extending along the Y-axis. The second coupler 421 is disposed in a right part of the front opening of the first case 11 in the X-axis.

Each of the plurality of second heat dissipation fins 420 has a plate shape protruding rearward from a rear surface of the second coupler 421. The plurality of second heat dissipation fins 420 are coupled together with the second coupler 421. The plurality of second heat dissipation fins 420 and the second coupler 421 are formed integrally.

The plurality of second heat dissipation fins 420 are disposed in the inside space of the first case 11. The plurality of second heat dissipation fins 420 are arranged along the Y-axis. A gap is formed between two second heat dissipation fins 420 adjacent to each other in the Y-axis.

The plurality of second heat dissipation fins 420 and the second coupler 421 are fixed to a protruding piece 162 (see FIG. 37) of the fixing plate 16 with screws, thereby being held by the housing 1.

The second supporting base part 425 is disposed on the front side of the second coupler 421. The second supporting base part 425 and the second coupler 421 are coupled together with screws, for example.

The second supporting base part 425 supports the second light source unit 202. The second supporting base part 425 supports the substrate 21 of the second light source unit 202 and to thereby support the second light source unit 202. A front surface of the second supporting base part 425 is parallel to the second inclined surface 132 of the light exit surface 13. The second light source unit 202 is disposed on the front surface of the second supporting base part 425. The second light source unit 202 (the substrate 21) is in contact with the second supporting base part 425. The second supporting base part 425 is thermally connected to the second light source unit 202.

The third heat dissipation part 43 has the heat conduction property. The third heat dissipation part 43 is made of metal, for example. The third heat dissipation part 43 includes a plurality of third heat dissipation fins 430 and a third coupler 431.

The third coupler 431 has a cuboid shape extending along the Y-axis. The third coupler 431 is disposed in a center part of the front opening of the first case 11 in the X-axis.

Each of the plurality of third heat dissipation fins 430 has a plate shape protruding rearward from a rear surface of the third coupler 431. The plurality of third heat dissipation fins 430 are coupled together with the third coupler 431. The plurality of third heat dissipation fins 430 and the third coupler 431 are formed integrally.

The plurality of third heat dissipation fins 430 are disposed in the inside space of the first case 11. The plurality of third heat dissipation fins 430 are arranged along the Y-axis. A gap is formed between two third heat dissipation fins 430 adjacent to each other in the Y-axis.

The plurality of third heat dissipation fins 430 and the third coupler 431 are fixed to a protruding piece 163 (see FIG. 37) of the fixing plate 16 with screws, thereby being held by the housing 1.

The first heat dissipation fin 410, the second heat dissipation fin 420, and the third heat dissipation fin 430 are located at the same position as one another in the Y-axis. Therefore, the gap of the first heat dissipation fins 410, the gap of the second heat dissipation fins 420, and the gap of the third heat dissipation fins 430 are located at the same position as one another in the Y-axis. Moreover, the gap of the first heat dissipation fins 410 (and also the gap of the second heat dissipation fins 420 and the gap of the third heat dissipation fins 430) is located at the same position as the air vent 111 (see FIG. 35) of the first case 11 in the Y-axis.

The cooler 5 is disposed to cool the light transmission member 3 (the plurality of light transmission pieces 30). The cooler 5 is disposed in the housing 1.

According to the present embodiment, as shown in FIG. 37, the cooler 5 includes the Peltier device 52. The Peltier device 52 is disposed in the second case 12. The Peltier device 52 is disposed such that the "cold" side is directed forward.

The heat conductor 50 is disposed on a front surface of the Peltier device 52. The heat conductor 50 has the heat conduction property. The heat conductor 50 is made of metal, for example. The heat conductor 50 is in contact with the front surface of the Peltier device 52. The heat conductor 50 is thermally connected to the front surface of the Peltier device 52.

As shown in FIG. 37, the heat conductor 50 is coupled to the holder 31 such that the light transmission member 3 (the plurality of light transmission pieces 30) is in contact with the heat conductor 50. The front surface of the Peltier device 52 is thermally connected to the light transmission member 3 (the plurality of light transmission pieces 30) via the heat conductor 50.

The cooler 5 (Peltier device 52) can facilitate the cooling of the light transmission member 3 (the plurality of light transmission pieces 30), which can suppress the increase in the temperature of the skin 92 around the surface layer in contact with the light transmission member 3. The uncomfortable feeling to be caused in a user can be reduced.

The cooler 5 (Peltier device 52) is controlled by the controller 71 such that the temperature of the surface of the skin 92 in contact with the light transmission member 3 falls within a predetermined temperature range, for example. The predetermined temperature range is a range of -5°C to 35°C, for example. Making the temperature of the surface of the skin 92 equal to or greater than -5°C can suppress the user's uncomfortable feeling due to the low temperature state of the skin 92. Making the temperature of the surface of the skin 92 equal to or smaller than 35°C can suppress the user's uncomfortable feeling due to the high temperature state of the skin 92.

The hair control device 10 may include a temperature sensor configured to measure the temperature of the skin 92. The controller 71 may be configured to operate the cooler 5 based on the measurement result of the temperature sensor. It should be noted that the hair control device 10 can make the temperature of the surface of the skin 92 fall within the above predetermined temperature range without the temperature sensor by, for example, preliminary setting the operation condition of the cooler 5 appropriately or allowing the user to turn the cooler 5 on and off by a switch and the like.

It is preferable that the temperature of the light transmission member 3 and also the temperature of the skin 92 is controlled to fall within a desired temperature range (-5°C to 35°C, for example) as described above. Accordingly, the Peltier device 52 is desirably provided to the light transmission member 3, since the Peltier device 52 is easy to control its cooling rate.

On the other hand, the temperature of the light emitting unit 2 may be greater than the desired temperature range of the light transmission member 3 (-5°C to 35°C, for example), as far as the increased temperature does not affect the luminous efficacy of the light emitting unit 2 or excessively heat the light transmission member 3. The temperature of the light emitting unit 2 does not need to be controlled strictly compared to that of the light transmission member 3.

As shown in FIG. 37, the cooler 5 further includes a cooling fan 51. The cooling fan 51 is disposed in the third case 17. The cooling fan 51 is disposed in the housing 1 and configured to send out the wind forward (flowing to the positive side in the Z-axis).

The wind sent out from the cooling fan 51 reaches a first heat dissipation part 41 to a third heat dissipation part 43 (described later) of the heat dissipation part 4 to take the heat away from the first heat dissipation part 41 to the third heat dissipation part 43. This can facilitate the cooling of the first heat dissipation part 41 to the third heat dissipation part 43. Accordingly, the cooling of the light emitting unit 2 is facilitated, and also the cooling of the light transmission member 3 is facilitated.

The wind sent out from the cooling fan 51 also reaches a space around the heat conductor 50, and forms a flow of the air in a space between the predetermined surface 500 of the heat conductor 50 and the heat dissipator 40. In short, the hair control device 10 of the present embodiment includes a fan (cooling fan51) disposed to form an air flow flowing in a space between the predetermined surface 500 of the heat conductor 50 and the heat dissipator 40.

The operations unit 6 is configured to be operated by the operator. The operations unit 6 includes a manual switch provided on the outer peripheral surface of the housing 1, for example.

The controller 71 is configured to control the operation of the hair control device 10. The controller 71 is configured to control the operations of the light emitting unit 2 and the cooler 5.

According to the hair control device 10 of the present embodiment, the light exit surface 13 has a recessed shape recessed inward the housing 1. The hair control device 10 of the present embodiment can reduce the uncomfortable feeling to be caused in the user, as with the hair control device 10 of the embodiment 1.

In the following, details of positional relations of the components of the hair control device 10 of the present embodiment will be described with reference to FIGS. 37, 38.

As shown in FIG. 37, according to the hair control device 10 of the present embodiment, the first supporting base part 415 of the first heat dissipation part 41, the heat conductor 50, and the second supporting base part 425 of the second heat dissipation part 42 are arranged in this order along one axis (X-axis). Moreover, the first supporting base part 415 of the first heat dissipation part 41 faces the heat conductor 50 with a gap therebetween. The second supporting base part 425 of the second heat dissipation part 42 faces the heat conductor 50 with a gap therebetween.

Specifically, as shown in FIG. 37, the heat conductor 50 has the predetermined surface 500. The predetermined surface 500 is at least a part of the surface of the heat conductor 50 and faces the heat dissipator 40 (the first heat dissipation part 41 and the second heat dissipation part 42). The predetermined surface 500 of the heat conductor 50 faces the heat dissipator 40 with a gap between the predetermined surface 500 and the heat dissipator 40.

According to the hair control device 10 of the present embodiment, the predetermined surface 500 of the heat conductor 50 faces both the first heat dissipation part 41 and the second heat dissipation part 42 with a gap therebetween. Specifically, the predetermined surface 500 of the heat conductor 50 includes a first region 501 and a second region 502. The first region 501 of the predetermined surface 500 of the heat conductor 50 faces the first heat dissipation part 41 (the first supporting base part 415) with a gap therebetween. The second region 502 of the predetermined surface 500 of the heat conductor 50 faces the second heat dissipation part 42 (the second supporting base part 425) with a gap therebetween. The first region 501 of the predetermined surface 500 of the heat conductor 50 faces the first heat dissipation part 41 in the one axis (X-axis). The second region 502 of the predetermined surface 500 of the heat conductor 50 faces the second heat dissipation part 42 in the one axis (X-axis).

According to the hair control device 10 of the present embodiment, the predetermined surface 500 of the heat conductor 50 and the heat dissipator 40 face each other with a gap G1 (see FIG. 38) left therebetween. That is, the predetermined surface 500 of the heat conductor 50 and the heat dissipator 40 face each other with an air space between the predetermined surface 500 and the heat dissipator 40.

As shown in FIG. 37, the first region 501 of the predetermined surface 500 of the heat conductor 50 is parallel to a surface, facing the first region 501, of the heat dissipator 40 (the first heat dissipation part 41). The second region 502 of the predetermined surface 500 of the heat conductor 50 is parallel to a surface, facing the second region 502, of the heat dissipator 40 (the second heat dissipation part 42). In short, the predetermined surface 500 of the heat conductor 50 and the surface of the heat dissipator 40 facing the predetermined surface 500 are parallel to each other.

When the cooler 5 (Peltier device 52) is operated to cool the heat conductor 50, a dew condensation water (water droplet W1; see FIG. 38) may appear on the predetermined surface 500 due to the dew condensation depending on the condition including the temperature and the humidity of the environment where the hair control device 10 is used. In this regard, according to the hair control device 10 of the present embodiment, a distance X5 between the predetermined surface 500 of the heat conductor 50 and the heat dissipator 40 is determined such that the water droplet W1 appeared on the predetermined surface 500 by the dew condensation can be brought into contact with the heat dissipator 40. That is, the distance X5 (see FIG. 38) between the predetermined surface 500 of the heat conductor 50 and the heat dissipator 40 is set to allow the dew condensation water on the predetermined surface 500 generated by the dew condensation to be in contact with the heat dissipator 40. The distance X5 may indicate a shortest distance between the predetermined surface 500 of the heat conductor 50 and the heat dissipator 40, for example.

The heat dissipator 40 is thermally connected to the light emitting unit 2. Therefore, the temperature of the heat dissipator 40 is increased according to the heat generated in the light emitting unit 2 and transmitted to the heat dissipator 40. When the dew condensation water (water droplet W1) generated on the predetermined surface 500 of the heat conductor 50 comes in contact with the heat dissipator 40 of which the temperature is increased, the dew condensation water is heated by the heat dissipator 40 and is vaporized, and takes away the heat (heat of vaporization) from the heat dissipator 40. This can facilitate the cooling of the heat dissipator 40. The cooling of the light emitting unit 2 is also facilitated by the cooling of the heat dissipator 40.

According to the hair control device 10 of the present embodiment, specifically, the distance X5 falls within the range equal to or greater than 50 µm and equal to or smaller than 20 mm. The distance X5 may be equal to or smaller than 5 mm, or equal to or smaller than 2 mm, or equal to or smaller than 1 mm. When the distance X5 between the predetermined surface 500 of the heat conductor 50 and the heat dissipator 40 facing each other with the air space (gap G1) therebetween falls within the above predetermined range, the dew condensation water generated on the predetermined surface 500 by the dew condensation tends to come in contact with the heat dissipator 40. This can facilitate the cooling of the heat dissipator 40, and to facilitate the cooling of the light emitting unit 2.

According to the hair control device 10 of the present embodiment, the heat dissipator 40 is out of contact from the heat conductor 50 but faces the heat conductor 50 with the gap G1 (air space) left therebetween. This can prevent the direct transmission of the heat between the heat dissipator 40 and the heat conductor 50. If the heat conductor 50 and the heat dissipator 40 are in contact with each other (and thermally connected to each other), the Peltier device 52 have to cool not only the heat conductor 50 but also the heat dissipator 40. This may increase the power consumption in the cooler 5 (Peltier device 52). Contrary to this, the hair control device 10 of the present embodiment can suppress the increase in the power consumption in the cooler 5, since the heat dissipator 40 is apart from the heat conductor 50.

According to the hair control device 10 of the present embodiment, the first heat dissipation part 41, the heat conductor 50 and the second heat dissipation part 42 are arranged in this order along the one axis (X-axis), as described above. Moreover, the first region 501 of the predetermined surface 500 of the heat conductor 50 faces the first heat dissipation part 41 in the one axis) and also the second region 502 of the predetermined surface 500 of the heat conductor 50 faces the second heat dissipation part 42 in the one axis. According to this, the cooling of the respective light sources 22 of the two light source units 20 (the first light source unit 201 and the second light source unit 202) can be performed by a single heat conductor 50. This can minimize the number of the Peltier device 52.

The hair control device 10 of the present embodiment includes the fan (cooling fan 51), as described above. The cooling fan 51 is disposed to form the air flow flowing in the space (gap G1) between the predetermined surface 500 of the heat conductor 50 and the heat dissipator 40. This can facilitate the vaporization of the condensation water generated on the predetermined surface 500 and in contact with the heat dissipator 40. This can further facilitate the cooling of the heat dissipator 40 and facilitate the cooling of the light emitting unit 2.

### (3.3) Variations of Embodiment 3

Variations of the embodiment 3 will be described. In the following description, the embodiment 3 described above may be referred to as a "basic example (of the embodiment 3)". In the description of the variations, components that are substantially the same as those of a basic aspect may be appropriately omitted. The basic example and the variations may be readily modified depending on a design choice or any other factor, without departing from a true spirit and scope of the present disclosure.

### (3.3.1) First Variation

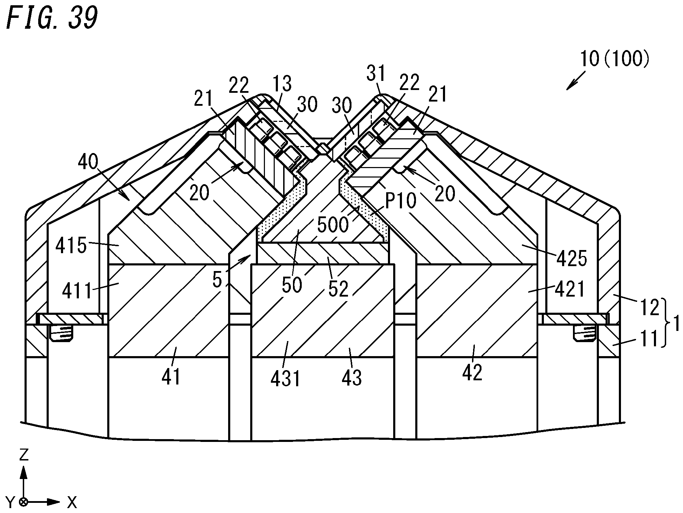

A hair control device 10 (photocosmetic device 100) of the present variation will be described with reference to FIG. 39. The hair control device 10 of the present variation differs from the hair control device 10 of the basic example in a configuration of a region between a predetermined surface 500 of a heat conductor 50 and a heat dissipator 40.

As shown in FIG. 39, according to the hair control device 10 of the present variation, porous member P10 is disposed in the region between the predetermined surface 500 of the heat conductor 50 and the heat dissipator 40. The porous member P10 is a thermal insulation member less likely to transfer the heat. That is, the hair control device 10 of the present variation includes the porous member P10 disposed between the predetermined surface 500 of the heat conductor 50 and the heat dissipator 40. The porous member P10 includes at least one material selected from the group consisting of mesoporous silica, silica gel, activated carbon, and zeolite. As shown in FIG. 39, the porous member P10 is in contact with both of the predetermined surface 500 of the heat conductor 50 and the heat dissipator 40.

According to the hair control device 10 of the present variation, a water droplet W1 of dew condensation water (see FIG. 38) on the predetermined surface 500 generated by the cooling of the heat conductor 50 is brought into contact with the heat dissipator 40 and then is vaporized, and takes the heat (heat of vaporization) away from the heat dissipator 40. This can facilitate the cooling of a light emitting unit 2 thermally connected to the heat dissipator 40.

Moreover, according to the hair control device 10 of the present variation, the dew condensation water (water droplet) generated on the predetermined surface 500 of the heat conductor 50 may move along the porous member P10 to reach the heat dissipator 40. Accordingly, the dew condensation water generated on the predetermined surface 500 by the dew condensation easily comes in contact with the heat dissipator 40. As a result, the cooling of the heat dissipator 40 and also the cooling of the light emitting unit 2 is facilitated.

According to the hair control device 10 of the present variation, the direct transition of the heat between the heat dissipator 40 and the heat conductor 50 is also less likely to occur. This can prevent the increase in the power consumption of the cooler 5 (Peltier device 52).

Note that the porous member P10 does not have to be in contact with both of the predetermined surface 500 of the heat conductor 50 and the heat dissipator 40. In an alternative example, the porous member P10 may be in contact with the predetermined surface 500 of the heat conductor 50 only and face the heat dissipator 40 with an air space (gap) between the porous member P10 and the heat dissipator 40.

### (3.3.2) Second Variation

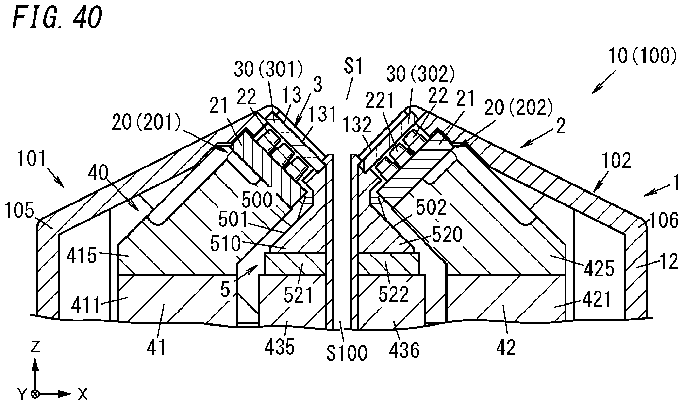

A hair control device 10 (photocosmetic device 100) of the present variation will be described with reference to FIG. 40. The hair control device 10 of the present variation differs from the hair control device 10 of the basic example in a configuration of a housing 1 (second case 12), a heat dissipation part 4, a cooler 5 etc.

As shown in FIG. 40, according to the hair control device 10 of the present variation, the housing 1 (specifically, the second case 12) includes a first housing part 105 in which a first light source unit 201 is housed and a second housing part 106 in which a second light source unit 202 is housed. The first housing part 105 and the second housing part 106 are disposed separately (separated) from each other in a direction in which the first light source unit 201 and the second light source unit 202 are arranged.

According to the hair control device 10 of the present variation, the cooler 5 includes a first Peltier device 521 and a second Peltier device 522. The first Peltier device 521 is housed in the first housing part 105 and configured to cool a first light transmission piece 301. The second Peltier device 522 is housed in the second housing part 106 and configured to cool a second light transmission piece 302.

A first heat conductor 510 is disposed on a front surface of the first Peltier device 521. The first heat conductor 510 is in contact with the first light transmission piece 301. The front surface of the first Peltier device 521 is thermally connected to the first light transmission piece 301 via the first heat conductor 510.

A second heat conductor 520 is disposed on a front surface of the second Peltier device 522. The second heat conductor 520 is in contact with the second light transmission piece 302. The front surface of the second Peltier device 522 is thermally connected to the second light transmission piece 302 via the second heat conductor 520.

According to the hair control device 10 of the present variation, a third heat dissipation part 43 includes a first part 435 coupled to the first Peltier device 521 and a second part 436 coupled to the second Peltier device 522.

Specifically, the hair control device 10 of the present variation includes a first light source block 101 and a second light source block 102.

The first light source block 101 includes the first light source unit 201, the first light transmission piece 301, a first heat dissipation part 41, the first Peltier device 521, the first heat conductor 510, the first part 435 of the third heat dissipation part 43, and the first housing part 105. The first housing part 105 has a box shape and houses therein the first light source unit 201, the first heat dissipation part 41, the first Peltier device 521, the first heat conductor 510, and the first part 435 of the third heat dissipation part 43. The first housing part 105 holds the first light transmission piece 301 such that the first light transmission piece 301 is positioned on an optical axis of the first light source unit 201. According to the first light source block 101, the first light source unit 201 and the first light transmission piece 301 are configured to be cooled by the first heat dissipation part 41, the first Peltier device 521, the first heat conductor 510 (including the dew condensation water generated on a surface 501 of the first heat conductor 510) and the first part 435 of the third heat dissipation part 43.

The second light source block 102 includes the second light source unit 202, the second light transmission piece 302, a second heat dissipation part 42, the second Peltier device 522, the second heat conductor 520, the second part 436 of the third heat dissipation part 43, and the second housing part 106. The second housing part 106 has a box shape and houses therein the second light source unit 202, the second heat dissipation part 42, the second Peltier device 522, the second heat conductor 520, and the second part 436 of the third heat dissipation part 43. The second housing part 106 holds the second light transmission piece 302 such that the second light transmission piece 302 is positioned on an optical axis of the second light source unit 202. According to the second light source block 102, the second light source unit 202 and the second light transmission piece 302 are configured to be cooled by the second heat dissipation part 42, the second Peltier device 522, the second heat conductor 520 (including the dew condensation water generated on a surface 502 of the second heat conductor 520) and the second part 436 of the third heat dissipation part 43.

As shown in FIG. 40, according to the hair control device 10 of the present variation, there is a space S100 between the first light source block 101 and the second light source block 102.

According to the hair control device 10 of the present variation, the first light source block 101 and the second light source block 102 are thermally separated from each other. The temperature control on (cooling of) the first light source block 101 and the temperature control on (cooling of) the second light source block 102 can be performed individually. Therefore, even when a temperature difference generates between the first light source block 101 (the first light transmission piece 301 and the first light source unit 201) and the second light source block 102 (the second light transmission piece 302 and the second light source unit 202), their temperatures (cooling) can be controlled individually.

### (3.3.3) Other Variations

In one variation, a housing 1 does not have to house therein an entire of a light emitting unit 2. A housing 1 may house a light emitting unit 2 with a part of a light emitting unit 2 (e.g., a part of a substrate 21) protruding from the housing 1, for example.

In one variation, a substrate 21 and a light transmission member 3 (a first light transmission piece 301 or a second light transmission piece 302) may not have to be in parallel to each other, but may be inclined. In this case, an inclined angle between a light transmission member 3 (light transmission piece 30) and a substrate 21 may be set in consideration with a loss of light caused by the total refection. The inclined angle between the light transmission piece 30 and the substrate 21 may be set such that (majority) of the light emitted from the light source 22 and traveling in the light transmission piece 30 enters a light exit surface 13 (front surface of the light transmission piece 30) by an incident angle equal to or smaller than the critical angle. The critical angle means the smallest angle of incidence that yields total reflection. The critical angle depends on the refractive index of the material of the light transmission piece 30 and the refractive index of the air. Disposing the substrate 21 and the light transmission member 3 in this manner can reduce the loss of light caused by the total reflection, and makes it easy to illuminate the desired amount of the light on the skin 92.

In one variation, a light source unit 20 may include an optical member configured to change a direction of an optical path of light of a light source 22 by the reflection or the refraction, such as a mirror, a half mirror, or a prism. In this case, an optical axis of the light source unit 20 may not coincide with the optical axis of the light source 22.

In one variation, a light transmission member 3 (light transmission piece 30) may have a shape serving as a lens.

In one variation, a predetermined surface 500 of a heat conductor 50 does not have to be in parallel to a surface of a heat dissipator 40 as far as it faces the heat dissipator 40.

In one variation, the number of light source units 20 included in a light emitting unit 2 is not limited to two, but may be three or more.

In one variation, a light exit surface 13 does not have to have a V-groove shape when viewed in the Y-axis, but may have a recessed groove shape when viewed in the Y-axis for example. In this case, the light exit surface 13 may include a first inclined surface 131 and a second inclined surface 132, which extend from both ends of a bottom of the recessed groove at right angles, and which face each other.

In one variation, a cooling fan 51 may be disposed in a housing 1 to sent out a wind rearward (flowing to the negative side in the Z-axis).

### (4) Embodiment 4

### (4.1) Overview of Embodiment 4

According to the laser beauty culture instrument described in the reference document (JP2000-153003A), when an optical member such as the spherical lens is cooled, a water (dew condensation water) may possibly stick to the optical member due to the dew condensation. When the light emitted from the light source reaches the dew condensation water, the light will then be scattered or refracted by the water. This may decrease the light extraction efficiency of the device. The decrease in the light extraction efficiency of the device causes the decrease in the intensity of the light illuminated on the skin, which may decrease the treatment efficiency for treating the skin.

The photocosmetic device 100 of the present embodiment is developed in view of the above circumstance, and an object thereof is to provide the photocosmetic device which can reduce the decline in the treatment efficiency for treating the skin.

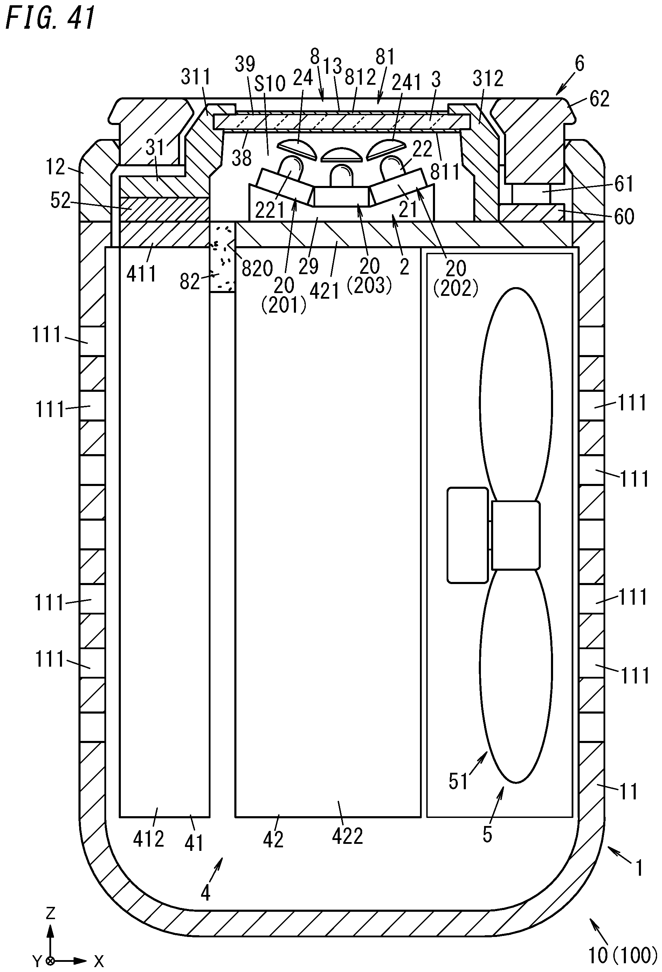

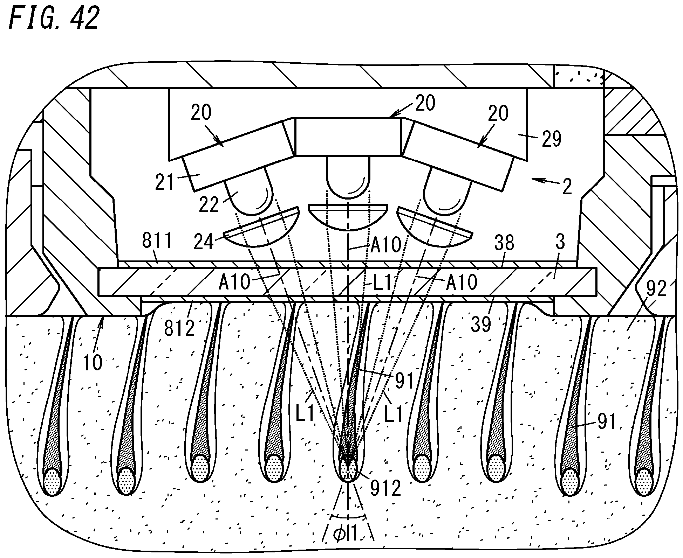

The photocosmetic device 100 of the present embodiment (see FIG. 41) is a device for illuminating light to a skin 92 (see FIG. 42) to cause absorption of the light in the skin 92 to treat the skin 92 with the function of the absorbed light.

As shown in FIG. 41, the photocosmetic device 100 of the present embodiment includes a light transmission member 3. The light transmission member 3 has a light incident surface 38 through which light of a light emitting unit 2 enters and a light emerging surface 39 from which the light entered through the light incident surface 38 is emerged to the outside of the housing 1. The light transmission member 3 has a cooling function for facilitating the cooling of the skin 92 in contact with the light transmission member 3. The photocosmetic device 100 of the present embodiment further includes a heat dissipation part 4 for dissipating the heat of the light transmission member 3, and a cooler 5 for cooling the light transmission member 3. The cooling function of the light transmission member 3 can be realized by the heat dissipation part 4 and the cooler 5 cooling the light transmission member 3. The photocosmetic device 100 of the present embodiment is used in a state where the light emerging surface 39 of the light transmission member 3 is in contact with a surface of the skin 92, as shown in FIG. 42, for example. Therefore, the skin 92 in contact with the light emerging surface 39 of the light transmission member 3 is cooled by the heat of the skin 92 being taken away by the light transmission member 3. This can suppress the increase in the temperature of the skin 92 around the surface layer. The nerves that detect the pain in the skin 92 will be less stimulated, and the uncomfortable feeling to be caused in a user can be reduced accordingly.

However, in some conditions of the environment including the temperature and the humidity where the device is used, the light transmission member 3 having the cooling function may cause the generation of the water (dew condensation water) on at least one of the light incident surface 38 or the light emerging surface 39 of the light transmission member 3 due to the dew condensation. When the light emitted from the light emitting unit 2 reaches the dew condensation water, the light may be scattered or refracted by the dew condensation water, which may change the traveling direction of the light. In this case, the light of the light emitting unit 2 may be less likely to reach a desired region (e.g., a region around the hair matrix 912) in the skin 92, which may decrease the treatment efficiency for treating the skin.

In this regard, the photocosmetic device 100 of the present embodiment further includes a dew condensation inhibitor 8. The dew condensation inhibitor 8 is configured to prevent dew condensation from occurring on the light transmission member 3 and/or to inhibit a dew condensation water on the light transmission member 3 generated by dew condensation from staying on the light transmission member 3. This can facilitate to prevent the occurrence of the dew condensation on the light transmission member 3 or to remove the dew condensation water. Accordingly, the light emitted from the light emitting unit 2 is less likely to be scattered or refracted by the dew condensation water. As a result, the light of the light emitting unit 2 is likely to reach the desired region in the skin 92. The decline in the treatment efficiency for treating the skin can be reduced.

### (4.2) Details of Embodiment 4

As shown in FIG. 41, the hair control device 10 includes a housing 1, a light emitting unit 2, the light transmission member 3, a heat dissipation part 4, the cooler 5, an operations unit 6, a controller 71 (see FIG. 5), a power source 72 (see FIG. 5), and the dew condensation inhibitor 8.

As shown in FIG. 41, the housing 1 includes a first case 11 and a second case 12.

The light emitting unit 2 is configured to emit light to be irradiated to the skin 92. As shown in FIG. 41, the light emitting unit 2 is housed in the housing 1. The light emitting unit 2 is disposed in a housing space S10 of the housing 1. The housing space S10 is a space surrounded by the light transmission member 3, a holder 31 (described later), and the heat dissipation part 4. That is, the housing 1 has the housing space S10 in which the light emitting unit 2 is housed.

As shown in FIG. 41, the light emitting unit 2 includes a plurality of (three, in the embodiment) light source units 20 (a first light source unit 201, a second light source unit 202, and a third light source unit 203) and a holding member 29.

As shown in FIG. 41, each of the plurality of light source units 20 includes a substrate 21 and a light source 22.

The light source 22 includes a plurality of LEDs 221, for example. The plurality of LEDs 221 are disposed on the substrate 21. The light source 22 includes eight-pieces of LEDs 221, for example. The eight-pieces of LEDs 221 are arranged in a line along the Y-axis when viewed from the front side.

Each of the plurality of LEDs 221 is mounted on the substrate 21 such that it exhibits a light distribution property where the direction of the peak intensity is aligned with the normal direction of the substrate 21. In other words, each of the plurality of LEDs 221 has an optical axis along the normal direction of the substrate 21.

In each light source unit 20, the light source 22 has an optical axis along the normal direction of the substrate 21. According to the present embodiment, the feature the "optical axis of the light source 22 including the plurality of LEDs 221" indicates an imaginary plane (planar axis) including the optical axes of the plurality of LEDs 221.

In the hair control device 10 of the present embodiment, each of the plurality of light source units 20 has an optical axis A10 (see FIG. 42). In the hair control device 10 of the present embodiment, the optical axis A10 of the light source unit 20 coincides with the optical axis of the light source 22. The optical axis A10 of the light source unit 20 is along the normal direction of the substrate 21.

The holding member 29 has the heat conduction property. The holding member 29 holds the substrates 21 of the plurality of (three) light source units 20. As shown in FIG. 42, the plurality of light source units 20 are held by the holding member 29 such that the normal directions of the substrates 21 of the plurality of light source units 20 cross one another. In the present embodiment, the optical axes A10 of the plurality of (three) light source units 20 are non-parallel to one another and cross one another at one point. As shown in FIG. 42, the optical axes A10 of the plurality of (three) light source units 20 cross one another on an outside of the housing 1 and in front of the light transmission member 3.

As shown in FIG. 42, the plurality of (three) light source units 20 are arranged such that the rays of the light L1 of the plurality of light source units 20 cross one another in a region around the hair matrix 912 (bulb) in the skin 92 in a state where the surface of the skin 92 is in contact with the light transmission member 3.

Each of the plurality of light source units 20 further includes an optical member 24. The optical member 24 is positioned on the optical axis of the light source 22. The optical member 24 includes a plurality of lenses 241 arranged to correspond one-to-one to the plurality of LEDs 221, for example. The lens 241 is a convex lens, in the embodiment. The lens 241 is disposed in front of a corresponding LED 221. The lenses 241 are designed such that the rays of the light L1 of the plurality of light source units 20 have a minimum width at a position where the rays of the light L1 cross one another, for example.

As shown in FIG. 41, the light transmission member 3 is disposed in front of the light emitting unit 2. The light transmission member 3 is located in front of the light sources 22 of the plurality of light source units 20. In the present embodiment, the light transmission member 3 of a single plate covers a whole of the plurality of light source units 20 from the front side.

The light transmission member 3 has a plate shape having a front surface and a rear surface. The rear surface of the light transmission member 3 faces the light emitting unit 2. The rear surface of the light transmission member 3 constitutes the light incident surface 38 through which the light of the light emitting unit 2 enters. The front surface of the light transmission member 3 constitutes the light emerging surface 39 (light exit surface 13) from which the light entered through the light incident surface 38 is emerged to the outside of the housing 1. The light transmission member 3 has the light transmission property allowing the light emitted from the light emitting unit 2 to pass through.

As shown in FIG. 41, the light transmission member 3 is held by the holder 31. The holder 31 has the heat conduction property. The holder 31 is made of metal, for example. The holder 31 holding the light transmission member 3 and having the heat conduction property can further facilitate the cooling of the body in contact with the light transmission member 3.

As shown in FIG. 41, the holder 31 includes a first holding arm 311 and a second holding arm 312. The first holding arm 311 pinches a left side part of the light transmission member 3 and the second holding arm 312 pinches a right side part of the light transmission member 3, whereby the holder 31 holds the light transmission member 3.

The cooler 5 includes a cooling fan 51 and a Peltier device 52. The cooling fan 51 is disposed in the first case 11 to be arranged side-by-side with a second heat dissipation member 422 and a first heat dissipation member 412 in the X-axis. The Peltier device 52 is disposed in the second case 12 and disposed between the first holding arm 311 of the holder 31 and the first coupler 411.

The cooling fan 51 is disposed to send out the wind leftward (flowing to the negative side in the X-axis). The wind sent out from the cooling fan 51 reaches the second heat dissipation member 422 and the first heat dissipation member 412 to take the heat away from the second heat dissipation member 422 and the first heat dissipation member 412. This can facilitate the cooling of the second heat dissipation member 422 and the first heat dissipation member 412. As a result, the light transmission member 3 thermally connected with the first heat dissipation member 412 and the second heat dissipation member 422 is cooled.

The Peltier device 52 is disposed such that the "cold" side is directed forward. Since the Peltier device 52 is provided to the first holding arm 311, the light transmission member 3 held by the first holding arm 311 is cooled by the Peltier device 52.

The dew condensation inhibitor 8 is configured to prevent dew condensation from occurring on the light transmission member 3 and/or to inhibit a dew condensation water on the light transmission member 3 generated by dew condensation from staying on the light transmission member 3. According to the hair control device 10 of the present embodiment, the dew condensation inhibitor 8 includes at least one of: a dew condensation preventer configured to prevent the dew condensation from occurring on the light transmission member 3; or a dew condensation water staying inhibitor configured to inhibit the dew condensation water generated on the light transmission member 3 by the dew condensation from staying on the light transmission member 3. According to the present disclosure, the feature "prevent the dew condensation from occurring on the light transmission member 3" indicates any of the situations of completely preventing the occurrence of the dew condensation or making it harder the occurrence of the dew condensation. According to the present disclosure, the feature "inhibit the dew condensation water generated on the light transmission member 3 by the dew condensation from staying on the light transmission member 3" indicates any of the situations of making it harder for the dew condensation water to remain on the light transmission member 3 or completely removing the dew condensation water from the light transmission member 3.

As shown in FIG. 41, the hair control device 10 of the present embodiment includes a coating 81 and a dehumidifying material 82, each of which serves as the dew condensation inhibitor 8.

The coating 81 is provided on a surface of the light transmission member 3. The coating 81 includes a thin film. The coating 81 has the light transmission property allowing the light emitted from the light emitting unit 2 to pass through.

The coating 81 is one example of the dew condensation water staying inhibitor. The coating 81 has the hydrophobicity. According to the present disclosure, the feature "the coating 81 has the hydrophobicity" indicates that the contact angle of the coating 81 with respect to the water is greater than a predetermined angle. The contact angle of the coating 81 having the hydrophobicity with respect to the water may be equal to or greater than 90°, or may be equal to or greater than 110°, or may be equal to or greater than 150°, for example. When the coating 81 has the contact angle equal to or greater than 90°, the water on the surface of the coating 81 generated by the dew condensation is likely to have a spherical shape (to form a water droplet). By tilting the light transmission member 3 with respect to the horizon direction, the water droplet (dew condensation water) on the surface of the coating 81 is moved and thus can be removed easily.

The coating 81 is made of the material exhibiting a large contact angle with respect to the water. The coating 81 may include a glass-coating of silicon dioxide, a fluorine coating, or a coated film made by applying hydrophobic fat-soluble components, for example.

The coating 81 is provided to at least one of the light incident surface 38 or the light emerging surface 39 of the light transmission member 3. According to the hair control device 10 of the present embodiment, the coating 81 includes a first coating 811 provided to the light incident surface 38 of the light transmission member 3 and a second coating 812 provided to the light emerging surface 39 of the light transmission member 3. That is, in the hair control device 10 of the present embodiment, the coatings 81 are provided to both of the light incident surface 38 and the light emerging surface 39 of the light transmission member 3. The first coating 811 and the second coating 812 may be made of the same material or alternatively made of the different materials.

The first coating 811 is provided to an entire of the light incident surface 38 of the light transmission member 3. The first coating 811 allows the dew condensation water generated on the light incident surface 38 of the light transmission member 3 (specifically, generated on the surface of the first coating 811) to be easily moved over the surface of the first coating 811 and then be removed from the light transmission member 3.

The second coating 812 is provided to an entire of the light emerging surface 39 of the light transmission member 3. The second coating 812 allows the dew condensation water generated on the light emerging surface 39 of the light transmission member 3 (specifically, generated on the surface of the second coating 812) to be easily moved over the surface of the second coating 812 and then be removed from the light transmission member 3.

A passage (grove) allowing the water to flow through may be formed in the first holding arm 311. A passage (grove) allowing the water to flow through may be formed in the second holding arm 312. The passage of the first holding arm 311 or the second holding arm 312 may be provided in the surface of the first holding arm 311 or the second holding arm 312 around a portion holding the light transmission member 3 and extend along the Y-axis. The water (water droplet) generated on the surface of the coating 81 by the dew condensation moves over the surface of the coating 81 to reach the first holding arm 311 or the second holding arm 312 and then is discharged through the passage. The passage formed in the second holding arm 312 may extend to reach the light emitting unit 2. When the water reaches the light emitting unit 2 through the passage and then evaporated, the light emitting unit 2 can be cooled by the water.

The dehumidifying material 82 is one example of the dew condensation preventer. The dehumidifying material 82 is disposed in the housing 1. The dehumidifying material 82 includes at least one material selected from the group consisting of calcium chloride, silica gel, quicklime, and zeolite. The dehumidifying material 82 absorbs the moisture in the housing 1 to decrease the humidity in the housing 1. The dehumidifying material 82 is configured to prevent the dew condensation on the light incident surface 38 of the light transmission member 3 (i.e., a surface of the light transmission member 3 on an inner side of the housing 1).

According to the hair control device 10 of the present embodiment, the dehumidifying material 82 is disposed in a passage 820 that is connected to the housing space S10 and that allows an air to pass through, as shown in FIG. 41. As described above, the housing space S10 is a space in which the light emitting unit 2 is disposed, of the housing 1. According to the hair control device 10 of the present embodiment, the dehumidifying material 82 is disposed in an entrance opening of the housing space S10 (i.e., disposed in an opening of the housing space S10 connected to the passage 820). The dehumidifying material 82 disposed in the passage 820 can reduce the humidity of the air before the air enters the housing space S10. Moreover, the moisture in the housing space S10 is absorbed in the dehumidifying material 82, which can reduce the humidity in the housing space S10. This can prevent the dew condensation from occurring on the light incident surface 38 of the light transmission member 3. The dehumidifying material 82 may be disposed in the housing space S10 in addition to or in alternative to the passage 820. This can reduce the humidity in the housing space S10 to prevent the dew condensation from occurring on the light incident surface 38 of the light transmission member 3.

According to the hair control device 10 of the present embodiment, the dehumidifying material 82 is disposed near the light emitting unit 2. The dehumidifying material 82 is thermally connected to the light source 22 of the light emitting unit 2. Specifically, the dehumidifying material 82 is in contact with the second coupler 421 of the heat dissipation part 4 and thus is thermally connected to the light source 22 of the light emitting unit 2 via the holding member 29 and the second coupler 421. The dehumidifying material 82 is therefore heated by the heat generated in the light source 22. The moisture once absorbed in the dehumidifying material 82 may be desorbed from the dehumidifying material 82 by the heat generated in the light source 22. This can recover the function of the dehumidifying material 82. In short, the dehumidifying material 82 is arranged such that the moisture absorbed in the dehumidifying material 82 is evaporated by the heat generated in the light emitting unit 2. At least part of the moisture desorbed (evaporated) from the dehumidifying material 82 is discharged to the outside of the housing 1 through air vents 111.

In sum, the hair control device 10 of the present embodiment includes the dew condensation inhibitor 8. The dew condensation inhibitor 8 includes at least one of (in the embodiment, both of) the dew condensation preventer (dehumidifying material 82) configured to prevent the dew condensation from occurring on the light transmission member 3 or (and) the dew condensation water staying inhibitor (coating 81) configured to inhibit the dew condensation water generated on the light transmission member 3 by the dew condensation from staying on the light transmission member 3. According to this, the light of the light emitting unit 2 is less likely to be scattered or refracted by the dew condensation water. Accordingly, the hair control device 10 of the present embodiment can make it easy for the light of the light emitting unit 2 reach the desired region (e.g., the region around the hair matrix 912) in the skin 92, thereby reducing the decline in the treatment efficiency for treating the skin.

The photocosmetic device 100 of the present embodiment includes, as the dew condensation inhibitor 8, a member configured to inhibit the dew condensation on the light emerging surface 39 of the light transmission member 3 (i.e., the second coating 812). When the light emerging surface 39 touches the skin 92 of the user while the dew condensation water (water droplet) is present on the light emerging surface 39, the user may feel uncomfortable due to the dew condensation water touching the skin 92. In this regard, the photocosmetic device 100 of the present embodiment including the second coating 812 can inhibits the situation where the dew condensation water touches the skin 92 of the user. The photocosmetic device 100 of the present embodiment thus can reduce the uncomfortable feeling to be caused in the user.

### (4.3) Variations of Embodiment 4

Variations of the embodiment 4 will be described. In the following description, the embodiment 4 described above may be referred to as a "basic example (of the embodiment 4)". In the description of the variations, components that are substantially the same as those of a basic aspect may be appropriately omitted. The basic example and the variations may be readily modified depending on a design choice or any other factor, without departing from a true spirit and scope of the present disclosure.

### (4.3.1) First Variation

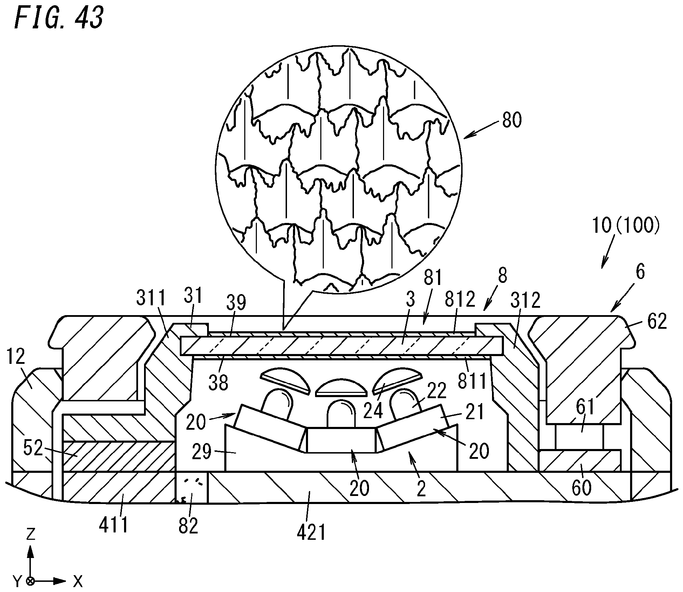

A hair control device 10 (photocosmetic device 100) of the present variation will be described with reference to FIG. 43. The hair control device 10 of the present variation differs from the hair control device 10 of the basic example in a configuration of a dew condensation inhibitor 8.

As shown in FIG. 43, the hair control device 10 of the present variation includes a water-repellent structure 80 serving as the dew condensation inhibitor 8. The water-repellent structure 80 is one example of a dew condensation water staying inhibitor.

Specifically, according to the hair control device 10 of the present variation, a coating 81 provided on a surface of a light transmission member 3 has the water-repellent structure 80. The material of the coating 81 is silicon, for example. As shown in FIG. 43, the water-repellent structure 80 of the present variation has a fine (µm-sized) honeycomb structure where pillars (protrusions) are formed on vertexes of regular hexagonal tubed walls. The shape and the size of the water-repellent structure 80 are appropriately set such that the coating 81 exhibits the hydrophobicity (water-repellent property). The contact angle of the coating 81 having the water-repellent structure 80 with respect to the water may be equal to or greater than 90°, or may be equal to or greater than 110°, or may be equal to or greater than 150°, for example.

According to the hair control device 10 of the present variation in which the coating 81 includes the water-repellent structure 80, the water generated on the surface of the coating 81 by the dew condensation is likely to have a spherical shape (to form a water droplet). By tilting the light transmission member 3 with respect to the horizon direction, the water droplet (dew condensation water) on the surface of the coating 81 is moved and thus can be removed easily, for example.

The water-repellent structure 80 is not limited to the honeycomb structure shown in FIG. 43, but may be a structure having many fine protrusions such as that on the surface of the lotus leaf, or a structure having many tetrapod structures such as that on the tetrapod type zinc oxide crystal, or other structures exhibiting the water-repellent property, for example.

The water-repellent structure 80 may be provided to a first coating 811, or to a second coating 812, or to both of them. The water-repellent structure 80 may not be formed in the coating 81, but may be directly formed in a surface of a sapphire glass constituting the light transmission member 3, for example.

### (4.3.2) Second Variation

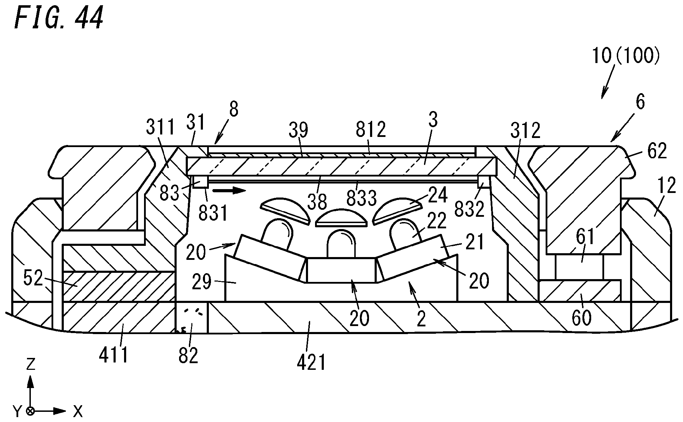

A hair control device 10 (photocosmetic device 100) of the present variation will be described with reference to FIG. 44. The hair control device 10 of the present variation differs from the hair control device 10 of the basic example in a configuration of a dew condensation inhibitor 8.

As shown in FIG. 44, the hair control device 10 of the present variation includes a wiping mechanism serving as the dew condensation inhibitor 8. The wiping mechanism 83 is one example of a dew condensation water staying inhibitor.

As shown in FIG. 44, the wiping mechanism 83 includes a wiping member 831, a driving member 832, and a coupling member 833.

The wiping member 831 is disposed to be slidably movable over a light incident surface 38 of a light transmission member 3. The wiping member 831 includes so-called a squeegee. The wiping member 831 has a box shape extending in the Y-axis, in the variation. The wiping member 831 has a contact portion configured to be brought into contact with the light incident surface 38 of the light transmission member 3. The contact portion has a blade shape extending in the Y-axis, and is made of silicon-based resin for example. On the rear sides (negative side in the Z-axis) of both ends in the Y-axis of the wiping member 831, a pair of supporters are provided to respectively support both ends of the wiping member 831 from the rear side (negative side in the Z-axis). The pair of supporters extend along the X-axis over a whole length between a first holding arm 311 and a second holding arm 312. The driving member 832 is configured to generate a motive power to move the wiping member 831. The driving member 832 includes a motor, a reel, and a spring member, for example. The coupling member 833 is like a string. The coupling member 833 couples between the wiping member 831 and the driving member 832. When the motor of the driving member 832 is driven (to rotate in a normal direction), the coupling member 833 is wound up by the reel and thus the wiping member 831 slidably moves rightward (toward the positive side in the X-axis) along the light incident surface 38 of the light transmission member 3. The dew condensation water on the light incident surface 38 of the light transmission member 3 is wiped by the wiping member 831 and thus removed from the light incident surface 38. Moreover, by rotating the motor in a reverse direction, the coupling member 833 is reeled out from the reel. By the elastic force of the spring member, the wiping member 831 slidably moves leftward (toward the negative side in the X-axis) along the light incident surface 38 of the light transmission member 3 to the original position.

The wiping member 831 further includes a dehumidifying agent. The dehumidifying agent is disposed around a portion, which is to be brought into contact with the light incident surface 38, of the wiping member 831. The dehumidifying agent includes at least one material selected from the group consisting of calcium chloride, silica gel, quicklime, and zeolite, for example. The wiping member 831 including the dehumidifying agent can absorb the moisture on the light incident surface 38 into the dehumidifying agent to remove the moisture. The removing efficiency of the dew condensation water is improved.

The driving member 832 and the coupling member 833 are not limited to the structures described above. Alternatively, the driving member 832 may be disposed on the left of the wiping member 831 and have a structure to push the wiping member 831 rightward by a pin ejected by the motive power of the motor. Alternatively, the coupling member 833 may have a lazy tongs structure where arm portions thereof are stretchable.

The wiping member 831 is not limited to be driven by the driving member 832, but may be manually moved by a hand.

A coating 81 (first coating 811) may be provided to the light incident surface 38 of the coating 81, although it is not provided in the present variation.

A wiping member 831 may be disposed to be slidably movable over a light emerging surface 39 of a light transmission member 3. Respective wiping members 831 may be provided to a light incident surface 38 and a light emerging surface 39 of a light transmission member 3.

### (4.3.3) Third Variation

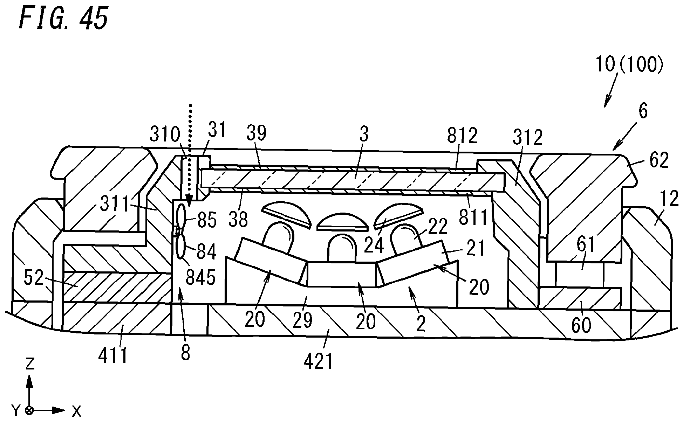

A hair control device 10 (photocosmetic device 100) of the present variation will be described with reference to FIG. 45. The hair control device 10 of the present variation differs from the hair control device 10 of the basic example in a configuration of a dew condensation inhibitor 8.

As shown in FIG. 45, the hair control device 10 of the present variation includes a blowing member 84 serving as the dew condensation inhibitor 8. The blowing member 84 is one example of a dew condensation preventer.

According to the hair control device 10 of the present variation, the blowing member 84 includes a fan 845. The fan 845 is disposed in a housing space S10. The fan 845 is disposed to form an air flow flowing in the housing space S10. Specifically, the fan 845 is disposed such that the wind is sent out toward the light source 22 of the light emitting unit 2 to form an air flow which takes away the heat generated in the light emitting unit 2 to be apart from the light transmission member 3.

According to the hair control device 10 of the present variation, the gas (air) warmed up by the heat of the light emitting unit 2 is taken away to be apart from the light transmission member 3 by the wind of the fan 845, not approaching the light transmission member 3. The temperature of the air around the light transmission member 3 can be maintained regardless of the heat generated in the light emitting unit 2. The temperature difference between the temperature of the light transmission member 3 and the temperature of the air around the light transmission member 3 is kept relatively small, which can prevent the dew condensation from occurring on a surface (a light incident surface 38 and a light emerging surface 39) of the light transmission member 3.

Moreover, the hair control device 10 of the present variation includes a blowing unit 85 serving as the dew condensation inhibitor 8, as shown in FIG. 45. The blowing unit 85 is one example of the dew condensation water staying inhibitor.

According to the hair control device 10 of the present variation, the blowing unit 85 includes the fan 845. The fan 845 is disposed such that the wind sent out by the fan 845 reaches the light incident surface 38 of the light transmission member 3. That is, the fan 845 is disposed to form an air flow flowing over the light incident surface 38 of the light transmission member 3. When the wind sent out by the fan 845 reaches the light incident surface 38, the dew condensation water generated on the light incident surface 38 by the dew condensation is moved along the light incident surface 38 by the wind. This can facilitate to remove the dew condensation water from the light incident surface 38 of the light transmission member 3.

Additionally or alternatively, the blowing unit 85 may include a fan disposed on the outside of the housing 1 and configured to form an air flow flowing over a light emerging surface 39 of the light transmission member 3, for example.

As shown in FIG. 45, according to the hair control device 10 of the present variation, a through hole 310 is formed in a first holding arm 311 of a holder 31. The through hole 310 allows the air of the outside of the housing 1 to be taken in the housing space S10 (see a dotted arrow shown in FIG. 45)

Moreover, a dehumidifying material 82 may be disposed in the through hole 310. Alternatively, no through hole 310 may be provided.

### (4.3.4) Fourth Variation

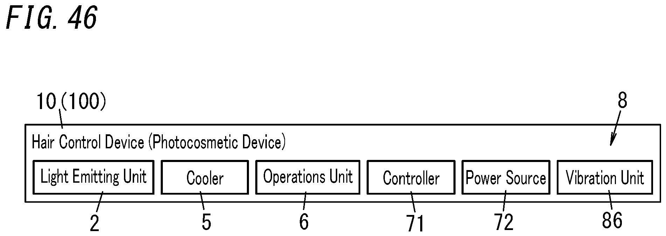

A hair control device 10 (photocosmetic device 100) of the present variation will be described with reference to FIG. 46. The hair control device 10 of the present variation differs from the hair control device 10 of the basic example in a configuration of a dew condensation inhibitor 8.

As shown in FIG. 46, the hair control device 10 of the present variation includes a vibration unit 86 serving as a dew condensation inhibitor 8. The vibration unit 86 is one example of a dew condensation water staying inhibitor.

The vibration unit 86 is configured to vibrate a light transmission member 3. The vibration unit 86 is not limited to its structure. The vibration unit 86 may be configured to vibrate a housing 1 (a first case 11 or a second case 12) to vibrate the light transmission member 3, for example. Additionally or alternatively, a first holding arm 311 (or a second holding arm 312) includes a holding part for holding the light transmission member 3 and a base part coupled to a first coupler 411 (or a second coupler 421), and the holding part is movably held by the base part. The vibration unit 86 may be configured to vibrate the holding part with respect to the base part.

By the vibration unit 86 vibrating the light transmission member 3, the water generated on a light incident surface 38 (or a light emerging surface 39) of the light transmission member 3 by the dew condensation can be easily moved to an end of the light incident surface 38 (or the light emerging surface 39). This can facilitate to remove the dew condensation water from the light incident surface 38 (or the light emerging surface 39).

### (4.3.5) Fifth Variation

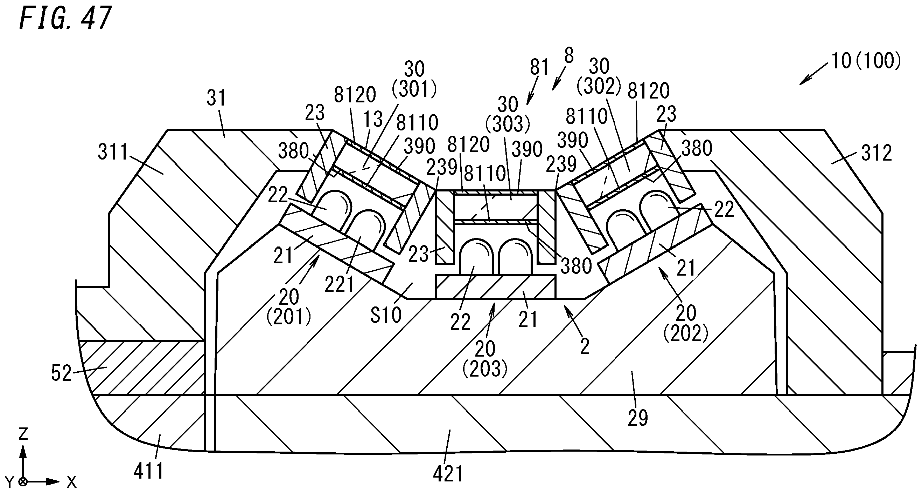

A hair control device 10 (photocosmetic device 100) of the present variation will be described with reference to FIG. 47. The hair control device 10 of the present variation differs from the hair control device 10 of the basic example mainly in a configuration of a light emitting unit 2 and a light transmission member 3.

As shown in FIG. 47, according to the hair control device 10 of the present variation, the light transmission member 3 includes a plurality of light transmission pieces 30 (a first light transmission piece 301 to a third light transmission piece 303). Each of the plurality of light transmission pieces 30 has a plate shape. The plurality of light transmission pieces 30 are arranged to correspond (one-to-one) to a plurality of light source units 20 (a first light source unit 201 to a third light source unit 203). Each of the plurality of light transmission pieces 30 is disposed to face a light source 22 of a corresponding light source unit 20. Each light transmission piece 30 is held by (fixed to) a light blocking member 23 of a corresponding light source unit 20 to occlude a front opening of the light blocking member 23. Each light transmission piece 30 has a light incident surface 380 through which light of a corresponding light source unit 20 enters and a light emerging surface 390 from which the light entered through the light incident surface 380 is emerged to an outside of the housing 1.

According to the hair control device 10 of the present variation, a light incident surface 38 of the light transmission member 3 is constituted by the light incident surfaces 380 of the plurality of light transmission pieces 30. A light emerging surface 39 of the light transmission member 3 (i.e., a light exit surface 13) is considered by the light emerging surfaces 390 of the plurality of light transmission pieces 30. The light emerging surface 39 of the light transmission member 3 (i.e., the light exit surface 13) has a recessed shape recessed inward the housing 1.

As shown in FIG. 47, according to the hair control device 10 of the present variation, each of the plurality of light source units 20 of the light emitting unit 2 includes, as the light source 22, a plurality of (16-pieces of) LEDs 221 disposed on a substrate 21 in a 2x8 array. Each light source unit 20 does not include an optical member 24, but includes the light blocking member 23.

The light blocking member 23 is a wall having a rectangular frame shape and extending along the Y-axis. The light blocking member 23 surrounds the light source 22 from the four sides. The light blocking member 23 has an opening in the front side. The light blocking member 23 is configured to prevent the light of the light source 22 from traveling (leaking) toward a light source 22 of an adjacent light source unit 20. The light blocking member 23 has an inside surface on which a mirror is formed to reflect forward the light of the light source 22.

According to the plurality of (three) light source units 20, front ends of the light blocking members 23 of adjacent two light source units 20 are connected to each other, thereby the three light blocking members 23 are formed integrally. A passage (groove 239) is formed between the front edges of the light blocking members 23 of the adjacent two light source units 20 and extends along the Y-axis.

The hair control device 10 of the present variation includes a hydrophobic coating 81 serving as a dew condensation inhibitor 8. The coating 81 includes a first coating 811 provided to the light incident surface 38 of the light transmission member 3 and a second coating 812 provided to the light emerging surface 39 of the light transmission member 3. The first coating 811 includes a plurality of first coating pieces 8110 provided to the light incident surfaces 380 of the plurality of light transmission pieces 30, respectively. The second coating 812 includes a plurality of second coating pieces 8120 provided to the light emerging surfaces 390 of the plurality of light transmission pieces 30, respectively.

The hair control device 10 of the present variation has the advantage of reducing the uncomfortable feeling to be caused in the user as with the embodiment 1, in addition to the advantage of reducing the decline in the treatment efficiency for treating the skin as with the hair control device 10 of the embodiment 4. According to the hair control device 10 of the present variation, by means of the dew condensation inhibitor 8 (the coating 81), it is possible to inhibit the staying of the dew condensation water on the light transmission member 3.

Moreover, according to the hair control device 10 of the present variation, the dew condensation water (water droplet) generated by the dew condensation on e.g., the light emerging surface 390 of the light transmission piece 30 located on the left will move along the inclined light emerging surface 390 to reach the groove 239 formed between the light blocking member 23 and then is discharged. Accordingly, the dew condensation water (water droplet) can be easily discharged.

### (4.3.6) Sixth Variation

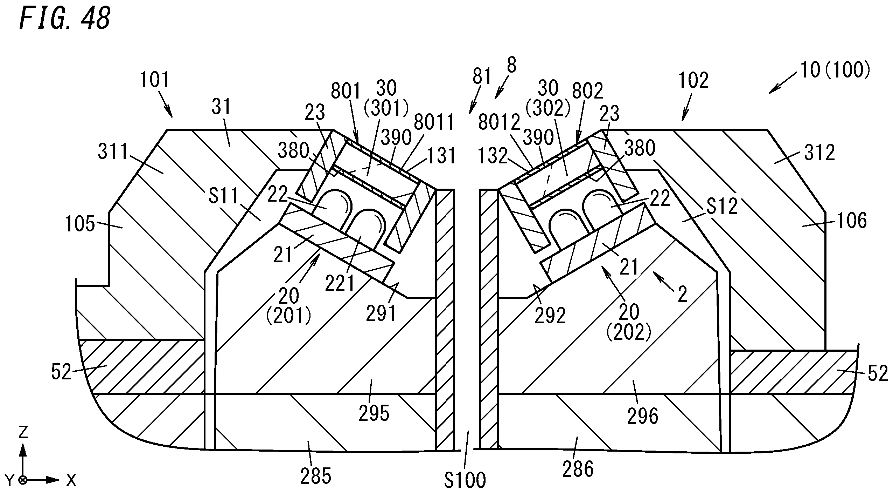

A hair control device 10 (photocosmetic device 100) of the present variation will be described with reference to FIG. 48. The hair control device 10 of the present variation differs from the hair control device 10 of the fifth variation in a configuration of a housing 1 (second case 12), a light emitting unit 2, a light transmission member 3, a dew condensation inhibitor 8 etc.

As shown in FIG. 48, according to the hair control device 10 of the present variation, the housing 1 (specifically, the second case 12) includes a first housing part 105 in which a first light source unit 201 is housed and a second housing part 106 in which a second light source unit 202 is housed. The dew condensation inhibitor 8 includes a first dew condensation inhibitor 801 and a second dew condensation inhibitor 802. The first dew condensation inhibitor 801 is configured to prevent dew condensation from occurring on a first light transmission piece 301 and/or to inhibit a dew condensation water on the light transmission member generated by dew condensation from staying on the light transmission member. The second dew condensation inhibitor 802 is configured to prevent dew condensation from occurring on a second light transmission piece 302 and/or to inhibit a dew condensation water on the light transmission member generated by dew condensation from staying on the light transmission member. The first housing part 105 and the second housing part 106 are disposed separately (separated) from each other in a direction in which the first light source unit 201 and the second light source unit 202 are arranged.

Specifically, the hair control device 10 of the present variation includes a first light source block 101 and a second light source block 102.

The first light source block 101 includes a first supporting plate 285, a first holding member 295, the first light source unit 201, the first light transmission piece 301, the first dew condensation inhibitor 801, and the first housing part 105. The first supporting plate 285 has a plate shape and extends along the Y-axis. The first holding member 295 has an inclined base shape extending along the Y-axis, and is supported on the first supporting plate 285. The first holding member 295 has a first inclined surface 291 inclined toward the second light source block 102. A substrate 21 of the first light source unit 201 is disposed on the first inclined surface 291. The first housing part 105 has a housing space S11 in which the first light source unit 201 is housed. The first housing part 105 has a peripheral wall shape and surrounds the first light source unit 201 and the first holding member 295. The first housing part 105 holds the first light transmission piece 301 such that the first light transmission piece 301 is positioned on an optical axis of the first light source unit 201. A front surface of the first light transmission piece 301 serves as a first inclined surface 131 of a light exit surface 13. The first dew condensation inhibitor 801 includes a dew condensation staying inhibitor (a coating 8011 provided to a surface of the first light transmission piece 301, for example).

The second light source block 102 includes a second supporting plate 286, a second holding member 296, the second light source unit 202, the second light transmission piece 302, the second dew condensation inhibitor 802, and the second housing part 106. The second supporting plate 286 has a plate shape and extends along the Y-axis. The second holding member 296 has an inclined base shape extending along the Y-axis, and is supported on the second supporting plate 286. The second holding member 296 has a second inclined surface 292 inclined toward the first light source block 101. A substrate 21 of the second light source unit 202 is disposed on the second inclined surface 292. The second housing part 106 has a housing space S12 in which the second light source unit 202 is housed. The second housing part 106 has a peripheral wall shape and surrounds the second light source unit 202 and the second holding member 296. The second housing part 106 holds the second light transmission piece 302 such that the second light transmission piece 302 is positioned on an optical axis of the second light source unit 202. A front surface of the second light transmission piece 302 serves as the second inclined surface 132 of the light exit surface 13. The second dew condensation inhibitor 802 includes a dew condensation staying inhibitor (a coating 8012 provided to s surface of the second light transmission piece 302, for example).

As shown in FIG. 48, in the hair control device 10 of the present variation, there is a space S100 between the first housing part 105 and the second housing part 106. According to the hair control device 10 of the present variation, therefore, the space S100 is formed between the first light source block 101 and the second light source block 102.

According to the hair control device 10 of the present variation, the dew condensation inhibitor 8 is (specifically, the first dew condensation inhibitor 801 and the second dew condensation inhibitor 802 are) individually provided to the first light transmission piece 301 and the second light transmission piece 302. Therefore, even when the dew condensation on the first light transmission piece 301 and the dew condensation on the second light transmission piece 302 are not balanced due to e.g., the difference of the degree of the contact with respect to the skin 92, it is possible to control the dew condensation water individually.

Note that the first housing part 105 and the second housing part 106 may be in contact with each other (i.e., a distance therebetween may be 0).

### (4.3.7) Other Variations

In one variation, a dew condensation inhibitor 8 may include either one of a dew condensation preventer or a dew condensation staying inhibitor. For example, a photocosmetic device 100 may includes, as the dew condensation inhibitor 8, a coating 81 only, or a dehumidifying material 82 only.

In one variation, a housing 1 does not have to house therein an entire of a light emitting unit 2. A housing 1 may house the light emitting unit 2 with a part of a light source unit 20 (e.g., a part of a substrate 21) protruding from the housing 1, for example.

In one variation, an operations unit 6 may include a switch provided on a side surface of a first case 11, for example.

In one variation, a light emitting unit 2 may include one, two, four or more light source units 20. A light source 22 of a light source unit 20 may include only one LED 221 or two or more LEDs 221.

In one variation, a light source 22 of a light source unit 20 is not limited to an LED 221 but may include a xenon lamp or a semiconductor laser.

### (5) Other Configurations

The photocosmetic device 100 according to the present disclosure includes, in its controller 71 for example, a computer system. In that case, the computer system may include, as principal hardware components, a processor and a memory. The functions of the controller 71 may be performed by making the processor execute a program stored in the memory of the computer system. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be made up of a single or a plurality of electronic circuits including a semiconductor integrated circuit (IC) or a large-scale integrated circuit (LSI). As used herein, the "integrated circuit" such as an IC or an LSI is called by a different name depending on the degree of integration thereof. Examples of the integrated circuits include a system LSI, a very large-scale integrated circuit (VLSI), and an ultra large-scale integrated circuit (ULSI). Optionally, a field-programmable gate array (FPGA) to be programmed after an LSI has been fabricated or a reconfigurable logic device allowing the connections or circuit sections inside of an LSI to be reconfigured may also be adopted as the processor. Those electronic circuits may be either integrated together on a single chip or distributed on multiple chips, whichever is appropriate. Those multiple chips may be integrated together in a single device or distributed in multiple devices without limitation. As used herein, the "computer system" includes a microcontroller including one or more processors and one or more memories. Thus, the microcontroller may also be implemented as a single or a plurality of electronic circuits including a semiconductor integrated circuit or a largescale integrated circuit.

Also, the photocosmetic device 100 is not limited to the configuration where the plurality of constituent elements (or the functions) of the controller 71 are integrated together in a single housing. Alternatively, those constituent elements (or functions) of the controller 71 may be distributed in multiple different housings. Still alternatively, at least some functions of the controller 71 may be implemented as a cloud computing system as well.

The photocosmetic device 100 (body hair treatment device) may be a hair growth promoting device suitable for the hair growth promotion.

The photocosmetic device 100 may be a device suitable for improving the firmness or the wrinkles of the skin 92. For example, the effect for improving the firmness or the wrinkles can be achieved by irradiating the skin 92 with the light having the wavelength falling within the range of 600 nm to 800 nm to focus the light on the fibroblast present in the dermic layer (0.1 mm to 5 mm depth from the surface) of the skin 92 to promote the collagen production. A light source 22 of a light emitting unit 2 may be used as a light source for the improvement of the firmness or the wrinkles of the skin. Alternatively, the photocosmetic device 100 may include another light source for the improvement of the firmness or the wrinkles of the skin.

The photocosmetic device 100 may be a device suitable for improving (reducing) the blemishes/spots on the skin 92. For example, the effect for improving the blemishes/spots can be achieved by irradiating the skin 92 with the light having the wavelength falling within the range of 600 nm to 1000 nm to focus the light on the melanocytes present in the lower part of the epidermal layer (about 0.1 mm depth from the surface) of the skin 92 to prevent the melanin-production by the heat load on the melanocytes. A light source 22 of a light emitting unit 2 may be used as a light source for the improvement of the blemishes/spots. Alternatively, the photocosmetic device 100 may include another light source for the improvement of the blemishes/spots.

The photocosmetic device 100 may be a device suitable for slimming body. For example, the effect for slimming the body can be achieved by irradiating the skin 92 with the light having the wavelength falling within the range of 800 nm to 1200 nm to focus the light on the body fat in fatty layer (about 5 mm or more depth from the surface) of the skin 92 to heat the body fat to promote the absorption. A light source 22 of a light emitting unit 2 may be used as a light source for slimming the body. Alternatively, the photocosmetic device 100 may include another light source for the slimming the body.

The photocosmetic device 100 may be used only for the cosmetic purpose other than the body hair treatment (e.g., the improvement of the firmness or the wrinkles of the skin, the improvement of the blemishes/spots, or the slimming body). That is, the photocosmetic device 100 of the present disclosure is not limited to the body hair treatment device.

### (6) Aspect

As apparent from the embodiments and the variations described above, the present disclosure discloses the aspect below.

A photocosmetic device (100) of a first aspect includes a plurality of light source units (20), a housing (1), and a light exit surface (13). Each of the plurality of light source units (20) includes a light source (22). The plurality of light source units (20) are housed in the housing (1). Light of the light source (22) of each of the plurality of light source units (20) is emitted from the light exit surface (13) to an outside of the housing (1). The light exit surface (13) has a recessed shape recessed inward the housing (1).

This aspect can reduce the uncomfortable feeling to be caused in a user.

In a photocosmetic device (100) of a second aspect realized in combination with the first aspect, each of the plurality of light source units (20) includes, as the light source (22), an LED array including a plurality of LEDs (221) arranged in an array.

This aspect can further reduce the uncomfortable feeling to be caused in a user.

A photocosmetic device (100) of a third aspect realized in combination with the first or second aspect further includes a light transmission member (3). The light transmission member (3) allows the light of the light source (22) of each of the plurality of light source units (20) to pass through. The light exit surface (13) is at least partially constituted by a surface of the light transmission member (3).

This aspect can uniquely set a distance between the light source (22) and an illumination target of the light of the light source (22).

In a photocosmetic device (100) of a fourth aspect realized in combination with the third aspect, the light transmission member (3) is made of the material having the thermal conductivity of 1 W/mK or more.

This aspect can further reduce the uncomfortable feeling to be caused in a user.

In a photocosmetic device (100) of a fifth aspect realized in combination with the third or fourth aspect, the light sources (22) of the plurality of light source units (20) have the same emission wavelength as one another. The light transmission member (3) is made of the material having the refractive index of 1.7 or more at the emission wavelength.

This aspect can further reduce the uncomfortable feeling to be caused in a user.

In a photocosmetic device (100) of a sixth aspect realized in combination with any one of the third to fifth aspects, the light transmission member (3) is made of at least one material selected from the group consisting of sapphire, zinc oxide, zirconium oxide, magnesium oxide, gallium nitride, aluminum nitride, and diamond.

This aspect can further reduce the uncomfortable feeling to be caused in a user.

In a photocosmetic device (100) of a seventh aspect realized in combination with any one of the third to sixth aspects, the light transmission member (3) includes a plurality of light transmission pieces (30) arranged to correspond to the plurality of light source units (20), respectively. Each of the plurality of light transmission pieces (30) is positioned on an optical axis (A10) of a corresponding one of the plurality of light source units (20).

This aspect can reduce the overlap of the rays of the light of the light sources (22) on the light exit surface (13). The uncomfortable feeling to be caused in a user can be further reduced accordingly.

A photocosmetic device (100) of an eighth aspect realized in combination with the seventh aspect further includes a partition (32) that separates the plurality of light transmission pieces (30) from one another in the light exit surface (13).

This aspect can further reduce the overlap of the rays of the light of the light sources (22) on the light exit surface (13). The uncomfortable feeling to be caused in a user can be further reduced accordingly.

A photocosmetic device (100) of a ninth aspect realized in combination with any one of the third to eighth aspects further includes a cooler (5) disposed to cool the light transmission member (3).

This aspect can further reduce the uncomfortable feeling to be caused in a user.

In a photocosmetic device (100) of a tenth aspect realized in combination with any one of the first to ninth aspects, the light sources (22) of the plurality of light source units (20) are apart from one another.

This aspect can reduce the overlap of the rays of the light of the light sources (22) on the light exit surface (13). The uncomfortable feeling to be caused in a user can be further reduced accordingly.

A photocosmetic device (100) of an eleventh aspect realized in combination with any one of the first to ninth aspects further includes a light blocking part (230). The light blocking part (230) is configured to reduce light traveling from a light source (22) of one of the plurality of light source units (20) toward a light source (22) of at least one of remainder of the plurality of light source units (20).

This aspect can improve the energy efficiency.

A photocosmetic device (100) of a twelfth aspect realized in combination with the eleventh aspect further includes a light transmission member (3). The light transmission member (3) constitutes at least part of the light exit surface (13). The light transmission member (3) allows the light of the light source (22) of each of the plurality of light source units (20) to pass through. The light blocking part (230) is in contact with the light transmission member (3).

This aspect allows the heat of the light transmission member (3) to be transmitted to the light blocking part (230).

In a photocosmetic device (100) of a thirteenth aspect realized in combination with any one of the first to twelfth aspects, a depth (X1) of a recessed space surrounded by the light exit surface (13) falls within the range of greater than 0 mm and equal to or smaller than 30 mm.

This aspect allows a plurality of rays of light to be emitted from the light exit surface (13) that are non-parallel to one another and thus allows the plurality of non-parallel rays of light to cross one another around the hair matrix (912), which can increase the amount of light focused onto the region around the hair matrix (912).

In a photocosmetic device (100) of a fourteenth aspect realized in combination with any one of the first to thirteenth aspects, the plurality of light source units (20) have respective optical axes (A10) each of which intersects with the light exit surface (13) at right angles.

This aspect can increase the light extraction efficiency of the light from the light exit surface (13).

In a photocosmetic device (100) of a fifteenth aspect realized in combination with any one of the first to fourteenth aspects, the plurality of light source units (20) includes a first light source unit (201) and a second light source unit (202). The housing (1) includes a first housing part (105) in which the first light source unit (201) is housed and a second housing part (106) in which the second light source unit (202) is housed. The first housing part (105) and the second housing part (106) are disposed separately from each other in a direction in which the first light source unit (201) and the second light source unit (202) are arranged.

This aspect can reduce light (leakage light) traveling from a light source (22) of one of the first light source unit (201) and the second light source unit (202) toward a light source (22) of the other.

In a photocosmetic device (100) of a sixteenth aspect realized in combination with the fifteenth aspect, at least one of the first housing part (105) or the second housing part (106) is movable in the direction in which the first light source unit (201) and the second light source unit (202) are arranged.

According to this aspect, the light exit surface (13) can be in contact with the skin (92) more closely.

A photocosmetic device (100) of a seventeenth aspect realized in combination with the sixteenth aspect further includes a stopper mechanism configured to restrict the movement of at least one of the first housing part (105) or the second housing part (106) such that a gap remains between the first housing part (105) and the second housing part (106) even in a state where the first housing part (105) and the second housing part (106) are approached closest each other.

This aspect can prevent the skin (92) from being tightly caught between the first housing part (105) and the second housing part (106). The uncomfortable feeling to be caused in a user can be reduced.

In a photocosmetic device (100) of an eighteenth aspect realized in combination with any one of the first to seventeenth aspects, each of the plurality of light source units (20) further includes a light guide member (25) configured to guide the light of the light source (22) toward the light exit surface (13).

A photocosmetic device (100) of an eighteenth aspect includes a plurality of light source units (20), a housing (1), and a light exit surface (13). Each of the plurality of light source units (20) includes a light source (22). The plurality of light source units (20) are housed in the housing (1). Light of the light source (22) of each of the plurality of light source units (20) is emitted from the light exit surface (13) to an outside of the housing (1). Each of the plurality of light source units (20) further includes a light guide member (25) configured to guide the light of the light source (22) toward the light exit surface (13).

This aspect allows the operator who operates the photocosmetic device (100) to easily locate the light exit surface (13) on a desired region of the skin (92), which can improve the handleability of the device.

In a photocosmetic device (100) of a nineteenth aspect realized in combination with the eighteenth aspect, each of the plurality of light source units (20) includes a plurality of LEDs (221) serving as the light source (22).

This aspect can improve the handleability of the device.

In a photocosmetic device (100) of a twentieth aspect realized in combination with the nineteenth aspect, in each of the plurality of light source units (20), the plurality of LEDs (221) are arranged in a line along one axis. In each of the plurality of light source units (20), the light guide member (25) has cross sectional shapes in any imaginary planes (Im10) perpendicular to the one axis, the cross sectional shapes being the same as each other.

This aspect can improve the handleability of the device.

In a photocosmetic device (100) of a twenty-first aspect realized in combination with any one of the eighteenth to twentieth aspects, in each of the plurality of light source units (20), the light guide member (25) has a recess (250) in a surface thereof that faces the light source (22). The light source (22) is disposed inside the recess (250).

According to this aspect, the light of the light source (22) can easily be entered the light guide member (25).

In a photocosmetic device (100) of a twenty-second aspect realized in combination with any one of the eighteenth to twenty-first aspects, in each of the plurality of light source units (20), the light guide member (25) has a light incident surface (251) through which the light of the light source (22) enters and a light emerging surface (252) from which the light entered through the light incident surface (251) is emerged to the light exit surface (13). In each of the plurality of light source units (20), the light incident surface (251) has a lens surface.

This aspect can align the traveling direction inside the light guide member (25) of the ray of light of the light source (22), which has entered the light guide member (25) through the light incident surface (251).

In a photocosmetic device (100) of a twenty-third aspect realized in combination with any one of the eighteenth to twenty-second aspects, in each of the plurality of light source units (20), the light guide member (25) has a light incident surface (251) through which the light of the light source (22) enters and a light emerging surface (252) from which the light entered through the light incident surface (251) is emerged to the light exit surface (13). In each of the plurality of light source units (20), the light emerging surface (252) has a lens surface.

This aspect can adjust the shape of the light of the light source unit (20) by the light emerging surface (252).

In a photocosmetic device (100) of a twenty-fourth aspect realized in combination with the twenty-third aspect, the light emerging surfaces (252) of the respective light guide members (25) of the plurality of light source units (20) are disposed such that rays of the light of the plurality of light source units (20) cross one another on an outside of the housing (1).

This aspect can facilitate the rays of the light of the plurality of light source units (20) to cross one another inside the skin (92) around the hair matrix. This allows the light to be efficiently absorbed in the skin (92) in the region around the hair matrix to efficiently damage the hair matrix.

In a photocosmetic device (100) of a twenty-fifth aspect realized in combination with any one of the eighteenth to twenty-fourth aspects, each of the plurality of light source units (20) further includes a light blocking member (23) configured to reduce light traveling from the light source (22) toward at least one of remainder of the plurality of light source units (20).

This aspect can reduce the light traveling from the light source (22) toward at least one of remainder of the plurality of light source units (20).

In a photocosmetic device (100) of a twenty-sixth aspect realized in combination with the twenty-fifth aspect, in each of the plurality of light source units (20), the light blocking member (23) is disposed to surround side surfaces of the light guide member (25).

This aspect can further reduce the light traveling from the light source (22) toward at least one of remainder of the plurality of light source units (20).

In a photocosmetic device (100) of a twenty-seventh aspect realized in combination with any one of the eighteenth to twenty-sixth aspects, in each of the plurality of light source units (20), the light guide member (25) has a light incident surface (251) through which the light of the light source (22) enters and a light emerging surface (252) from which the light entered through the light incident surface (251) is emerged to the light exit surface (13). In at least one of the plurality of light source units (20), the light guide member (25) has a cross sectional area that decreases from the light incident surface (251) to the light emerging surface (252).

This aspect can reduce the size of the front end of the hair control device where the light exit surface (13) is provide, which allows the operator to easily position the light exit surface (13) on a desired region of the surface of the skin (92).

A photocosmetic device (100) of a twenty-eighth aspect realized in combination with any one of the eighteenth to twenty-seventh aspects further includes a light transmission member (3) that allows the light of the plurality of light source units (20) to pass through. The light exit surface (13) is at least partially constituted by a surface of the light transmission member (3).

This aspect can uniquely set a distance between the light source unit (20) and an illumination target of the light of the light source unit (20).

In a photocosmetic device (100) of a twenty-ninth aspect realized in combination with the twenty-eighth aspect, the light transmission member (3) is made of the material having the thermal conductivity of 1 W/mK or more.

This aspect can reduce the uncomfortable feeling to be caused in a user.

A photocosmetic device (100) of a thirtieth aspect realized in combination with any one of the eighteenth to twenty-ninth aspects further includes a heat dissipation part (4) configured to dissipate heat generated in the light source (22) of each of the plurality of light source units (20).

This aspect can facilitate the cooling of the plurality of light source units (20).

In a photocosmetic device (100) of a thirty-first aspect realized in combination with the thirtieth aspect, the housing (1) includes a first case (11) in which the heat dissipation part (4) is housed and a second case (12) which protrudes from the first case (11) along a first axis and in which respective light guide members (25) of the plurality of light source units (20) are housed. The plurality of light source units (20) are arranged along a second axis perpendicular to the first axis. A maximum dimension along the second axis of the first case (11) is greater than a maximum dimension along the second axis of the second case (12).

This aspect can reduce the size of the front end of the housing (1) where the light exit surface (13) is provide, which allows the operator to easily position the light exit surface (13) on a desired region of the surface of the skin (92).

In a photocosmetic device (100) of a thirty-second aspect realized in combination with the thirty-first aspect, an area of a first projection region is greater than an area of a second projection region. The first projection region is a region enclosed by a first projection line obtained by projecting a circumference of the first case (11) on an imaginary plane (Im1) perpendicular to the first axis. The second projection region is a region enclosed by a second projection line obtained by projecting a circumference of the second case (12) on the imaginary plane (Im1).

This aspect can reduce the size of the front end of the housing (1) where the light exit surface (13) is provide, which allows the operator to easily position the light exit surface (13) on a desired region of the surface of the skin (92).

In a photocosmetic device (100) of a thirty-third aspect realized in combination with the thirty-first or thirty-second aspect, the second case (12) has a far end away from the first case (11) in the first axis. The light exit surface (13) is located at the far end of the second case (12).

This aspect can reduce the size of the front end of the housing (1) where the light exit surface (13) is provide, which allows the operator to easily position the light exit surface (13) on a desired region of the surface of the skin (92).

A photocosmetic device (100) of a thirty-fourth aspect realized in combination with any one of the first to thirty-third aspects includes a light emitting unit (2), a heat dissipator (40), a light transmission member (3), a heat conductor (50), and a cooler (5). The light emitting unit (2) includes the plurality of light source units (20). The heat dissipator (40) is thermally connected to the light emitting unit (2). The heat dissipator (40) is configured to dissipate heat generated in the light emitting unit (2). The light transmission member (3) allows light of the light emitting unit (2) to pass through. The heat conductor (50) is thermally connected to the light transmission member (3). The heat conductor (50) is configured to receive heat from the light transmission member (3). The cooler (5) is disposed to cool the heat conductor (50). A predetermined surface (500) is at least part of a surface of the heat conductor (50). The predetermined surface (500) faces the heat dissipator (40) with a gap between the predetermined surface (500) and the heat dissipator (40).

A photocosmetic device (100) of a thirty-fourth aspect includes a light emitting unit (2), a heat dissipator (40), a light transmission member (3), a heat conductor (50), and a cooler (5). The light emitting unit (2) is configured to emit light to be illuminated on a skin (92). The heat dissipator (40) is thermally connected to the light emitting unit (2). The heat dissipator (40) is configured to dissipate heat generated in the light emitting unit (2). The light transmission member (3) allows the light of the light emitting unit (2) to pass through. The heat conductor (50) is thermally connected to the light transmission member (3). The heat conductor (50) is configured to receive heat from the light transmission member (3). The cooler (5) is disposed to cool the heat conductor (50). A predetermined surface (500) is at least part of a surface of the heat conductor (50). The predetermined surface (500) faces the heat dissipator (40) with a gap between the predetermined surface (500) and the heat dissipator (40).

This aspect can balance the cooling of the skin (92) and the cooling of the light source. Moreover, a water droplet (W1) of dew condensation water on the predetermined surface (500) generated by the cooling of the heat conductor (50) is brought into contact with the heat dissipator (40) and then is vaporized, and takes the heat (heat of vaporization) away from the heat dissipator (40). This can facilitate the cooling of a light emitting unit (2) thermally connected to the heat dissipator (40).

In a photocosmetic device (100) of a thirty-fifth aspect realized in combination with the thirty-fourth aspect, a distance (X5) between the predetermined surface (500) of the heat conductor (50) and the heat dissipator (40) in a gap (G1) is set to allow a dew condensation water on the predetermined surface (500) generated by dew condensation to be in contact with the heat dissipator (40).

This aspect can facilitate the cooling of the light emitting unit (2).

In a photocosmetic device (100) of a thirty-sixth aspect realized in combination with the thirty-fourth or thirty-fifth aspect, the distance between the predetermined surface (500) of the heat conductor (50) and the heat dissipator (40) in the gap (G1) falls within the range equal or greater than 50 µm and equal to or smaller than 20 mm.

This aspect can facilitate the cooling of the light emitting unit (2).

In a photocosmetic device (100) of a thirty-seventh aspect realized in combination with any one of the thirty-fourth to thirty-sixth aspects, the cooler (5) includes a Peltier device (52).

This aspect can facilitate the cooling of the light emitting unit (2).

In a photocosmetic device (100) of a thirty-eighth aspect realized in combination with any one of the thirty-fourth to thirty-seventh aspects, the predetermined surface (500) of the heat conductor (50) and the heat dissipator (40) face each other with an air space between the predetermined surface (500) and the heat dissipator (40).

This aspect can facilitate the cooling of the light emitting unit (2).

A photocosmetic device (100) of a thirty-ninth aspect realized in combination with any one of the thirty-fourth to thirty-seventh aspects further includes a porous member (P10) disposed in the gap (G1) between the predetermined surface (500) of the heat conductor (50) and the heat dissipator (40).

This aspect can facilitate the cooling of the light emitting unit (2).

In a photocosmetic device (100) of a fortieth aspect realized in combination with the thirty-ninth aspect, the porous member (P10) includes at least one material selected from the group consisting of mesoporous silica, silica gel, activated carbon, and zeolite.

This aspect can facilitate the cooling of the light emitting unit (2).

In a photocosmetic device (100) of a forty-first aspect realized in combination with the thirty-ninth or fortieth aspect, the porous member (P10) is in contact with both the predetermined surface (500) of the heat conductor (50) and the heat dissipator (40).

This aspect can facilitate the cooling of the light emitting unit (2).

A photocosmetic device (100) of a forty-second aspect realized in combination with any one of the thirty-fourth to forty-first aspects further includes a fan (cooling fan 51) disposed to form an air flow flowing in a space between the predetermined surface (500) of the heat conductor (50) and the heat dissipator (40).

This aspect can facilitate the cooling of the light emitting unit (2).

In a photocosmetic device (100) of a forty-third aspect realized in combination with any one of the thirty-fourth to forty-second aspects, the light emitting unit (2) includes a first light source unit (201) and a second light source unit (202) each of which includes a light source (22). The heat dissipator (40) includes a first heat dissipation part (41) for dissipating heat generated in the first light source unit (201) and a second heat dissipation part (42) for dissipating heat generated in the second light source unit (202). The predetermined surface (500) of the heat conductor (50) includes a first region (501) and a second region (502). The first region (501) of the predetermined surface (500) of the heat conductor (50) faces the first heat dissipation part (41) with a gap between the first region (501) and the first heat dissipation part (41). The second region (502) of the predetermined surface (500) of the heat conductor (50) faces the second heat dissipation part (42) with a gap between the second region (502) and the second heat dissipation part (42).

According to this aspect, the cooling of both of the first light source unit (201) and the second light source unit (202) can be performed by a single heat conductor (50).

In a photocosmetic device (100) of a forty-fourth aspect realized in combination with the forty-third aspect, the first heat dissipation part (41), the heat conductor (50), and the second heat dissipation part (42) are arranged in an order of the first heat dissipation part (41), the heat conductor (50), and the second heat dissipation part (42) along one axis. The first region (501) of the predetermined surface (500) of the heat conductor (50) faces the first heat dissipation part (41) in the one axis. The second region (502) of the predetermined surface (500) of the heat conductor (50) faces the second heat dissipation part (42) in the one axis.

According to this aspect, the cooling of both of the first light source unit (201) and the second light source unit (202) can be performed by a single heat conductor (50).

In a photocosmetic device (100) of a forty-fifth aspect realized in combination with the forty-third or forty-fourth aspect, the light transmission member (3) includes a first light transmission piece (301) allowing light of the light source (22) of the first light source unit (201) to pass through, and a second light transmission piece (302) allowing light of the light source (22) of the second light source unit (202) to pass through. The first light transmission piece (301) and the second light transmission piece (302) are arranged such that a recess is formed between the first light transmission piece (301) and the second light transmission piece (302).

This aspect can facilitate the cooling of the light emitting unit (2).

In a photocosmetic device (100) of a forty-sixth aspect realized in combination with any one of the thirty-fourth to forty-fifth aspects, the light emitting unit (2) includes a plurality of LEDs (221) serving as the light source (22).

This aspect can reduce the uncomfortable feeling to be caused in a user.

A photocosmetic device (100) of a forty-seventh aspect realized in combination with any one of the first to forty-sixth aspects includes a light emitting unit (2), a housing (1), a light transmission member (3), and a dew condensation inhibitor (8). The light emitting unit (2) includes the plurality of light source units (20). The light emitting unit (2) is housed in the housing (1). The light transmission member (3) has a light incident surface (38) through which light of the light emitting unit (2) enters and a light emerging surface (39) from which the light entered through the light incident surface (38) is emerged to the outside of the housing (1). The dew condensation inhibitor (8) is configured to prevent dew condensation from occurring on the light transmission member (3) and/or to inhibit a dew condensation water on the light transmission member (3) generated by dew condensation from staying on the light transmission member (3).

A photocosmetic device (100) of a forty-seventh aspect includes a light emitting unit (2), a housing (1), a light transmission member (3), and a dew condensation inhibitor (8). The light emitting unit (2) is configured to emit light to be illuminated on a skin (92). The light emitting unit (2) is housed in the housing (1). The light transmission member (3) has a light incident surface (38) through which light of the light emitting unit (2) enters and a light emerging surface (39) from which the light entered through the light incident surface (38) is emerged to an outside of the housing (1). The dew condensation inhibitor (8) is configured to prevent dew condensation from occurring on the light transmission member (3) and/or to inhibit a dew condensation water on the light transmission member (3) generated by dew condensation from staying on the light transmission member (3).

This aspect can reduce the decline in the treatment efficiency for treating the skin (92).

In a photocosmetic device (100) of a forty-eighth aspect realized in combination with the forty-seventh aspect, the dew condensation inhibitor (8) includes a hydrophobic coating (81) provided to at least one of the light incident surface (38) or the light emerging surface (39) of the light transmission member (3).

This aspect can inhibit the staying of the dew condensation water on the light transmission member (3) generated by the dew condensation and can reduce the decline in the treatment efficiency for treating the skin (92).

In a photocosmetic device (100) of a forty-ninth aspect realized in combination with the forty-seventh or forty-eighth aspect, the dew condensation inhibitor (8) includes a water-repellent structure (80) provided to at least one of the light incident surface (38) or the light emerging surface (39) of the light transmission member (3).

This aspect can inhibit the staying of the dew condensation water on the light transmission member (3) generated by the dew condensation and can reduce the decline in the treatment efficiency for treating the skin (92).

In a photocosmetic device (100) of a fiftieth aspect realized in combination with any one of the forty-seventh to forty-ninth aspects, the dew condensation inhibitor (8) includes a dehumidifying material (82) disposed in the housing (1).

This aspect can prevent the dew condensation on the light transmission member (3) and can reduce the decline in the treatment efficiency for treating the skin (92).

In a photocosmetic device (100) of a fifty-first aspect realized in combination with the fiftieth aspect, the housing (1) has a housing space (S10) in which the light emitting unit (2) is housed. The dehumidifying material (82) is disposed in a passage connected to the housing space (S10) and allowing an air to pass through.

This aspect can prevent the dew condensation on the light transmission member (3) and can reduce the decline in the treatment efficiency for treating the skin (92).

In a photocosmetic device (100) of a fifty-second aspect realized in combination with the fiftieth or fifty-first aspect, the housing (1) has a housing space (S10) in which the light emitting unit (2) is housed. The dehumidifying material (82) is disposed in the housing space (S10).

This aspect can prevent the dew condensation on the light transmission member (3) and can reduce the decline in the treatment efficiency for treating the skin (92).

In a photocosmetic device (100) of a fifty-third aspect realized in combination with any one of the fiftieth to fifty-second aspects, the dehumidifying material (82) includes at least one material selected from the group consisting of calcium chloride, silica gel, quicklime, and zeolite.

This aspect can prevent the dew condensation on the light transmission member (3) and can reduce the decline in the treatment efficiency for treating the skin (92).

In a photocosmetic device (100) of a fifty-fourth aspect realized in combination with any one of the fiftieth to fifty-third aspects, the dehumidifying material (82) is arranged such that moisture absorbed in the dehumidifying material (82) is evaporated by heat generated in the light emitting unit (2).

This aspect can recover the function of the dehumidifying material (82).

In a photocosmetic device (100) of a fifty-fifth aspect realized in combination with any one of the forty-seventh to fifty-fourth aspects, the dew condensation inhibitor (8) includes a wiping member (831) slidably movable over at least one of the light incident surface (38) or the light emerging surface (39) of the light transmission member (3).

This aspect can inhibit the staying of the dew condensation water on the light transmission member (3) generated by the dew condensation and can reduce the decline in the treatment efficiency for treating the skin (92).

In a photocosmetic device (100) of a fifty-sixth aspect realized in combination with the fifty-fifth aspect, the wiping member (831) includes a dehumidifying agent.

This aspect can inhibit the staying of the dew condensation water on the light transmission member (3) generated by the dew condensation and can reduce the decline in the treatment efficiency for treating the skin (92).

In a photocosmetic device (100) of a fifty-seventh aspect realized in combination with any one of the forty-seventh to fifty-sixth aspects, the dew condensation inhibitor (8) includes a vibration unit (86) configured to vibrate the light transmission member (3).

This aspect can inhibit the staying of the dew condensation water on the light transmission member (3) generated by the dew condensation and can reduce the decline in the treatment efficiency for treating the skin (92).

In a photocosmetic device (100) of a fifty-eighth aspect realized in combination with any one of the forty-seventh to fifty-seventh aspects, the dew condensation inhibitor (8) includes a blowing member (84) configured to form an air flow to take away heat generated in the light emitting unit (2) to be apart from the light transmission member (3).

This aspect can prevent the dew condensation on the light transmission member (3) and can reduce the decline in the treatment efficiency for treating the skin (92).

In a photocosmetic device (100) of a fifty-ninth aspect realized in combination with any one of the forty-seventh to fifty-eighth aspects, the dew condensation inhibitor (8) includes a blowing unit (85) configured to form an air flow flowing over at least one of the light incident surface (38) or the light emerging surface (39) of the light transmission member (3).

This aspect can inhibit the staying of the dew condensation water on the light transmission member (3) generated by the dew condensation and can reduce the decline in the treatment efficiency for treating the skin (92).

A photocosmetic device (100) of a sixtieth aspect realized in combination with any one of the forty-seventh to fifty-ninth aspects further includes a cooler (5) disposed to cool the light transmission member (3).

This aspect can prevent the dew condensation from occurring on the light transmission member (3) while it is cooled by the cooler (5) and can reduce the decline in the treatment efficiency for treating the skin (92).

In a photocosmetic device (100) of a sixty-first aspect realized in combination with any one of the forty-seventh to sixtieth aspects, the dew condensation inhibitor (8) is configured to prevent the dew condensation from occurring on the light incident surface (38) of the light transmission member (3).

In a photocosmetic device (100) of a sixty-second aspect realized in combination with any one of the forty-seventh to sixty-first aspects, the dew condensation inhibitor (8) is configured to prevent the dew condensation from occurring on the light emerging surface (39) of the light transmission member (3).

In a photocosmetic device (100) of a sixty-third aspect realized in combination with any one of the forty-seventh to sixty-second aspects, the dew condensation inhibitor (8) is configured to inhibit the dew condensation water on the light incident surface (38) of the light transmission member (3) generated by dew condensation from staying on the light transmission member (3).

In a photocosmetic device (100) of a sixty-fourth aspect realized in combination with any one of the forty-seventh to sixty-third aspects, the dew condensation inhibitor (8) is configured to inhibit the dew condensation water on the light emerging surface (39) of the light transmission member (3) generated by dew condensation from staying on the light transmission member (3).

Features of the second to sixty-fourth aspects are optional for the photocosmetic device (100) and may be appropriately omitted.

### Reference Signs List

- 100: Photocosmetic device
- 1: Housing
- 105: First Housing Part
- 106: Second Housing Part
- 11: First Case
- 12: Second Case
- 13: Light Exit Surface
- 2: Light Emitting Unit
- 20: Light Source Unit
- 201: First Light Source Unit
- 202: Second Light Source Unit
- 22: Light Source
- 221: LED
- 23: Light Blocking Member
- 230: Light Blocking Part
- 25: Light Guide Member
- 250: Recess
- 251: Light Incident Surface
- 252: Light Emerging Surface
- 3: Light Transmission Member
- 30: Light Transmission Piece
- 301: First Light Transmission Piece
- 302: Second Light Transmission Piece
- 32: Partition
- 38: Light Incident Surface
- 39: Light Emerging Surface
- 4: Heat Dissipation Part
- 40: Heat Dissipator
- 41: First Heat Dissipation Part
- 42: Second Heat Dissipation Part
- 5: Cooler
- 50: Heat Conductor
- 500: Predetermined Surface
- 501: First Region
- 502: Second Region
- 51: Cooling Fan (Fan)
- 52: Peltier Device
- 8: Dew Condensation Inhibitor
- 80: Water-Repellent Structure
- 81: Coating
- 82: Dehumidifying Material
- 831: Wiping member
- 84: Blowing Member
- 85: Blowing Unit
- 86: Vibration Unit
- 92: Skin
- A10: Optical Axis
- G1: Gap
- Im1: Imaginary Plane
- Im10: Imaginary Plane
- P10: Porous Member
- S10: Housing Space
- X1: Depth
- X5: Distance

## Claims

1. A photocosmetic device comprising:
a plurality of light source units each of which includes a light source;
a housing in which the plurality of light source units are housed; and
a light exit surface from which light of the light source of each of the plurality of light source units is emitted to an outside of the housing,
the light exit surface having a recessed shape recessed inward the housing.

2. The photocosmetic device of claim 1, wherein
each of the plurality of light source units includes, as the light source, an LED array including a plurality of LEDs arranged in an array.

3. The photocosmetic device of claim 1 or 2, further comprising a light transmission member allowing the light of the light source of each of the plurality of light source units to pass through, wherein
the light exit surface is at least partially constituted by a surface of the light transmission member.

4. The photocosmetic device of claim 3, wherein
the light transmission member includes a plurality of light transmission pieces arranged to correspond to the plurality of light source units, respectively,
each of the plurality of light transmission pieces is positioned on an optical axis of a corresponding one of the plurality of light source units.

5. The photocosmetic device of claim 4, further comprising a partition that separates the plurality of light transmission pieces from one another in the light exit surface.

6. The photocosmetic device of any one of claims 3 to 5, further comprising a cooler disposed to cool the light transmission member.

7. The photocosmetic device of any one of claims 1 to 6, wherein light sources of the plurality of light source units are apart from one another.

8. The photocosmetic device of any one of claims 1 to 7, further comprising a light blocking part configured to reduce light traveling from a light source of one of the plurality of light source units toward a light source of at least one of remainder of the plurality of light source units.

9. The photocosmetic device of claim 8, further comprising a light transmission member constituting at least part of the light exit surface and allowing the light of the light source of each of the plurality of light source units to pass through,
the light blocking part is in contact with the light transmission member.

10. The photocosmetic device of any one of claims 1 to 9, the plurality of light source units have respective optical axes each of which intersects with the light exit surface at right angles.

11. The photocosmetic device of any one of claims 1 to 10, wherein
the plurality of light source units includes a first light source unit and a second light source unit,
the housing includes a first housing part in which the first light source unit is housed and a second housing part in which the second light source unit is housed,
the first housing part and the second housing part are disposed separately from each other in a direction in which the first light source unit and the second light source unit are arranged.

12. The photocosmetic device of claim 11, wherein at least one of the first housing part or the second housing part is movable in the direction in which the first light source unit and the second light source unit are arranged.

13. The photocosmetic device of claim 12, further comprising a stopper mechanism configured to restrict a movement of at least one of the first housing part or the second housing part such that a gap remains between the first housing part and the second housing part even in a state where the first housing part and the second housing part are approached closest each other.

14. The photocosmetic device of any one of claims 1 to 13, wherein each of the plurality of light source units further includes a light guide member configured to guide the light of the light source toward the light exit surface.

15. The photocosmetic device of claim 14, wherein
each of the plurality of light source units including a plurality of LEDs serving as the light source, and
in each of the plurality of light source units,
the plurality of LEDs are arranged in a line along one axis, and
the light guide member has cross sectional shapes in any imaginary planes perpendicular to the one axis, the cross sectional shapes being same as each other.

16. The photocosmetic device of claim 14 or 15, wherein
in each of the plurality of light source units,
the light guide member has a recess in a surface that faces the light source, and
the light source is disposed inside the recess.

17. The photocosmetic device of any one of claims 14 to 16, wherein
in each of the plurality of light source units,
the light guide member has a light incident surface through which the light of the light source enters and a light emerging surface from which the light entered through the light incident surface is emerged to the light exit surface, and
the light incident surface has a lens surface.

18. The photocosmetic device of any one of claims 14 to 17, wherein
in each of the plurality of light source units,
the light guide member has a light incident surface through which the light of the light source enters and a light emerging surface from which the light entered through the light incident surface is emerged to the light exit surface, and
the light emerging surface has a lens surface.

19. The photocosmetic device of claim 18, wherein
light emerging surfaces of respective light guide members of the plurality of light source units are disposed such that rays of the light of the plurality of light source units cross one another on an outside of the housing.

20. The photocosmetic device of any one of claims 14 to 19, wherein
each of the plurality of light source units further includes a light blocking member configured to reduce light traveling from the light source toward at least one of remainder of the plurality of light source units.

21. The photocosmetic device of claim 20, wherein
in each of the plurality of light source units, the light blocking member is disposed to surround side surfaces of the light guide member.

22. The photocosmetic device of any one of claims 14 to 21, wherein
in each of the plurality of light source units, the light guide member has a light incident surface through which the light of the light source enters and a light emerging surface from which the light entered through the light incident surface is emerged to the light exit surface, and
in at least one of the plurality of light source units, the light guide member has a cross sectional area that decreases from the light incident surface to the light emerging surface.

23. The photocosmetic device of any one of claims 14 to 22, further comprising a heat dissipation part configured to dissipate heat generated in the light source of each of the plurality of light source units.

24. The photocosmetic device of claim 23, wherein
the housing includes
a first case in which the heat dissipation part is housed and
a second case which protrudes from the first case along a first axis and in which respective light guide members of the plurality of light source units are housed,
the plurality of light source units are arranged along a second axis perpendicular to the first axis, and
a maximum dimension along the second axis of the first case is greater than a maximum dimension along the second axis of the second case.

25. The photocosmetic device of claim 24, wherein
an area of a first projection region is greater than an area of a second projection region, the first projection region being a region enclosed by a first projection line obtained by projecting a circumference of the first case on an imaginary plane perpendicular to the first axis, the second projection region being a region enclosed by a second projection line obtained by projecting a circumference of the second case on the imaginary plane.

26. The photocosmetic device of claim 24 or 25, wherein
the second case has a far end away from the first case in the first axis, and the light exit surface is located at the far end of the second case.

27. The photocosmetic device of any one of claims 1 to 26, comprising:
a light emitting unit including the plurality of light source units;
a heat dissipator thermally connected to the light emitting unit and configured to dissipate heat generated in the light emitting unit;
a light transmission member allowing light of the light emitting unit to pass through;
a heat conductor thermally connected to the light transmission member to receive heat from the light transmission member; and
a cooler disposed to cool the heat conductor, wherein
a predetermined surface, which is at least part of a surface of the heat conductor, faces the heat dissipator with a gap between the predetermined surface and the heat dissipator.

28. The photocosmetic device of claim 27, wherein
a distance between the predetermined surface of the heat conductor and the heat dissipator is set to allow a dew condensation water on the predetermined surface generated by dew condensation to be in contact with the heat dissipator.

29. The photocosmetic device of claim 27 or 28, wherein
the predetermined surface of the heat conductor and the heat dissipator face each other with an air space between the predetermined surface and the heat dissipator.

30. The photocosmetic device of any one of claims 27 to 29, further comprising a porous member disposed between the predetermined surface of the heat conductor and the heat dissipator.

31. The photocosmetic device of claim 30, wherein
the porous member is in contact with both the predetermined surface of the heat conductor and the heat dissipator.

32. The photocosmetic device of any one of claims 27 to 31, further comprising a fan disposed to form an air flow flowing in a space between the predetermined surface of the heat conductor and the heat dissipator.

33. The photocosmetic device of any one of claims 27 to 32, wherein
the plurality of light source units includes a first light source unit and a second light source unit,
the heat dissipator includes a first heat dissipation part for dissipating heat generated in the first light source unit and a second heat dissipation part for dissipating heat generated in the second light source unit,
the predetermined surface of the heat conductor includes a first region and a second region,
the first region of the predetermined surface of the heat conductor faces the first heat dissipation part with a gap between the first region and the first heat dissipation part, and
the second region of the predetermined surface of the heat conductor faces the second heat dissipation part with a gap between the second region and the second heat dissipation part.

34. The photocosmetic device of claim 33, wherein
the first heat dissipation part, the heat conductor, and the second heat dissipation part are arranged in an order of the first heat dissipation part, the heat conductor, and the second heat dissipation part along one axis,
the first region of the predetermined surface of the heat conductor faces the first heat dissipation part in the one axis, and
the second region of the predetermined surface of the heat conductor faces the second heat dissipation part in the one axis.

35. The photocosmetic device of claim 33 or 34, wherein
the light transmission member includes
a first light transmission piece allowing light of the light source of the first light source unit to pass through, and
a second light transmission piece allowing light of the light source of the second light source unit to pass through, and
the first light transmission piece and the second light transmission piece are arranged such that a recess is formed between the first light transmission piece and the second light transmission piece.

36. The photocosmetic device of any one of claims 1 to 35, comprising:
a light emitting unit including the plurality of light source units;
the housing in which the light emitting unit is housed;
a light transmission member having a light incident surface through which light of the light emitting unit enters and a light emerging surface from which the light entered through the light incident surface is emerged to the outside of the housing; and
a dew condensation inhibitor configured to prevent dew condensation from occurring on the light transmission member and/or to inhibit a dew condensation water on the light transmission member generated by dew condensation from staying on the light transmission member.

37. The photocosmetic device of claim 36, wherein
the dew condensation inhibitor includes a hydrophobic coating provided to at least one of the light incident surface or the light emerging surface of the light transmission member.

38. The photocosmetic device of claim 36 or 37, wherein
the dew condensation inhibitor includes a water-repellent structure provided to at least one of the light incident surface or the light emerging surface of the light transmission member.

39. The photocosmetic device of any one of claims 36 to 38, wherein
the dew condensation inhibitor includes a dehumidifying material disposed in the housing.

40. The photocosmetic device of claim 39, wherein
the housing has a housing space in which the light emitting unit is housed, and
the dehumidifying material is disposed in a passage connected to the housing space and allowing an air to pass through.

41. The photocosmetic device of claim 39 or 40, wherein
the housing has a housing space in which the light emitting unit is housed, and
the dehumidifying material is disposed in the housing space.

42. The photocosmetic device of any one of claims 39 to 41, wherein
the dehumidifying material is arranged such that moisture absorbed in the dehumidifying material is evaporated by heat generated in the light emitting unit.

43. The photocosmetic device of any one of claims 36 to 42, wherein
the dew condensation inhibitor includes a wiping member slidably movable over at least one of the light incident surface or the light emerging surface of the light transmission member.

44. The photocosmetic device of claim 43, wherein
the wiping member includes a dehumidifying agent.

45. The photocosmetic device of any one of claims 36 to 44, wherein
the dew condensation inhibitor includes a vibration unit configured to vibrate the light transmission member.

46. The photocosmetic device of any one of claims 36 to 45, wherein
the dew condensation inhibitor includes a blowing member configured to form an air flow to take away heat generated in the light emitting unit to be apart from the light transmission member.

47. The photocosmetic device of any one of claims 36 to 46, wherein
the dew condensation inhibitor includes a blowing unit configured to form an air flow flowing over at least one of the light incident surface or the light emerging surface of the light transmission member.

48. The photocosmetic device of any one of claims 36 to 47, further comprising a cooler disposed to cool the light transmission member.

49. The photocosmetic device of any one of claims 36 to 48, wherein
the dew condensation inhibitor is configured to prevent the dew condensation from occurring on the light incident surface of the light transmission member.

50. The photocosmetic device of any one of claims 36 to 49, wherein
the dew condensation inhibitor is configured to prevent the dew condensation from occurring on the light emerging surface of the light transmission member.

51. The photocosmetic device of any one of claims 36 to 50, wherein
the dew condensation inhibitor is configured to inhibit the dew condensation water on the light incident surface of the light transmission member generated by dew condensation from staying on the light transmission member.

52. The photocosmetic device of any one of claims 36 to 51, wherein
the dew condensation inhibitor is configured to inhibit the dew condensation water on the light emerging surface of the light transmission member generated by dew condensation from staying on the light transmission member.
